# EUROPEAN PATENT SPECIFICATION

(11) **EP 2 981 533 B1**
(45) Date of publication and mention of the grant of the patent: **06.12.2017**
(21) Application number: 14722686.4
(22) Date of filing: 03.04.2014
(51) Int. Cl.: C07D 401/14, C07D 471/04, A61K 31/4439, A61P 35/00

(54) **PROTEIN KINASE INHIBITORS**
PROTEINKINASEHEMMER
INHIBITEURS DE PROTÉINES KINASES

(30) Priority: 04.04.2013 IN 382KO2013
(43) Date of publication of application: 10.02.2016
(73) Proprietor: Orion Corporation, 02200 Espoo (FI)
(72) Inventor: RAJAGOPALAN, Srinivasan, Bangalore 560-068 (IN); APPUKUTTAN, Prasad, Bangalore 560076 India (IN); NARASINGAPURAM ARUMUGAM, Karthikeyan, Vellore 632515 India (IN); UJJINAMATADA, Ravi Kotrabasaiah, Bangalore 560102 India (IN); GEORGE, Shyla, Bangalore-100 India (IN); LINNANEN, Tero, FI-04300 Tuusula (FI)
(74) Representative: J A Kemp
(86) International application number: PCT/FI2014/000003
(87) International publication number: WO 2014/162039

(56) References cited:
- WO-A1-2005/021531
- WO-A1-2010/119284
- WO-A1-2011/038579
- WO-A1-2013/053983
- WO-A2-2008/078100

## Description

### Technical field

The present invention relates to therapeutically active compounds and pharmaceutically acceptable salts thereof which are useful e.g. in the treatment of cancer.

### Background of the invention

Protein kinases are a class of proteins (enzymes) that regulate a variety of cellular functions. This is accomplished by phosphorylation of specific amino acids on protein substrates resulting in conformational alteration of the substrate protein. The conformational change modulates the activity of the substrate or its ability to interact with other binding partners. Tyrosine kinases are a subset of protein kinases that catalyze the transfer of the terminal phosphate of adenosine triphosphate (ATP) to tyrosine residues on protein substrates. The human genome contains around 90 tyrosine kinases and 43 tyrosine kinase like genes, the products of which regulate cellular proliferation, survival, differentiation, function and motility.

Tyrosine kinases are of two varieties, i.e. receptor and non-receptor tyrosine kinases. Receptor tyrosine kinases (e.g., FGFR) are trans-membrane proteins with a ligand-binding extracellular domain and a catalytic intracellular kinase domain, while non-receptor tyrosine kinases (e.g., c-ABL) lack trans-membrane domains and are found in the cytosol, nucleus and inner surface of cell membrane. Kinase domains of all tyrosine kinases have bilobar architecture, with an N-terminal lobe that binds ATP and magnesium, a C-terminal lobe containing an activation loop, and a cleft between the lobes to which polypeptide substrates bind.

Receptor tyrosine kinases become activated when ligand binds to the extracellular domain, resulting in receptor oligomerization and autophosphorylation of a regulatory tyrosine within the activation loop of the kinase domain. These phenomena reorient important amino acid residues, thereby enhancing catalytic activity of the enzyme.

Fibroblast growth factor (FGF) has been recognized as an important mediator of many physiological processes, such as cell migration, proliferation, survival and differentiation during development and angiogenesis. There are currently over 25 known members of the FGF family. The fibroblast growth factor receptor (FGFR) family consists of four members with each composed of an extra cellular ligand binding domain, a single trans-membrane domain and an intracellular cytoplasmic protein tyrosine kinase domain. Upon stimulation with FGF, FGFRs undergo dimerisation and transphosphorylation. Upon dimerization, FGFRs activate range of downstream signaling pathways, such as MAPK and PKB/Akt pathways (Zhou, W. et. al. Chemistry & Biology, 2010, 17, 285). Abnormal FGFR signaling has been reported in many tumor types including multiple myeloma, gastric, endometrial, prostate and breast (Squires M. et. al. Mol. Cancer Ther., September 2011, 10:1542-1552). FGFs also have role in tumor angiogenesis and mediate resistance to vascular endothelial growth factor receptor 2 (VEGFR2) inhibitors (Casanovas, O. et. al., Cancer Cell, 2005, 8, 299). Consequently, FGF and FGFRs have the potential to initiate and/or promote tumorigenesis. Due to this, the FGF signaling system happens to be an attractive therapeutic target, mainly because therapies targeting FGFRs and/or FGF signaling may affect both the tumor cells and also tumor angiogenesis (Foote, K. M. et. al., WO 2009/019518 A1). Consequently, FGF and FGFRs have the potential to initiate and/or promote tumorigenesis.

WO 2008/078100 relates to bicyclic heterocyclic derivative compounds and to use of the compounds in the treatment of diseases, e.g. cancer.

### Summary of the invention

It has been found that compounds of formula (I) inhibit or modulate the activity of certain protein kinases, more specifically protein tyrosine kinases. In particular, it has been found that the compounds of formula (I) are potent and selective inhibitors of FGFR kinases. The compounds of the invention have antiproliferative activity and are particularly useful in the treatment of cancer.

The compounds of the present invention have a structure represented by formula (I) 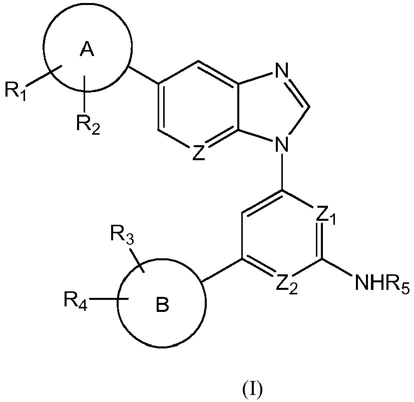 wherein
Z₁ is N and Z₂ is CH, or
Z₁ is CH and Z₂ is N, or
Z₁ and Z₂ is N;
Z is CH or N;
A is a phenyl ring or a 5 -12 membered heterocyclic ring;
R₁ is H, C₁₋₇ alkyl, C₃₋₇ cycloalkyl, C₃₋₇ cycloalkyl C₁₋₇ alkyl, C₁₋₇ alkoxy, C₁₋₇ alkyl carbonyl, amino, hydroxy, hydroxy C₁₋₇ alkyl, halo C₁₋₇ alkyl, C₁₋₇ alkylamino C₁₋₇ alkyl, -R₁₆-C(O)-R₁₇ or -E-R₆;
R₂ is H, halogen or C₁₋₇ alkyl;
B is a 5-12 membered carbocyclic ring and R₃ is halogen, C₁₋₇ alkyl, C₁₋₇ alkoxy, halo C₁₋₇ alkyl or halo C₁₋₇ alkoxy; or B is a 5-12 membered heterocyclic ring and
R₃ is H, halogen, C₁₋₇ alkyl, C₁₋₇ alkoxy, halo C₁₋₇ alkyl or halo C₁₋₇ alkoxy;
R₄ is H, halogen, C₁₋₇ alkyl or oxo;
R₅ is H, -C(O)R₇, -SO₂R₈ or an optionally substituted 5-6 membered heterocyclic ring;
R₆ is an optionally substituted 5-6 membered heterocyclic ring;
R₇ is C₁₋₇ alkyl, C₂₋₇ alkenyl, C₁₋₇ alkoxy, C₁₋₇ alkoxy C₁₋₇ alkyl, carboxy C₁₋₇ alkyl, C₁₋₇ alkoxy carbonyl C₁₋₇ alkyl, C₁₋₇ alkylamino C₁₋₇ alkyl, -NH-R₁₀ or -NH-X₁-R₁₁;
R₈ is C₁₋₇ alkyl, C₂₋₇ alkenyl, C₃₋₇ cycloalkyl, hydroxy C₁₋₇ alkyl, -NR₁₃R₁₄,-NH-X₂-R₁₅, phenyl or an optionally substituted 5-6 membered heterocyclic ring;
R₁₀ is C₁₋₇ alkyl or C₃₋₇ cycloalkyl;
R₁₁ is phenyl or an optionally substituted 5-6 membered heterocyclic ring;
R₁₃ and R₁₄ are, independently, H, C₁₋₇ alkyl or C₃₋₇ cycloalkyl;
R₁₅ is phenyl or an optionally substituted 5-6 membered heterocyclic ring;
R₁₆ is a bond or a C₁₋₇ alkyl;
R₁₇ is C₁₋₇ alkyl, C₁₋₇ alkoxy, C₁₋₇ alkylamino, amino or hydroxy;
E is a bond or a C₁₋₇ alkyl;
X₁ and X₂ are, independently, a bond or C₁₋₇ alkyl;
and pharmaceutically acceptable salts thereof.

In one class of compounds are compounds of formula (I), wherein Z is CH. In another class of compounds are compounds of formula (I), wherein Z₁ is N and Z₂ is CH. In another class of compounds are compounds of formula (I), wherein Z₁ is CH and Z₂ is N. In another class of compounds are compounds of formula (I), wherein Z₁ and Z₂ is N.

In a subclass class of any of the above classes are compounds of formula (I), wherein ring A is any one of the following groups or tautomers thereof: 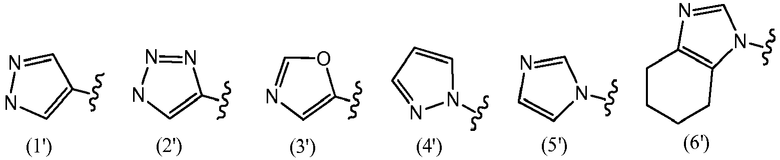 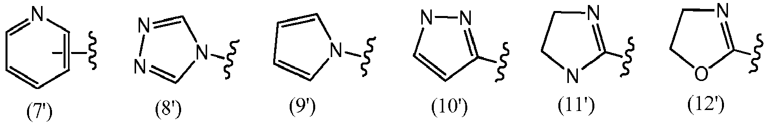 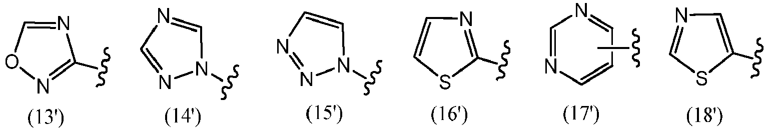 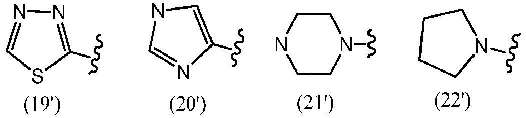 and R₁ and R₂, as defined above, are attached to the above A-rings.

In a subclass class of any of the above classes are compounds of formula (I), wherein ring B is any one of the following groups or tautomers thereof: 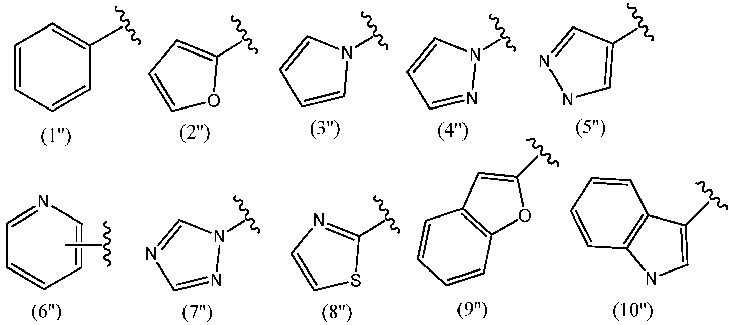 and R₃ and R₄, as defined above, are attached to the above B-rings.

Another subclass of the above classes are compounds wherein
A is a ring of formula (1'), (2'), (3'), (4'), (5'), (7'), (14'), (16') or (20');
R₁ is H, C₁₋₇ alkyl, C₁₋₇ alkoxy, hydroxy C₁₋₇ alkyl, C₁₋₇ alkylamino C₁₋₇ alkyl or
-E-R₆;
R₂ is H;
Z is CH;
B is a ring of formula (1 "), (2"), (3 "), (4") or (6");
E is a bond or C₁₋₇ alkyl;
R₆ is any of the following groups 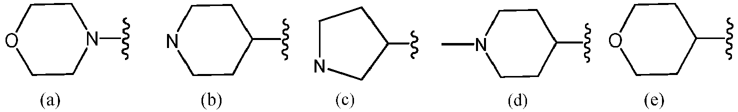 R₃ is H, halogen, C₁₋₇ alkyl, C₁₋₇ alkoxy;
R₄ is H or halogen;
R₅ is -C(O)R₇ or -SO₂R₈ or any one of the following groups 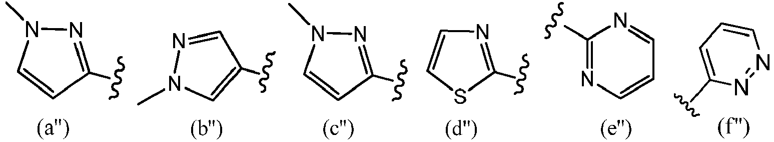 R₇ is C₁₋₇ alkyl, C₂₋₇ alkenyl or -NH-R₁₀;
R₈ is C₁₋₇ alkyl, C₂₋₇ alkenyl, C₃₋₇ cycloalkyl, hydroxy C₁₋₇ alkyl or -NR₁₃R₁₄; and
R₁₀ is C₁₋₇ alkyl or C₃₋₇ cycloalkyl.

In one class of compounds are compounds of formula (I), wherein R₅ is - C(O)R₇ or -SO₂R₈ or any one of the following groups

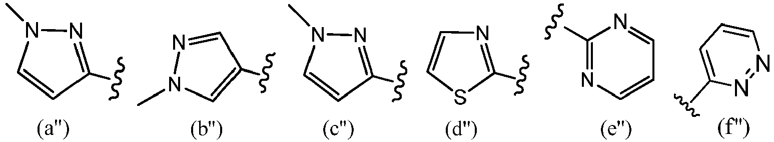

In one class of compounds are compounds of formula (I), wherein R₅ is - SO₂R₈.

In one class of compounds are compounds of formula (I), wherein R₆ is any of the following groups

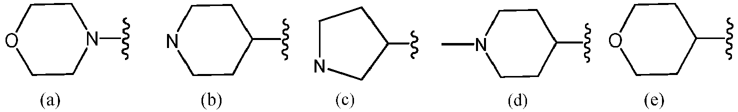

The present invention also provides a pharmaceutical composition comprising a compound of formula (I) or a pharmaceutically acceptable salt thereof together with a pharmaceutically acceptable carrier.

The present invention provides a compound of formula (I) or a pharmaceutically acceptable salt thereof for use in the treatment of a condition, where FGFR kinase inhibition is desired.

The present invention provides a compound of formula (I) or a pharmaceutically acceptable salt thereof for use in the treatment of cancer.

### Detailed description of the invention

The compounds of the invention can be prepared by a variety of synthetic routes analogously to the methods known in the literature using suitable starting materials. The compounds according to formula (I) can be prepared e.g. analogously or according to the following reaction Schemes. Some compounds included in the formula (I) can be obtained by converting the functional groups of the other compounds of formula (I) obtained in accordance with the following Schemes, by well known reaction steps such as oxidation, reduction, hydrolysis, acylation, alkylation, amidation, amination, sulfonation and others. It should be noted that any appropriate leaving groups, e.g. N-protecting groups, such as a t-butoxycarbonyl (t-BOC) group or a phenylsulfonyl group, can be used in well known manner during the syntheses in order to improve the selectivity of the reaction steps

Compounds of formula (I), wherein R₅ is -C(O)CH₃ can be prepared, for example, according to Scheme 1, wherein R₁, R₂, R₃, R₄, ring A, ring B and Z, Z₁ and Z₂ are as defined above, and R is hydrogen or alkyl. In the method of Scheme 1, the *N*-(3-bromo-5-nitrophenyl)acetamide [1] is coupled in a suitable solvent such as 1,2-dimethoxyethane with a boronic acid derivative [2] or a suitable ester thereof in the presence of Pd(dppf)Cl₂ and aqueous sodium carbonate at elevated temperature. The nitro group of the obtained compound [3] is reduced, e.g. with hydrogen and Pd/C catalyst, iron powder and aqueous calcium chloride or zinc and aqueous ammonium chloride, and the resulting amine [4] is reacted with compound [5] in a suitable solvent such as DMF in the presence of potassium fluoride at elevated temperature to obtain compound [6]. In case Z is CH in the compound [5], X" is suitably fluoro, and when Z is N, X" is suitably chloro. The nitro group in compound [6] is reduced, e.g. by using zinc and aqueous ammonium chloride or iron powder and aqueous calcium chloride, and the resulting amine [7] is heated with formic acid to produce compound [8] in a ring closure reaction. Finally, compound [10] is obtained by the Suzuki coupling between compound [8] and a boronic acid derivative [9] or a suitable ester thereof in a suitable solvent such as 1,2-dimethoxyethane in the presence of Pd(dppf)Cl₂ and aqueous sodium carbonate at elevated temperature.

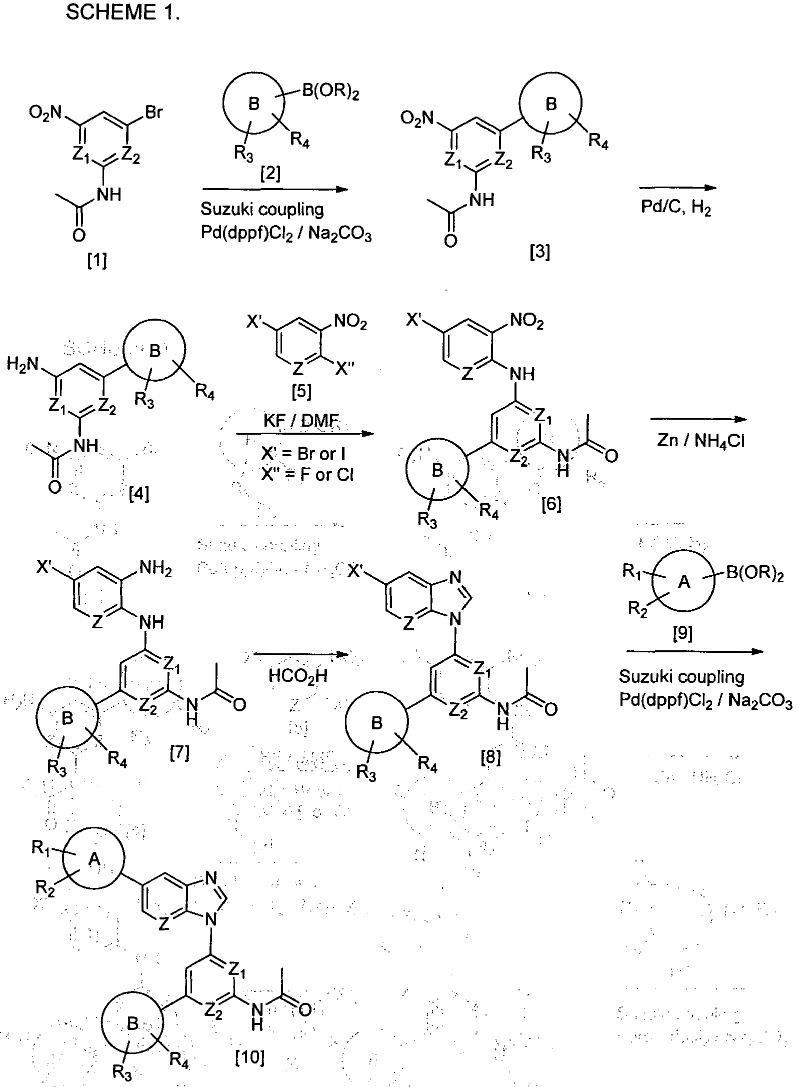

Alternatively, the compound of formula [3] can be prepared according to Scheme 2, wherein R₃, R₄, Z₁ and Z₂, ring B and R are as defined above, using the boronic acid derivative [11] or a suitable ester thereof in the presence of Pd(dppf)Cl₂ and aqueous sodium carbonate. Compound [11] can be prepared, e.g. by treating *N*-(3-bromo-5-nitrophenyl)acetamide with bis(pinacolato)diboron in the presence of Pd(dppf)Cl₂ and potassium acetate.

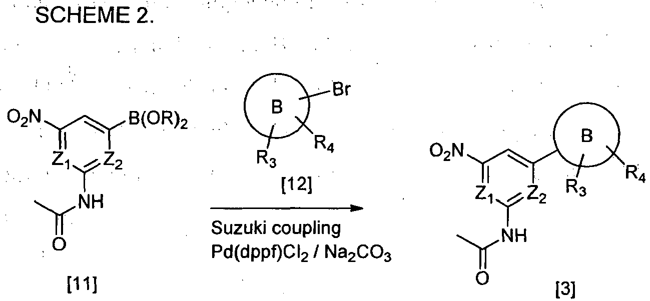

In case the B-ring in the compound [3] is a heterocycle linked to phenyl via a nitrogen heteroatom, the compound [3] can be also prepared using a copper-catalyzed Buchwald amination in the presence of a base such cesium carbonate or potassium carbonate according to Scheme 3, wherein Z₁, Z₂, R₃ and R₄ are as defined above.

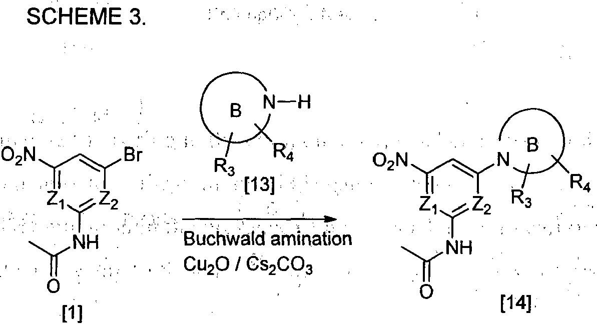

In case the B-ring in the compound [3] is pyrrole ring linked to phenyl via a nitrogen atom, the compound [3] can be also prepared from 3,5-dinitroaniline [15] and 2,5-dimethoxytetrahydrofuran according to Scheme 4, wherein Z₁ and Z₂ are as defined above. The pyrrole derivative [16] formed is reduced using ammonium sulphide to obtain compound [17], which is subsequently reacted with acetic anhydride to afford compound [18].

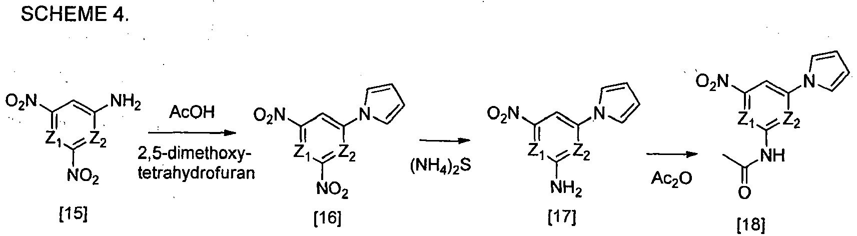

In case where ring A in the compound [10] is an oxazol-5-yl ring, the compound [10] can be also prepared according to Scheme 5, wherein ring B, R₃, R₄, Z₁ and Z₂ are as defined above. In this method the compound [4] is treated with 4-fluoro-3-nitrobenzaldehyde and the resulting compound [20] is thereafter reacted with toluene-sulfonylmethyl isocyanide to produce the oxazol-5-yl compound [21] in a ring closure reaction. The nitro group of compound [21] can be further reduced, e.g. by hydrogenation, to produce the corresponding amine, which can be then treated with formic acid according to Scheme 1 to afford the end product in the ring closure reaction.

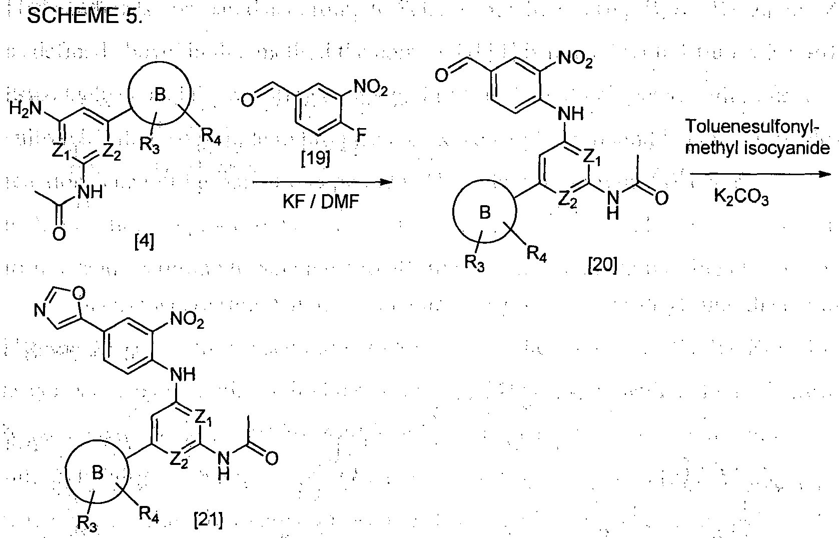

In case where ring A in the compound [10] is a heterocycle linked to the carbon atom of the bicyclic ring via a nitrogen heteroatom, the compound [10] can be also prepared using Buchwald coupling according to Scheme 6, wherein X', ring B, R₁, R₂, R₃, R₄, Z₁ and Z₂ are as defined above.

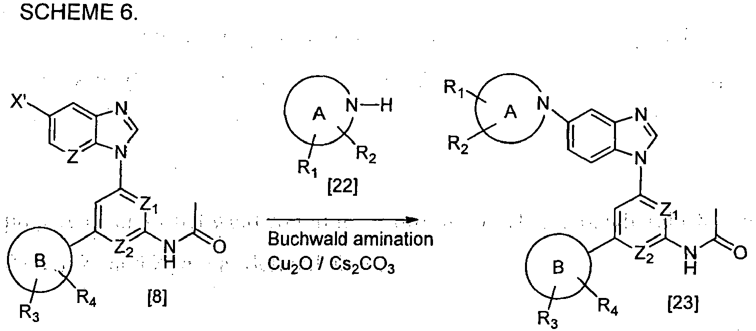

In case where ring A in the compound [10] is an 1H-1,2,3-triazol-4-yl ring and R2 is hydrogen, the compound [10] can be also prepared according to Scheme 7, wherein X', Z, R₁, R₃, R₄, Z₁, Z₂ and ring B, are as defined above. The starting compound [8] is silylated by reacting with ethynyltrimethylsilane in the presence of tetrakis(triphenylphosphine)palladium(0) (Pd(PPh₃)₄) and Cu(I)iodide to produce compound [32]. Treatment with TBAF affords the ethynyl compound [33] which can be reacted with azido compound R₁-N₃ in a suitable solvent, such as DMSO:THF:water (1:1:1) or DMSO:DCM:water (1:1:1) to afford compound [34].

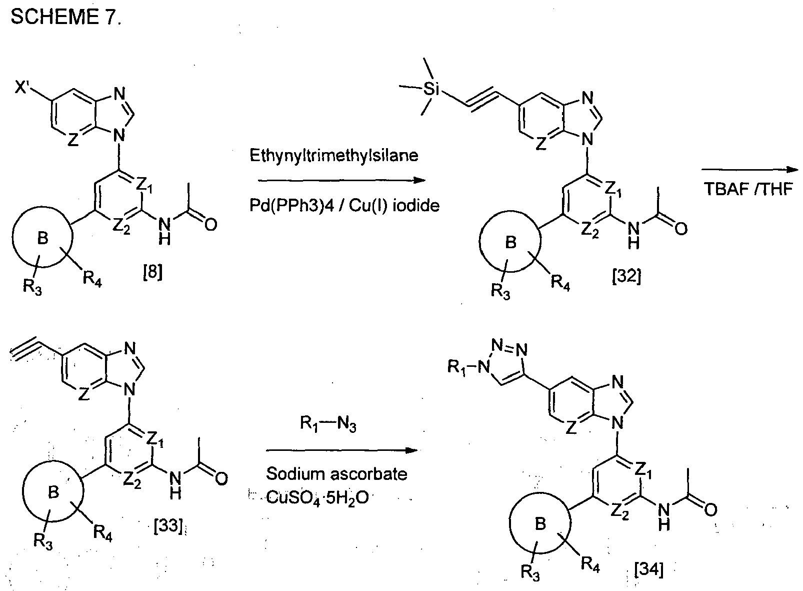

In case where ring A in the compound [10] is a 1-methyl-1H-pyrazol-3-yl ring, the compound [10] can be also prepared according to Scheme 8, wherein R₃, R₄, Z₁, Z₂ and ring B, are as defined above. In this method the compound [4] is treated with 1-(4-fluoro-3-nitrophenyl)ethanone and the resulting compound [36] is thereafter reacted with DMF dimethylacetal to produce the oxazol-5-yl compound [37]. Subsequent treatment with methyl hydrazine produces compound [38] in a ring closure reaction. The nitro group of compound [38] can be further reduced, e.g. by aqueous ammonium and zinc, to produce the corresponding amine, which can be then treated with formic acid according to Scheme 1 to afford the end product in the ring closure reaction.

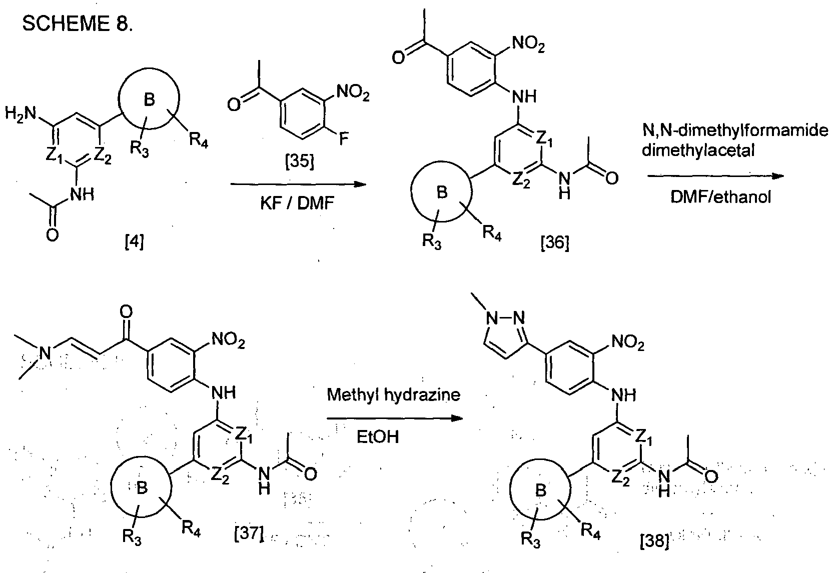

In case where ring A in the compound [10] is a 1H-imidazol-2-yl ring, the compound [10] can be also prepared according to Scheme 9, wherein R₃, R₄, Z₁, Z₂ and ring B, are as defined above. In this method the compound [20] of Scheme 5 is treated with ethylene diamine and *N*-bromosuccinimide affording compound [39] in a ring closure reaction. The nitro group of compound [39] can be further reduced, e.g. by aqueous ammonium and zinc, to produce the corresponding amine, which can be then treated with formic acid according to Scheme 1 to afford the end product in the ring closure reaction.

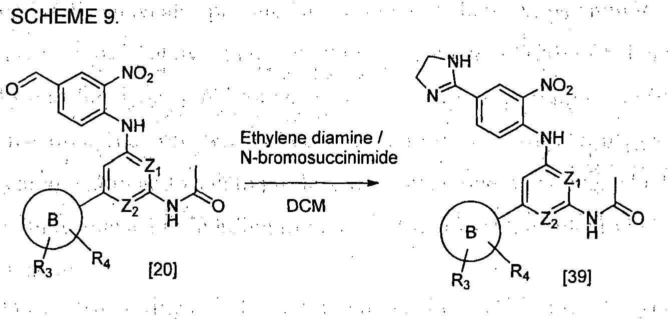

Various compounds of formula (I), wherein R₅ is other than -C(O)CH₃, can be prepared, for example, according to Scheme 10, wherein R₁, R₂, R₃, R₄, R₇, R₈, Z, Z₁, Z₂, ring A and ring B are as defined above. The acetamide compound [10] can be converted to its corresponding amine [24], for example, by heating in ethanol in the presence of a base, such as aqueous sodium hydroxide or potassium hydroxide, or an acid such as aqueous HCl. The obtained amine [24] can be used as a starting material for subsequent reaction steps. The compounds of formula (I), wherein R₅ is -SO₂R₈ can be prepared, for example, by treating the amine [24] with Cl-SO₂R₈ in suitable solvent such as DCM in the presence of pyridine. Compounds of formula (I), wherein R₅ is -C(O)R₇ and R₇ is C₁₋₇ alkyl or C₁₋₇ alkylamino C₁₋₇ alkyl, can be prepared, for example, by reacting the amine [24] with HOOC-R₇ in suitable solvent such as DMF in the presence of 2-(1H-7-azabenzotriazol-1-yl)-1,1,3,3-tetramethyl uronium hexafluorophosphate methanaminium (HATU) and DIPEA.

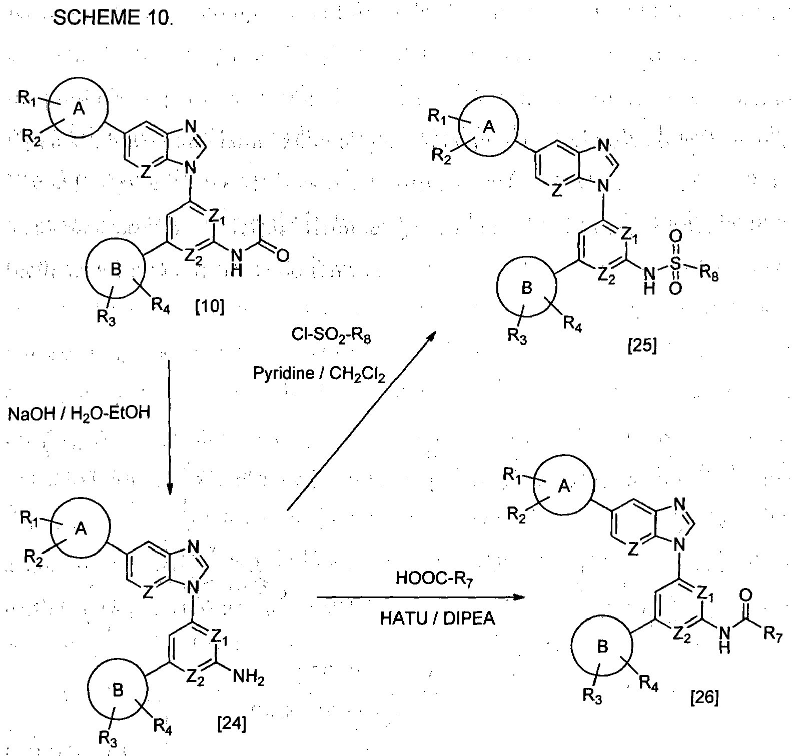

Compounds of formula (I), wherein R₇ is -NH-R₁₀ or -NH-X-R₁₁, can be prepared, for example, according to Scheme 11 by reacting the amine [24] in a suitable solvent such n-butanol with isocyanato derivatives O=C=N-R₁₀ or O=C=N-X-R₁₁ in the presence of suitable base such as triethylamine (TEA). Alternatively, compounds wherein R₇ is -NH-X-R₁₁ can be prepared by treating amine [24] in suitable solvent such as DCM with phosgene and then with H₂N-X-R₁₁, see Scheme 11.

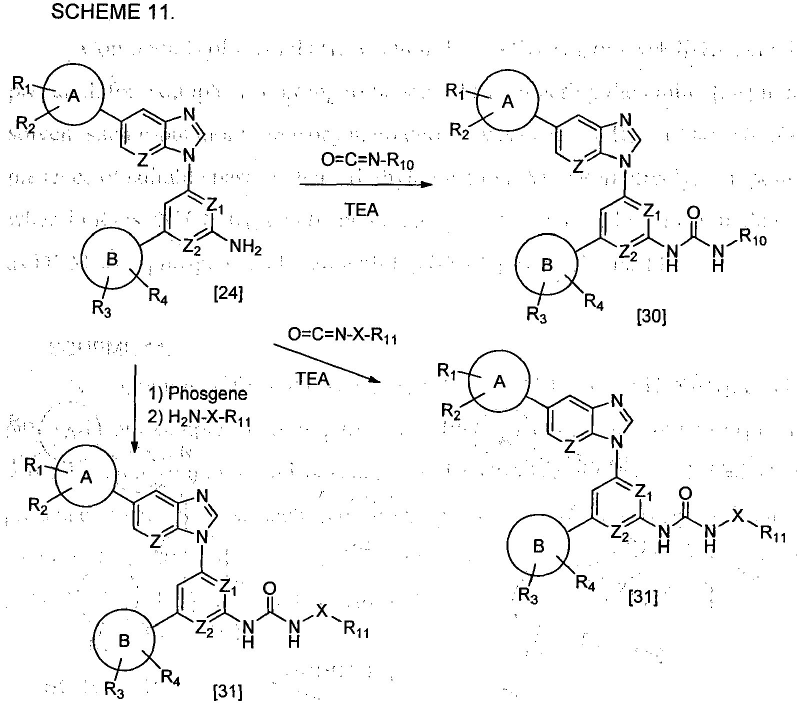

Compounds wherein R₅ is -C(O)R₇, -SO₂R₈ or an optionally substituted 5-6 membered heterocyclic ring can also be prepared according to Scheme 12 starting from compound [40] wherein X is a halogen such as Br or Cl, and R₁, R₂, R₃, R₄, Z, Z₁, Z₂ and ring B are as defined above, using palladium (e.g. Pd₂(dba)₃) catalyzed C-N coupling in the presence of a metal chelating ligand such as Xantphos.

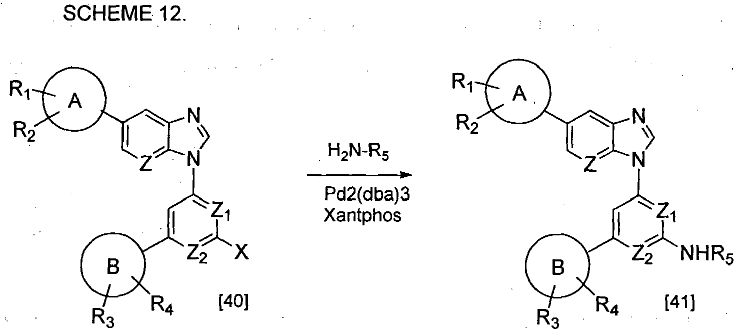

Compounds of formula (I) can be also prepared according to Scheme 13 by reacting compound [42] with compound [43] to produce compound [44] wherein X is a halogen such as Cl or Br, and R₁, R₂, R₃, R₄, Z, Z₁, Z₂ and ring B are as defined above, followed by the bicyclic ring closure as in Scheme 1 and addition of the -NHR₅ group according to Scheme 12.

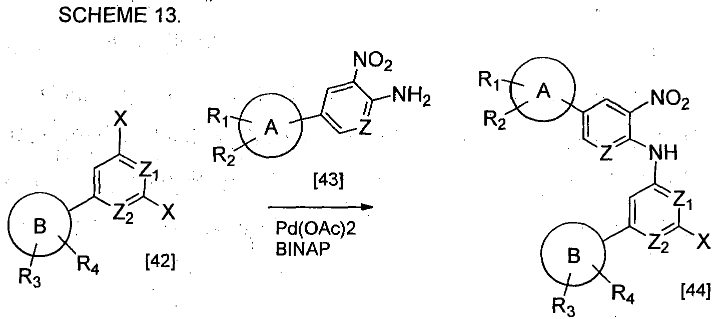

Compounds of formula (I) can be also prepared according to Scheme 14 by reacting compound [45] with compound [46] wherein X is a halogen such as Cl or Br, and R₁, R₂, R₃, R₄, R₅, Z, Z₁, Z₂ and rings A and B are as defined above.

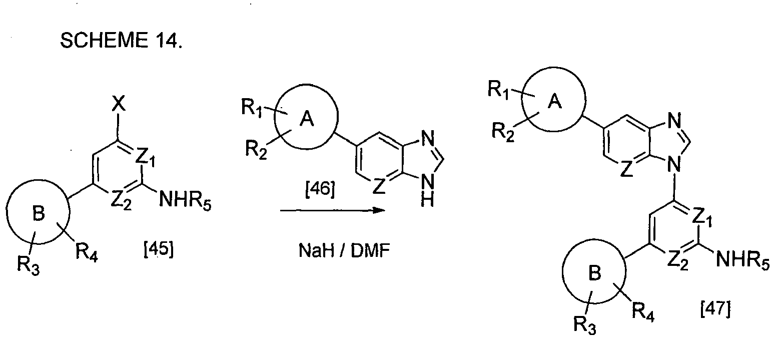

Compounds of formula (I) can be also prepared according to Scheme 15 by reacting compound [48] with compound [49] wherein X is a halogen such as Cl or Br, and R₁, R₂, R₅, Z, Z₁, Z₂ and rings A and B are as defined above. The formed compound [50] can be subjected to bicyclic ring closure and addition of B-ring by Suzuki coupling as described in Scheme 1 to obtaing compounds of formula (I).

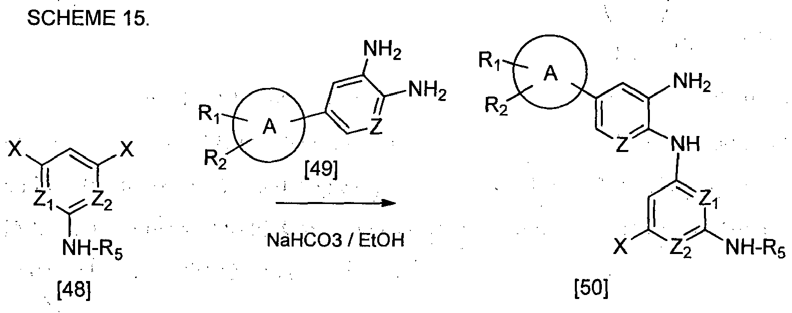

Pharmaceutically acceptable salts are well known in the field of pharmaceuticals. Non-limiting examples of suitable salts include metal salts, ammonium salt, salts with organic base, salts with inorganic acid, salts with organic acid, and salts with basic or acidic amino acid. Non-limiting examples of metal salts include alkali metal salts such as sodium salt and potassium salt; alkaline earth metal salts such as calcium salt, and magnesium salt. Non-limiting examples of salts with inorganic or organic acids include chlorides, bromides, sulfates, nitrates, phosphates, sulfonates, formates, tartrates, maleates, citrates, benzoates, salicylates and ascorbates. Pharmaceutically acceptable esters, when applicable, may be prepared by known methods using pharmaceutically acceptable acids that are conventional in the field of pharmaceuticals and that retain the pharmacological properties of the free form. Non-limiting examples of these esters include esters of aliphatic or aromatic alcohols, e.g. methyl, ethyl, propyl, isopropyl, butyl, isobutyl, *sec*-butyl, *tert*-butyl esters. Phosphate esters and carbonate esters, are also within the scope of the invention.

The terms employed herein have the following meanings:
The term "halo" or "halogen", as employed herein as such or as part of another group, refers to chlorine, bromine, fluorine or iodine.

The term "C₁₋₇ alkyl", as employed herein as such or as part of another group, refers to a straight or branched chain saturated hydrocarbon group having 1, 2, 3, 4, 5, 6 or 7 carbon atom(s). Representative examples of C₁₋₇ alkyl include, but are not limited to, methyl, ethyl, *n-*propyl, *iso*-propyl, *n*-butyl, *iso*-butyl, *sec*-butyl, *tert*-butyl, *n*-pentyl, *iso*-pentyl and *n*-hexyl. The term "C₁₋₃ alkyl" refers to an preferred embodiment of "C₁₋₇ alkyl" having 1, 2 or 3 carbon atoms.

The term "C₃₋₇ cycloalkyl", as employed herein as such or as part of another group, refers to a saturated cyclic hydrocarbon group containing 3, 4, 5, 6 or 7 carbon atoms. Representative examples of cycloalkyl include, but are not limited to, cyclopropyl, cyclobutyl, cyclopentyl and cyclohexyl.

The term "C₃₋₇ cycloalkyl C₁₋₇ alkyl", as employed herein refers to a C₃₋₇ cycloalkyle group, as defined herein, appended to the parent molecular moiety through a C₁₋₇ alkyl group, as defined herein.

The term "C₂₋₇ alkenyl", as employed herein as such or as part of another group, refers to an aliphatic hydrocarbon group having 2 to 7 carbon atoms and containing one or several double bonds. Representative examples include, but are not limited to, ethenyl, propenyl and cyclohexenyl.

The term "hydroxy", as employed herein as such or as part of another group, refers to an -OH group. The term "cyano", as employed herein as such or as part of another group, refers to a -CN group. The term "carboxy", as employed herein as such or as part of another group, refers to -COOH group. The term "carbonyl", as employed herein as such or as part of another group, refers to a carbon atom double-bonded to an oxygen atom (C=O). The term "oxo", as employed herein as such or as part of another group, refers to oxygen atom linked to another atom by a double bond (=O).

The term "C₁₋₇ alkoxy", as employed herein as such or as part of another group, refers to C₁₋₇ alkyl, as defined herein, appended to the parent molecular moiety through an oxygen atom. Representative examples of C₁₋₇ alkoxy include, but are not limited to methoxy, ethoxy, propoxy, butoxy, isobutoxy, *sec*-butoxy and *tert*-butoxy.

The term "hydroxyl C₁₋₇ alkyl", as employed herein, refers to at least one hydroxy group, as defined herein, appended to the parent molecular moiety through a C₁₋₇ alkyl group, as defined herein. Representative examples of hydroxyl C₁₋₇ alkyl include, but are not limited to, hydroxyethyl, 2,2-dihydroxyethyl, 1-hydroxyethyl, 3-hydroxypropyl, 1-hydroxypropyl; 1-methyl-1-hydroxyethyl and 1-methyl-1-hydroxypropyl.

The term "halo C₁₋₇ alkyl", as employed herein, refers to at least one halogen, as defined herein, appended to the parent molecular moiety through a C₁₋₇ alkyl group, as defined herein. Representative examples of halo C₁₋₇ alkyl include, but are not limited to, fluoromethyl, difluoromethyl, trifluoromethyl, 2-chloroethyl and 3-bromopropyl.

The term "cyano C₁₋₇ alkyl", as employed herein, refers to a cyano group, as defined herein, appended to the parent molecular moiety through a C₁₋₇ alkyl group, as defined herein. Representative examples of cyano C₁₋₇ alkyl include, but are not limited to, cyanomethyl, 1-cyanoethyl, 1-cyanopropyl and 2-cyanopropyl.

The term "carboxy C₁₋₇ alkyl", as employed herein as such or as part of another group, refers to a carboxy group, as defined herein, appended to the parent molecular moiety through a C₁₋₇ alkyl group, as defined herein.

The term "halo C₁₋₇ alkoxy", as employed herein, refers to at least one halogen, as defined herein, appended to the parent molecular moiety through a C₁₋₇ alkoxy group, as defined herein.

The term "phenyl C₁₋₇ alkoxy", as employed herein, refers to at least one phenyl group appended to the parent molecular moiety through a C₁₋₇ alkoxy group, as defined herein.

The term "C₁₋₇ alkylcarbonyl", as employed herein as such or as part of another group, refers to a C₁₋₇ alkyl group, as defined herein, appended to the parent molecular moiety through a carbonyl group, as defined herein.

The term "C₁₋₇ alkoxycarbonyl", as employed herein as such or as part of another group, refers to a C₁₋₇ alkoxy group, as defined herein, appended to the parent molecular moiety through a carbonyl group, as defined herein.

The term "C₁₋₇ alkoxycarbonyl C₁₋₇ alkyl", as employed herein as such or as part of another group, refers to a C₁₋₇ alkoxycarbonyl group, as defined herein, appended to the parent molecular moiety through a C₁₋₇ alkyl group, as defined herein.

The term "aminocarbonyl", as employed herein as such or as part of another group, refers to an amino group appended to the parent molecular moiety through a carbonyl group, as defined herein.

The term "amino C₁₋₇ alkyl", as employed herein, refers to at least one amino group appended to the parent molecular moiety through a C₁₋₇ alkyl group, as defined herein. Representative examples of amino C₁₋₇ alkyl include, but are not limited to, aminomethyl, 2-aminoethyl, 1-aminoethyl, 2,2-diaminoethyl, 3-aminopropyl, 2-aminopropyl, 4-aminobutyl and 1-methyl-1-aminoethyl.

The term "C₁₋₇ alkylamino", as employed herein as such or as part of another group, refers to at least one C₁₋₇ alkyl group, as defined herein, appended to the parent molecular moiety through an amino group. Representative examples of C₁₋₇ alkylamino include, but are not limited to methylamino, ethylamino, propylamino, butylamino, dimethylamino, diethylamino and *N*-ethyl-*N*-methylamino.

The term "C₁₋₇ alkylamino C₁₋₇ alkyl", as employed herein as such or as part of another group, refers to at least one C₁₋₇ alkylamino group, as defined herein, appended to the parent molecular moiety through an C₁₋₇ alkyl group, as defined herein.

The term "carboxy C₁₋₇ alkylamino", as employed herein as such or as part of another group, refers to at least one carboxy group, as defined herein, appended to the parent molecular moiety through an C₁₋₇ alkylamino group, as defined herein

The term "C₁₋₇ alkoxy C₁₋₇ alkyl"; as employed herein, refers to at least one C₁₋₇ alkoxy group, as defined herein, appended to the parent molecular moiety through an C₁₋₇ alkyl group, as defined herein.

The term "C₁₋₇ alkoxycarbonyl C₁₋₇ alkyl", as employed herein, refers to at least one C₁₋₇ alkoxycarbonyl group, as defined herein, appended to the parent molecular moiety through an C₁₋₇ alkyl group, as defined herein.

The term "substituted" as used herein in connection with various residues refers to halogen substituents, such as fluorine, chlorine, bromine, iodine, or C₁₋₇ alkyl, C₃₋₇ cycloalkyl, halo C₁₋₇ alkyl, hydroxy, amino, C₁₋₇ alkoxy, C₁₋₇ acyl C₁₋₇ alkylamino, amino C₁₋₇ alkyl, nitro, cyano, thiol or methylsulfonyl substituents. Preferred are halogen, C₁₋₇ alkyl, halo C₁₋₇ alkyl, hydroxy, amino, C₁₋₇ alkoxy and methylsulfonyl substituents. Particularly preferred are 1 to 3 of C₁₋₃ alkyl substituents.

The "substituted" groups may contain 1 to 3, preferably 1 or 2, of the above mentioned substituents.

The term "5 - 6 membered heterocyclic ring" as employed herein, refers to a saturated, partially saturated or aromatic ring with 5 or 6 ring atoms, of which 1-4 atoms are heteroatoms selected from a group consisting of N, O and S. Representative examples of 5-6-membered heterocyclic ring include, but are not limited to, pyrazolyl, 1,2,4-triazol-1-yl, 1,2,3-triazol-1-yl, pyrimidinyl, pyridinyl, tetrazolyl, piperazinyl, furanyl, triorpholinyl; piperidinyl, pyrrolidinyl, thiazolyl, isoxazolyl, pyrazinyl tetrahydropyranyl, 1,2,4-oxadiazolyl, oxazolyl, imidazolyl, indolyl and 4,5-dihydroimidazolyl rings.

The term "5 - 12 membered heterocyclic ring" as employed herein, refers to a monocyclic or bicyclic saturated, partially saturated or aromatic ring with 5 to 12 ring atoms, of which 1-5 atoms are heteroatoms selected from a group consisting of N, O and S. Representative examples of 5 - 12 membered heterocyclic ring include the examples given above and additionally, but not limited to, indazolyl, pyrazolo[1,5-a]pyrimidinyl, benzo[d]imidazolyl, imidazo[4,5-b]pyridinyl, 4,5,6,7-tetrahydrobenzo[d]imidazolyl and benzofuranyl rings.

The term "5 - 12 membered carbocyclic ring" as employed herein, refers to a saturated, partially saturated or aromatic ring with 5 to 12 ring atoms consisting of carbon atoms only. Representative examples of 5 - 12 membered carbocyclic ring include, but are not limited to, phenyl, naphtyl and cyclohexyl rings.

The definition of formula (I) above is inclusive of all the possible isotopes and Z₂ isomers, such as stereoisomers, of the compounds, including geometric isomers, for example Z and E isomers (*cis* and *trans* isomers), and optical isomers, such as diastereomers and enantiomers, and prodrug esters, e.g. phosphate esters and carbonate esters.

It will be appreciated by those skilled in the art that the present compounds may contain at least one chiral center. Accordingly, the compounds may exist in optically active or racemic forms. It is to be understood that the formula (I) includes any racemic or optically active form, or mixtures thereof. In one embodiment, the compounds are the pure (R)-isomers. In another embodiment, the compounds are the pure (S)-isomers. In another embodiment, the compounds are a mixture of the (R) and the (S) isomers. In another embodiment, the compounds are a racemic mixture comprising an equal amount of the (R) and the (S) isomers. The compounds may contain two chiral centers. In such case, according to one embodiment, the compounds of the invention are pure diasteromers. According to other embodiment, the compounds are a mixture of several diasteromers. The individual isomers may be obtained using the corresponding isomeric forms of the starting material or they may be separated after the preparation of the end compound according to conventional separation methods. For the separation of optical isomers, e.g. enantiomers or diastereomers, from the mixture thereof the conventional resolution methods, e.g. fractional crystallisation, may be used.

The present compounds may also exist as tautomers or equilibrium mixtures thereof wherein a proton of a compound shifts from one atom to another. Examples of tautomers include, but are not limited to, amido-imido, keto-enol, phenol-keto, oxime-nitroso, nitro-aci, imine-enamine and the like. Tautomeric forms of compounds of formula (I) are intended to be encompassed by compounds formula (I) even though only one tautomeric form may be depicted. For example, compounds of formula (Ib') 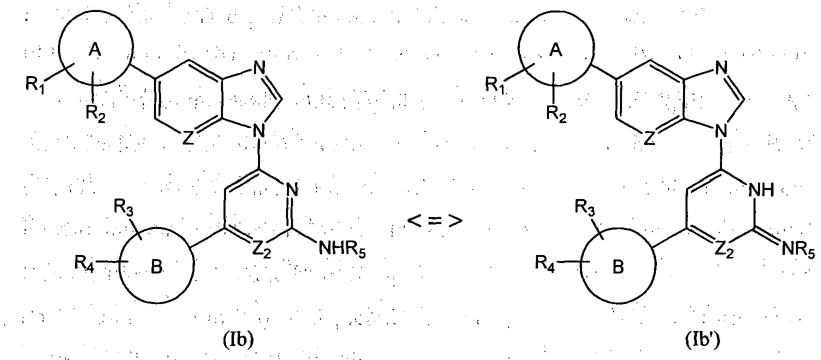 wherein R₁, R₂, R₃, R₄, R₅, Z, Z₂ and rings A and B are as defined above, are imido tautomers of amido compounds of formula (Ib) and are, therefore, within the scope of formula (I) as defined herein.

Examples of preferred compounds of formula (I) include
4-(2,4-Difluorophenyl)-N-(1-methyl-1H-pyrazol-3-yl)-6-(5-(1-methyl-1H-pyrazol-4-yl)-1H-benzo[d]imidazol-1-yl) pyridin-2-amine;
N-(4-(2,4-difluorophenyl)-6-(5-(1-methyl-1H-pyrazol-4-yl)-1H-benzo[d]-imidazol-1-yl)pyridin-2-yl)cyclopropanesulfonamide;
Sodium salt of imido form of N-(4-(2,4-difluorophenyl)-6-(5-(1-methyl-1H-pyrazol-4-yl)-1H-benzo[d]imidazol-1-yl)pyridin-2-yl)cyclopropanesulfonamide;
N-(4-(2,4-difluorophenyl)-6-(5-(1-methyl-1H-pyrazol-4-yl)-1H-benzo[d]-imidazol-1=yl)pyridin-2-yl)methanesulfonamide;
N-(4-(2,4-difluorophenyl)-6-(5-(1-methyl-1H-pyrazol-4-yl)-1H-benzo[d]-imidazol-1-yl)pyridin-2-yl)ethanesulfonamide;
Sodium salt of imido form of N-(4-(2,4-difluorophenyl)-6-(5-(1-methyl-1H-pyrazol-4-yl)-1H-benzo[d]imidazol-1-yl)pyridin-2-yl)ethanesulfonamide;
N-(4-'(2,4-difluorophenyl)-6-(5-(1-methyl-1H-pyrazol-4-yl)-1H-benzo[d]-imidazol-1-yl)pyridin-2-yl)propane-2-sulfonamide;
Imido form of N-(4-(2,4-difluorophenyl)-6-(5-(1-methyl-1H-pyrazol-4-yl)-1H-benzo[d]imidazol-1-yl)pyridin-2-yl)propane-2-sulfonamide;
N-(4-(2-fluorophenyl)-6-(5-(1-methyl-1H-pyrazol-4-yl)-1H-benzo[d]imidazol-1-yl)pyridin-2-yl)cyclopropanesulfonamide;
Sodium salt of imido form of N-(4-(2-fluorophenyl)-6-(5-(1-methyl-1H-pyrazol-4-yl)-1H-benzo[d]imidazol-1-yl)pyridin-2-yl)cyclopropanesulfonamide;
N-(4-(2-fluorophenyl)-6-(5-(1-methyl-1H-pyrazol-4-yl)-1H-benzo[d]imidazol-1-yl)pyridin-2-yl)methanesulfonamide;
N-(4-(2-fluorophenyl)-6-(5-(1-methyl-1H-pyrazol-4-yl)-1H-benzo[d]imidazol-1-yl)pyridin-2-yl)ethanesulfonamide;
Sodium salt of imido form of N-(4-(2-fluorophenyl)-6-(5-(1-methyl-1H-pyrazol-4-yl)-1H-benzo[d]imidazol-1-yl)pyridin-2-yl)ethanesulfonamide;
N-(4-(2-fluorophenyl)-6-(5-(1-methyl-1H-pyrazol-4-yl)-1H-benzo[d]imidazol-1-yl)pyridin-2-yl)propane-2-sulfonamide;
Sodium salt of imido form of N-(4-(2-fluorophenyl)-6-(5-(1-methyl-1H-pyrazol-4-yl)-1H-benzo[d]imidazol-1-yl)pyridin-2-yl)propane-2-sulfonamide;
N-(4-(4-fluorophenyl)-6-(5-(1-methyl-1H-pyrazol-4-yl)-1H-benzo[d]imidazol-1-yl)pyridin-2-yl)cyclopropanesulfonamide;
N-(4-(4-fluorophenyl)-6-(5-(1-methyl-1H-pyrazol-4-yl)-1H-benzo[d]imidazol-1-yl)pyridin-2-yl)methanesulfonamide;
N-(4-(4-fluorophenyl)-6-(5-(1-methyl-1H-pyrazol-4-yl)-1H-benzo[d]imidazol-1-yl)pyridin-2-yl)propane-2-sulfonamide;
Sodium salt of imido form of N-(4-(4-fluorophenyl)-6-(5-(1-methyl-1H-pyrazol-4-yl)-1H-benzo[d]imidazol-1-yl)pyridin-2-yl)propane-2-sulfonamide;
N-(3-fluoro-6'-(5-(1-methyl-1H-pyrazol-4-yl)-1H-benzo[d]imidazol-1-yl)-[2,4'-bipyridin]-2'-yl)cyclopropanesulfonamide;
N-(3-fluoro-6'-(5-(1-methyl-1H-pyrazol-4-yl)-1H-benzo[d]imidazol-1-yl)-[2,4'-bipyridin]-2'-yl)acetamide;
N-(4-(2,4-difluorophenyl)-6-(5-(1-(2-(dimethylamino)ethyl)-1H-pyrazol-4-yl)-1H-benzo[d]imidazol-1-yl)pyridin-2-yl)cyclopropanesulfonamide;
N-(4-(2-fluorophenyl)-6-(5-(1-methyl-1H-pyrazol-4-yl)-1H-benzo[d]imidazol-1-yl)pyrimidin-2-yl)cyclopropanesulfonamide;
N-(6-(5-(1H-pyrazol-1-yl)-1H-benzo[d]imidazol-1-yl)-4-(2,4-difluorophenyl)-pyridin-2-yl)cyclopropanesulfonamide;
N-(3,5-difluor6-6'-(5-(1-methyl-1H-pyrazol-4-yl)-1H-benzo[d]imidazol-1-yl)-[2,4'-bipyridin]-2'-yl)cyclopropanesulfonamide;
N-(3,5-difluoro-6'-(5-(1-methyl-1H-pyrazol-4-yl)-1H-benzo[d]imidazol-1-yl)-[2,4'-bipyridin]-2'-yl)acetamide;
N-(4-(2-chlorophenyl)-6-(5-(1-methyl-1H-pyrazol-4-yl)-1H-benzo[d]imidazol-1-yl)pyridin-2-yl)cyclopropanesulfonamide;
N-(3-chloro-6'-(5-(1-methyl-1H-pyrazol-4-yl)-1H-benzo[d]imidazol-1-yl)-[2,4'-bipyridin]-2'-yl)cyclopropanesulfonamide;
N-(5-fluoro-6'-(5-(1-methyl-1H-pyrazol-4-yl)-1H-benzo[d]imidazol-1-yl)-[2,4'-bipyridin]-2'-yl)cyclopropanesulfonamide;
N-(6-(5-(1H-imidazol-1-yl)-1H-benzo[d]imidazol-1-yl)-4-(2,4-difluorophenyl)-pyridin-2-yl)cyclopropanesulfonamide;
N-(4-(2,4-difluorophenyl)-6-(5-(1-(2-morpholinoethyl)-1H-pyrazol-4-yl)-1H-benzo[d]imidazol-1-yl)pyridin-2-yl)cyclopropanesulfonamide;
N-(4-(2,4-difluorophenyl)-6-(5-(1-(pyrrolidin-3-yl)-1H-pyrazol-4-yl)-1H-benzo[d]imidazol-1-yl)pyridin-2-yl)cyclopropanesulfonamide;
N-(4-(2,4-difluorophenyl)-6-(5-(1-ethyl-1H-1,2,3-triazol-4-yl)-1H-benzo[d]-imidazol-1-yl)pyridin-2-yl)cyclopropanesulfonamide;
N-(4-(2,4-difluorophenyl)-6-(5-(1-methyl-1H-1,2,3-triazol-4-yl)-1H-benzo[d]-imidazol-1-yl)pyridin-2-yl)cyclopropanesulfonamide;
N-(4-(2,4-difluorophenyl)-6-(5-(1-methyl-1H-imidazol-4-yl)-1H-benzo[d]-imidazol-1-yl)pyridin-2-yl)cyclopropanesulfonamide;
N-(4-(2,4-difluorophenyl)-6-(5-(1-methyl-1H-pyrazol-4-yl)-1H-benzo[d]-imidazol-1-yl) pyrimidin-2-yl) acetamide;
Ethyl 1-(1-(6-(cyclopropanesulfonamido)-4-(2,4-difluorophenyl)pyridin-2-yl)-1H-benzo[d]imidazol-5-yl)-1H-1,2,3-triazole-4-carboxylate;
N-(4-(2-(difluoromethoxy)-4-fluorophenyl)-6-(5-(1-methyl-1H-pyrazol-4-yl)-1H-benzo[d]imidazol-1-yl)pyridin-2-yl)cyclopropanesulfonamide;
N-(4-(2,4-difluorophenyl)-6-(5-(1-methyl-1H-pyrazol-4-yl)-1H-benzo[d]-imidazol-1-yl)pyrimidin-2-yl)cyclopropanesulfonamide;
N-(6-(2, 4-difluorophenyl)-4-(5-(1-methyl-1H-pyrazol-4-yl)-1H-benzo[d]-imidazol-1-yl) pyridin-2-yl) cyclopropanesulfonamide;
or a pharmaceutically acceptable salt or tautomer thereof.

Compounds of the invention may be administered to a patient in therapeutically effective amounts which range usually from about 0.1 to about 5000 mg, preferably from about 1 to about 2000 mg, per day depending on the age, weight, ethnic group, condition of the patient, condition to be treated, administration route and the active ingredient used. The compounds of the invention can be formulated into dosage forms using the principles known in the art. The compound can be given to a patient as such or in combination with suitable pharmaceutical excipients in the form of tablets, granules, capsules, suppositories, emulsions, suspensions or solutions. Choosing suitable ingredients for the composition is a routine for those of ordinary skill in the art. Suitable carriers, solvents, gel forming ingredients, dispersion forming ingredients, antioxidants, colours, sweeteners, wetting compounds and other ingredients normally used in this field of technology may be also used. The compositions containing the active compound can be given enterally or parenterally, the oral route being the preferred way. The contents of the active compound in the composition is from about 0.5 to 100 %, preferably from about 0.5 to about 20 %, per weight of the total composition.

The compounds of the invention can be given to the subject as the sole active ingredient or in combination with one of more other active ingredients for treatment of a particular disease, for example cancer.

The present invention will be explained in more detail by the following experiments and examples. The experiments and examples are meant only for illustrating purposes and do not limit the scope of the invention defined in claims.

### EXPERIMENTS

### 1. Inhibition of FGFR1 kinase

### FGFR1 assay

Compounds were screened in the TR-FRET assay with FGFR1 kinase. 5 ng of FGFR1 [Upstate, USA] kinase was used for assay. The compound was incubated with the kinase for 30 minutes at room temperature. After the incubation, substrate mix [40 nM Ultra light poly GT (Perkin Elmer, USA) and 13 µM ATP (Sigma)] was added. The above reaction was stopped by the addition of 40mM EDTA after the 30 min kinase reaction. The Eu-labelled antiphospho-tyrosine antibody [Perkin Elmer, USA] was added at 0.5 nM and the fluorescence emission at 615 nm/665 nm [excitation at 340 nm] was measured. The compounds were initially screened at 100 nM and 1 µM concentrations. The compounds with >50 % inhibition at 100 nM of FGFR1 were taken for the full dose response studies. The final DMSO concentration in the assay was 1 %. For IC₅₀ determination, 1/3^{rd} serial dilution was made from the 20 mM DMSO stock solution. 2 µl of these were transferred to the test wells containing 20 µl of the reaction mixture [total reaction volume 20 µl]. The fluorescence was measured in Perkin Elmer Wallac 1420 Multilabel Counter Victor 3. The IC₅₀ was determined by fitting the dose response data to sigmoidal curve fitting equation using GraphPad Prism software V5.

### Results

Enzymatic activity and selectivity of selected compounds of the invention on different kinases is presented in Table 1. The compounds of the invention were found to be potent and selective FGFR kinase inhibitors.

**TABLE 1. Inhibition of FGFR1 kinase**

| **Compound** | **Inhibition (%) of FGFR1 at 1000 nM** | **IC₅₀ of FGFR1 inhibition (nM)** |
|---|---|---|
| Example 1 | 97 | 16.5 |
| Example 2 | 99 | 3.9 |
| Example 2 (imido) | 97 | 3.3 |
| Example 4 | 98 | 4.3 |
| Example 5 | 99 | 1.1 |
| Example 5 (imido) | 98 | 4.4 |
| Example 6 | 99 | 1.7 |
| Example 6 (imido) | 98 | 1.7 |
| Example 8 | 99 | 5.42 |
| Example 8 (imido) | 97 | 3.3 |
| Example 10 | 97 | 5.8 |
| Example 11 | 98 | 2.8 |
| Example 11 (imido) | 98 | 3.2 |
| Example 12 | 97 | 1.5 |
| Example 12 (imido) | 99 | 5.2 |
| Example 14 | 98 | 14.4 |
| Example 16 | 97 | 3.4 |
| Example 17 | 96 | 4.6 |
| Example 17 (imido) | 97 | 5.2 |
| Example 18 | 97 | 5 |
| Example 19 | 96 | 28.5 |
| Example 21 | 99 | 3.7 |
| Example 22 | 97 | 9.2 |
| Example 23 | 97 | 10 |
| Example 25 | 97 | 3.1 |
| Example 26 | 94 | 21.9 |
| Example 28 | 97 | 7.2 |
| Example 29 | 97 | 9.7 |
| Example 30 | 96 | 24 |
| Example 32 | 99 | 21.7 |
| Example 33 | 99 | 2 |
| Example 34 | 99 | 1.6 |
| Example 35 | 99 | 2 |
| Example 36 | 99 | 4.7 |
| Example 37 | 99 | 1.3 |
| Example 38 | 87 | 12 |
| Example 39 | 81 | 294.2 |
| Example 40 | 76 | 293.5 |
| Example 41 | 93 | 32.7 |
| Example 43 | 94 | 43.4 |

The preparation of the compounds of the invention is illustrated by the following Examples.

### EXAMPLES.

LCMS data has been recorded in +ve mode unless otherwise mentioned.

### Intermediate Example 1.

### 4-(1-Methyl-1H-pyrazol-4-yl)-2-nitroaniline

A solution of 4-bromo-2-nitroaniline (6 g, 27.6 mmol) in 1, 2-dimethoxyethane (15 ml) was degassed by N₂ bubbling for 5 min. 1-Methyl-4-(4,4,5,5-tetramethyl-1,3,2-dioxaborolan-2-yl)-1H-pyrazole (6.90 g, 33.1 mmol, 1.2 eq) was added and the mixture was degassed for another 5 min. Pd(dppf)Cl₂ (2.25 g, 27.6 mmol, 0.1 eq) and aqueous sodium carbonate (8.79 g, 82.9 mmol, 3.0 eq) were added sequentially and the mixture was further degassed for 5 min and then heated at 90 °C for 2 h. The reaction mixture was quenched with water and extracted with ethyl acetate (3 × 50 ml). The combined organic layer was washed with water, brine and dried over sodium sulphate. The solvent was distilled off under reduced pressure to afford the crude residue which was purified by column chromatography (60-120 silica gel, 40 % ethyl acetate in hexane) to afford the title product in 75 % yield (4.5 g). LC-MS (ESI): Calculated mass: 218.21; Observed mass: 218.9 [M+H]⁺ (rt: 0.390 min).

### Intermediate Example 2.

### tert-Butyl 3-(4-(1H-benzo[d]imidazol-5-yl)-1H-pyrazol-1-yl)pyrrolidine-1-carboxylate

### a) tert-Butyl 3-(methylsulfonyl) pyrrolidine-1-carboxylate

To a solution of tert-butyl 3-hydroxypyrrolidine-1-carboxylate (0.5 g, 2.67 mmol, 1.0 eq) in DCM (10 ml) was added TEA (0.8 ml, 5.35 mmol, 2.0 eq). The mixture was stirred at RT for 15 min. Then methanesulfonyl chloride (0.23 ml, 2.94 mmol 1.1 eq) was added and the mixture was stirred for 3 h. The mixture was then quenched with water and extracted with CH₂Cl₂ (3 × 50 ml). The solvent was distilled off to afford the title product in 86 % yield (0.6 g).

### b) tert-butyl 3-(4-(4, 4, 5, 5-tetramethyl-1, 3, 2-dioxaborolan-2-yl)-1H-pyrazol-1-yl) pyrrolidine-1-carboxylate

To a solution of 4 4-(4,4,5,5-tetramethyl-1,3,2-dioxaborolan-2-yl)-1H-pyrazole (0.6 g, 2.26 mmol) in DMF were added sodium hydride at 0 °C (0.108 g, 4.53 mmol, 2 eq) and the compound of Intermediate Example 2(a) (0.44 g, 2.26 mmol, 1.0 eq.). The mixture was stirred at RT for 1 h and heated at 90 °C for 4 h and quenched with ice and extracted as in Intermediate Example 1. The solvent was distilled off to afford the crude residue in 50 % yield (0.4 g).

### c) tert-Butyl 3-(4-(1H-benzo[d]imidazol-5-yl)-1H-pyrazol-1-yl)pyrrolidine-1-carboxylate

To a degassed (N₂ bubbling) solution of 5-bromo-1H-benzo[d]imidazole (0.050 g, 0.15 mmol) in 1,4-dioxane (10 ml) were added the compound of Intermediate Example 2(b) (0.052 g, 0.15 mmol, 1 eq), Pd(PPh₃)₄ (16 mg, 0.015 mmol, 0.1 eq) and cesium carbonate (0.118 g, 0.36 mmol, 2.5 eq) using the procedure of Intermediate Example 1. The mixture was heated at 90 °C for 16 h. The reaction mixture was quenched and extracted as in Intermediate Example 1. The solvent was distilled off to afford the product in 40 % yield (20 mg). LC-MS (ESI): Calculated mass: 353.2; Observed mass: 354.4 [M+H]⁺ (rt: 0.11 min).

### Intermediate Example 3.

### 4-(1-(2-(Dimethylamino)ethyl)-1H-pyiazol-4-yl)-2-nitroaniline

### a) 2-(4-iodo-1H-pyrazol-1-yl)-N,N-dimethylethanamine

To a solution of 4-iodo-1H-pyrazole (2.8 g, 10 mmol) in acetonitrile (50 ml) were added cesium carbonate (5.04 g, 15 mmol, 1.5 eq) and 2-chloro-*N,N-*dimethyl-ethanamine hydrochloride (2.96 g, 20 mmol, 2 eq) and the mixture was stirred at RT for 8 h. The mixture was quenched with water and extracted with EtOAc (3 × 150 ml). The combined organic layer was washed with water, brine and dried over sodium sulphate. The solvent was distilled off to afford the crude product in 38 % yield (1 g).

### b) 4-(1-(2-(dimethylamino)ethyl)-1H-pyrazol-4-yl)-2-nitroaniline

A solution of the compound of Example 3(a) (0.5 g, 1.9 mmol) in 1, 2-dimethoxyethane (15 ml) was degassed by N₂ bubbling for 5 min. 2-Nitro-4-(4,4,5,5-tetramethyl-1,3,2-dioxaborolan-2-yl)aniline (0.74 g, 2.8 mmol, 1.5 eq) was added and the mixture was degassed for another 5 min. Pd(dppf)Cl₂ (0.16g, 0.2 mmol, 0.1 eq) and aqueous sodium carbonate (0.5 g, 4.7 mmol, 2.5 eq) were added sequentially using the procedure of Intermediate Example 1 and the mixture was heated at 90 °C for 16 h. The reaction mixture was quenched and extracted as in Intermediate Example 1. The solvent was distilled off to afford the crude residue which was purified by column chromatography (60-120 silica gel, 70 % ethyl acetate in hexane) to afford the title product in 65 % yield (0.3 g). LC-MS (ESI): Calculated mass: 275.14; Observed mass: 276.15 [M+H]⁺ (rt: 0.18 min).

### Intermediate Example 4.

### 5-(1H-Pyrazol-1-yl)-1H-benzo[d]imidazole

### a) 2-nitro-4-(1H-pyrazol-1-yl)aniline

To a solution of 4-bromo-2-nitroaniline (3 g, 13.8 mmol) in DMF (12 ml) were added pyrazole (1.12 g, 16.4 mmol, 1.2 eq.), copper(I) oxide (0.1 g, 0.69 mmol, 0.05 eq.) and cesium carbonate (8 g, 24.6 mmol, 1.8 eq.) and the mixture was heated at 100 °C overnight. The mixture was quenched and extracted as in Intermediate Example 1. The solvent was distilled off and the crude residue was purified by column chromatography (60-120 silica gel, 20 % ethyl acetate in hexane) to afford the title product in 53.5% yield (1:5 g).

### b) 5-(1H-pyrazol-1-yl)-1H-benzo[d]imidazole

A mixture of the compound of Example 4(a) (0.5 g, 2.5 mmol) and iron powder (1.39 g, 25 mmol, 10 eq) were refluxed in formic acid (20 ml) overnight. The formic acid was distilled off and the crude product was dissolved in ethyl acetate and filtered. The ethyl acetate layer was washed with water, brine and dried over sodium sulphate. The solvent was distilled off to afford the title product in 87 % yield (0.4 g). LC-MS (ESI): Calculated mass: 184.07; Observed mass: 185.3 [M+H]⁺ (rt: 0.097 min).

### Intermediate Example 5.

### N-(6-chloro-4-(2,4-difluorophenyl)pyridin-2-yl)cyclopropanesulfonamide

### a) 2,6-dichloro-4-(2,4-difluorophenyl)pyridine

A solution of 2,6-dichloro-4-iodopyridine (1.5 g, 5.49 mmol) in 1,2-dimethoxyethane (15 ml) was degassed by N₂ bubbling for 5 min. 2,4-Difluorophenylboronic acid (0.86 g; 5.49 mmol, 1 eq) was added and the mixture was degassed for another 5 min. Pd(dppf)Cl₂ (0.358 g, 0.43 mmol, 0.08 eq) and aqueous sodium carbonate (1.45 g, 13.7 mmol, 2.5 eq) were added sequentially using the procedure of Intermediate Example 1 and the mixture was heated at 90 °C for 16 h. The reaction mixture was quenched and extracted as in Intermediate Example 1. The solvent was distilled off to afford the crude residue which was purified by column chromatography (60-120 silica gel, 3 % ethyl acetate in hexane) to yield the title product in 98.5 % yield (1.4 g).

### b) N-(6-chloro-4-(2,4-difluorophenyl)pyridin-2-yl)cyclopropanesulfonamide

A solution of the compound of Intermediate Example 5(a) (0.9 g, 3.46 mmol) in dioxane (20 ml) was degassed by N₂ bubbling for 5 min. Cyclopropane sulfonamide (0.36 g, 2.94 mmol, 1 eq) was added and, the mixture was degassed for another 5 min. Palladium acetate (39 mg, 0.173 mmol, 0.05 eq) and xantphos (200 mg, 0.35 mmol, 0.1 eq) and Cs₂CO₃ (3.37 g, 10.4 mmol, 3.0 eq) were added and the mixture was further degassed for 5 min and then heated at 100 °C for 16 h. The mixture was filtered through celite and extracted with ethyl acetate (3 × 50 ml). The combined organic layer was washed with water, brine and dried over sodium sulphate. The solvent was distilled off to afford the crude residue which was purified by column chromatography (60-120 silica gel, 30 % ethyl acetate in hexane) to afford the title product in 50 % yield (0.6 g). ¹H NMR (300 MHz, DMSO-*d₆*): δ 11.05 (s, 1H), 7.76-7.68 (m, 1H), 7.51-7.43 (m, 1H), 7.35 (s, 1H), 7.30-7.24 (m, 1H), 7.16 (s, 1H), 3.09-3.01 (m, 1H), 1.22-1.09 (m, 2H), 1.08-1.03 (m, 2H).

### Intermediate Example 6.

### 5-(1-Methyl-1H-pyrazol-4-yl)-1H-benzo[d]imidazole

A mixture of the compound of Intermediate Example 1 (3 g, 13.7 mmol) and iron powder (7.68 g, 137 mmol, 10 eq) were refluxed in formic acid (50 ml) overnight. The formic acid was distilled off and the crude product was dissolved in ethyl acetate and filtered. The ethyl acetate layer was washed with water, brine and dried over sodium sulphate. The solvent was distilled off to afford the title product in 55.5 % yield (1.5 g). LC-MS (ESI): Calculated mass: 198.09; Observed mass: 199.2 [M+H]⁺ (rt: 0.097 min).

### Intermediate Example 7.

### 5-(1-Methyl-1H-pyrazol-4-yl)-3-nitropyridin-2-amine

A solution of 5-bromo-3-nitropyridin-2-amine (5 g, 23 mmol) in 1, 2-dimethoxy-ethane (50 ml) was degassed by N₂ bubbling for 5 min. 1-Methyl-4-(4,4,5,5-tetramethyl-1,3,2-dioxaborolan-2-yl)-1H-pyrazole (7.2 g, 37 mmol, 1.5 eq) was added and the mixture was degassed for another 5 min. Pd(dppf)Cl₂ (1.88 g, 2.3 mmol, 0.1 eq) and aqueous sodium carbonate (6.1 g, 52 mmol, 2.5 eq) were added sequentially using the procedure of Intermediate Example 1 and the mixture was heated at 90 °C for 16 h. The reaction mixture was quenched and extracted as in Intermediate Example 1. The solvent was distilled off to afford the crude residue which was purified by column chromatography (60-120 silica gel, 70 % ethyl acetate in hexane) to afford the title product in 50 % yield (2.5 g). LC-MS (ESI): Calculated mass: 219.08; Observed mass: 220.1 [M+H]⁺ (rt: 0.22 min).

### Intermediate Example 8.

### 4-(1-(2-Morpholinoethyl)-1H-pyrazol-4-yl)-2-nitroaniline

### a) 4-(2-(4-bromo-1H-pyrazol-1-yl)ethyl)morpholine

To a solution of 4-(2-chloroethyl)morpholine (2.55 g, 13.6 mmol) and 4-bromo-1H-pyrazole (2 g, 13.6 mmol, 1 eq) in DMF (50 ml) was added K₂CO₃ (0.16 g, 6.72 mmol, 1.5 eq) and the mixture was stirred at RT for 24 h. The mixture was then quenched with water and extracted with ethyl acetate (3 × 100 ml). The combined organic layer was washed with water, brine and dried over sodium sulphate. The solvent was distilled off to afford the crude product in 57 % yield (2 g).

### b) 4-(2-(4-(4,4,5,5-tetramethyl-1,3,2-dioxaborolan-2-yl)-1H-pyrazol-1-yl)ethyl)-morpholine

A solution of the compound of Intermediate Example 8(a) (1 g, 3.8 mmol) in DME (15 ml) was degassed by N₂ bubbling for 5 min. Bispinacolato diborane (1.46 g, 5.7 mmol, 1.5 eq) was added and the mixture was degassed for another 5 min. Pd(dppf)Cl₂ (0.313 g, 0.38 mmol, 0.1 eq) and potassium acetate (1.32 g, 13.4 mmol, 3.5 eq) were added sequentially using the procedure of Intermediate Example 1 and the mixture was then heated at 100 °C for 4 h. The reaction mixture was then quenched and extracted as in Intermediate Example 1. The solvent was distilled off to afford the crude residue which was purified by column chromatography (60-120 silica gel, 50 % ethyl acetate in hexane) to afford the title product in 87 % yield (1 g). LC-MS (ESI): Calculated mass: 307.2; Observed mass: 308.5 [M+H]⁺ (rt: 0.10 min).

### c) 4-(1-(2-morpholinoethyl)-1H-pyrazol-4-yl)-2-nitroaniline

A solution of the compound of Intermediate Example 8(b) (1 g, 4.6 mmol) in 1,2-dimethoxyethane (15 ml) was degassed by N₂ bubbling for 5 min. 4-Bromo-2-nitroaniline (1.41 g, 4.6 mmol, 1 eq) was added and the mixture was degassed for another 5 min. Pd(dppf)Cl₂ (0.38 g, 0.46 mmol, 0.1 eq) and aqueous sodium carbonate (1.41 g, 13.8 mmol, 3 eq) were added sequentially using the procedure of Intermediate Example 1 and the mixture was then heated at 100 °C for 4 h. The reaction mixture was then quenched and extracted as in Intermediate Example 1. The solvent was distilled off to afford the crude residue which was purified by column chromatography (60-120 silica gel, 50 % ethyl acetate in hexane) to afford the title product in 42 % yield (0.6 g). LC-MS (ESI): Calculated mass: 317.15; Observed mass: 318.05 [M+H]⁺ (rt: 0.20 min).

### Intermediate Example 9.

### 4-(1H-Imidazol-1-yl)-2-nitroaniline

To a solution of 4-bromo-2-nitroaniline (3 g, 13.8 mmol) in DMF (12 ml) were added imidazole (2.71 g, 27.6 mmol, 2 eq), copper(I) oxide (0.1 g, 0.69 mmol, 0.05 eq. and cesium carbonate (13.4 g, 41.4 mmol, 3 eq) and the mixture was heated at 100 °C overnight. The mixture was quenched and extracted as in Intermediate Example 1. The solvent was distilled off and the crude residue was purified by column chromatography (60-120 silica gel, 50 % ethyl acetate in hexane) to afford the title product in 40 % yield (1.1 g). ¹H NMR (300 MHz, DMSO-*d₆*): δ 8.17 (br s, 1H), 8.12 (d, 1H), 7.73-7.68 (m, 2H), 7.59 (s, 2H), 7.15 (d, 1H), 7.08 (s, 1H).

### Intermediate Example 10.

### 5-(1-Ethyl-1H-1,2,3-triazol-4-yl)-1H-benzo[d]imidazole

### a) 2-Nitro-4-((trimethylsilyl)ethynyl)aniline

A solution of the compound of 4-iodo-2-nitroaniline (1 g, 3.8 mmol) in DMF-Et₃N (1:1; 20 ml) was degassed by N₂ bubbling for 15 min. Pd(PPh₃)₄ (0.22 g, 0.19 mmol, 0.05 eq.), copper(I) iodide (0.073g, 0.386 mmol, 0.1 eq.) and ethynyltrimethyl-silane (0.45 ml, 4.63 mmol, 1.2 eq.) were added sequentially and the mixture was stirred for 12 h at RT. The mixture was quenched and extracted as in Intermediate Example 1. The solvent was distilled off and the crude residue was purified by column chromatography (60-120 silica gel, 10 % ethyl acetate in hexane) to afford the title product in 67 % yield (0.6 g).

### b) 4-Ethynyl-2-nitroaniline

To a solution of the compound of Intermediate Example 10(a) (0.5 g, 2.15 mmol) in THF (10 ml) at 0°C was added TBAF (0.5 g, 2.17 mmol, 1.2 eq.) and the mixture was stirred for 0.5 h. The mixture was filtered over a pad of silica and distilled to afford the title product in 86 % yield (0.3 g).

### c) 4-(1-Ethyl-1H-1,2,3-triazol-4-yl)-2-nitroaniline

A mixture of the compound of Intermediate Example 10(b) (0.3 g, 1.85 mmol), sodium azide (0.24 g, 3.7 mmol, 1.0 eq.), methyl iodide (0.23 g, 1.85 mmol, 1.0 eq.), sodium ascorbate (0.36 g, 1.85 mmol, 1.0 eq.) and copper sulfate pentahydrate (0.23 g, 0.92 mmol), 0.5 eq.) in DMSO, THF and water (1:1:0.5, 5 ml) was stirred for 12 h at RT. The mixture was quenched with water and the precipitate formed was filtered and dried. The crude product was purified by by column chromatography (60-120 silica gel, 30 % ethyl acetate in hexane) to afford the title product in 25 % yield (100 mg).

### d) 5-(1-Ethyl-1H-1,2,3-triazol-4-yl)-1H-benzo[d]imidazole

A solution of the compound of Intermediate Example 10(c) (0.1 g, 0.42 mmol) in formic acid (2 ml), iron (0.23 g, 4.2 mmol) was added and heated at 100 °C for 16 h. The formic acid was distilled off under reduced pressure and the crude product was dissolved in ethyl acetate. The ethyl acetate layer was washed with water, brine and dried over sodium sulphate. The solvent was distilled off to afford the title product in 33 % yield (30 mg). LC-MS (ESI): Calculated mass: 213.1; Observed mass: 214.1 [M+H]⁺ (rt: 0.14 min).

### Intermediate Example 11.

### 5-(1-Methyl-1H-imidazol-4-yl)-1H-benzo[d]imidazole

### a) 4-(1-Methyl-1H-imidazol-4-yl)-2-nitroaniline

A solution of 2-nitro-4-(4,4,5,5-tetramethyl-1,3,2-dioxaborolan-2-yl)aniline (1.45 g, 5.55 mmol, 1.1 eq) in 1,2-dimethoxyethane (15 ml) was degassed by N₂ bubbling for 5 min. 4-Bromo-1-methyl-1H-imidazole (0.81 g, 5 mmol, 1 eq) was added and the mixture was degassed for another 5 min. Pd(dppf)Cl₂ (0.4 g, 0.5 mmol, 0.1 eq) and aqueous sodium carbonate (1.59 g, 15 mmol, 3 eq) were added sequentially using the procedure of Intermediate Example 1 and then heated at 100 °C for 4 h. The reaction mixture was then quenched and extracted as in Intermediate Example 1. The solvent was distilled off to afford the crude residue which was purified by column chromatography (60-120 silica gel, 50 % ethyl acetate in hexane) to afford the title product in 60 % yield (0.6 g). LC-MS (ESI): Calculated mass: 218.08; Observed mass: 219.2 [M+H]⁺ (rt: 0.09 min).

### b) 5-(1-methyl-1H-imidazol-4-yl)-1H-benzo[d]imidazole

To a solution of the compound of Intermediate Example 11(a) (0.3 g, 1.376 mmol) in formic acid (5 ml), iron (0.77 g, 13.76 mmol) was added and the mixture was heated at 90 °C for 12 h. The formic acid was distilled off and the crude product was dissolved in ethyl acetate. The ethyl acetate layer was washed with water, brine and dried over sodium sulphate. The solvent was distilled off to afford the title product in 26 % yield (0.07 g). LC-MS (ESI): Calculated mass: 198.09; Observed mass: 199.2 [M+H]⁺ (rt: 0.10 min).

### Intermediate Example 12.

### 5-((Trimethylsilyl)ethynyl)-1H-benzo[d]imidazole

### a) 5-Iodo-1H-benzo[d]imidazole

To a solution 4-iodo-2-nitroaniline (1 g, 3.7 mmol) in formic acid (10 ml), iron (2.1 g, 37 mmol) was added and heated at 90 °C for 12 h. The formic acid was distilled off and the crude was dissolved in ethyl acetate. The ethyl acetate layer was washed with water, brine and dried over sodium sulphate. The solvent was distilled off to afford the title product in 68 % yield (0.85 g). LC-MS (ESI): Calculated mass: 243.95; Observed mass: 244.8 [M+H]⁺ (rt: 0.173 min).

### b) 5-((Trimethylsilyl)ethynyl)-1H-benzo[d]imidazole

A solution of of the compound of Intermediate Example 12(a) (0.7 g, 2.56 mmol) in DMF-Et₃N (1:1; 10 ml) was degassed by N₂ bubbling for 15 min. Pd(dppf)Cl₂ (0.11 g, 0.14 mmol, 0.05 eq), copper(I) iodide (0.054g, 0.25 mmol, 0.1 eq) and ethynyltri-methylsilane (0.3 g, 3.15 mmol, 1.1 eq) were added sequentially and the mixture was stirred for 12 h at RT. The mixture was quenched and extracted as in Intermediate Example 1. The solvent was distilled off and the crude residue was recrystallized from hexane to afford the title product in 57 % yield (0.35 g). LC-MS (ESI): Calculated mass: 214.09; Observed mass: 215.5 [M+H]⁺ (rt: 0.22 min).

### Intermediate Example 13.

### 4-(1-Methyl-1H-pyrazol-4-yl) benzene-1,2-diamine

To a solution of the compound Intermediate Example 1 (1 g, 4.58 mmol) in THF (10 ml) were added a solution of ammonium chloride (1.486 g, 27.5 mmol, 6 eq) in water (5 ml) and zinc (1.78 g, 27.5 mmol, 6 eq). The reaction mixture was stirred at RT for 6 h and filtered. The filtrate was diluted with water and extracted with ethyl acetate (3 × 100 ml). The combined organic layer was washed with water, brine and dried over sodium sulphate. The solvent was distilled off to afford the crude residue which was purified by column chromatography (60-120 silica gel, 3 % methanol in DCM) to afford the title product in 58 % yield (0.5 g). LC-MS (ESI): Calculated mass: 188.11; Observed mass: 189.0 [M+H]⁺ (rt: 0.113 min).

### Intermediate Example 14.

### Ethyl 1-(1H-benzo[d]imidazol-5-yl)-1H-1,2,3-triazole-4-carboxylate

### a) 1H-benzo[d]imidazol-5-amine

To a solution of 5-nitro-1H-benzo[d]imidazole (5 g, 44.2 mmol) in methanol (100 ml) was added Pd/C and the reaction mixture was stirred at RT for 16 h and filtered. The filtrate was diluted with water and extracted with ethyl acetate (3 × 100 ml). The combined organic layer was washed with water, brine and dried over sodium sulphate. The solvent was distilled off to afford the crude residue which was purified by washing with diethyl ether to afford the title product in 85 % yield (2.5 g). LC-MS (ESI): Calculated mass: 133.06; Observed mass: 134.2 [M+H]⁺ (rt: 0.175 min).

### b) 5-Azido-1H-benzo[d]imidazole

To a solution of the compound of Intermediate Example 14(a) (2 g, 15 mmol) in concentrated HCl (8 ml) at 0 °C was added aqueous solution of NaNO₂ (1.3 g, 18.7 mmol, 1.25 eq) dropwise and the mixture was stirred at 0 °C for 30 min. Then NaN₃ (1.13 g, 18.7 mmol, 1.25 eq) was added at 0 °C and the mixture was stirred for 15 min. The mixture was quenched and extracted as in Intermediate Example 1(a). The solvent was distilled off to afford the product in 75 % yield (1.8 g). LC-MS (ESI): Calculated mass: 159.05; Observed mass: 160.0 [M+H]⁺ (rt: 0.136 min).

### c) Ethyl 1-(1H-benzo[d]imidazol-5-yl)-1H-1,2,3-triazole-4-carboxylate

A mixture of the compound of Intermediate Example 14(b) (1.75 g, 10.99 mmol), ethyl propiolate (1.08 g, 10.99 mmol, 1.0 eq), sodium ascorbate (0.22 g, 1.09 mmol, 0.1 eq) and copper sulfate pentahydrate (30 mg, 0.1 mmol, 0.01 eq) in *t*-butanol and water (1:0.5, 20 ml) was stirred for 48 h at RT. The volatiles were evaporated and the reaction mixture was extracted with 10 % methanol in CH₂Cl₂ (3 × 100 ml). The combined organic layer was washed with water, brine and dried over sodium sulphate and concentrated. The light brown solid obtained (1.6 g, 56.6 % yield) was directly taken for the next step without further purification. LC-MS (ESI): Calculated mass: 257.09; Observed mass: 258.05 [M+H]⁺ (rt: 0.193 min).

### Intermediate Example 15.

### N-(6-chloro-4-(2-(difluoromethoxy)-4-fluorophehyl)pyridin-2-yl)cyclopropane-sulfonamide

### a) 1-Bromo-2-(tlifluoromethoxy)-4-fluorobenzene

To a solution of 2-bromo-5-fluorophenol (3 g, 15.7 mmol) in DMF (5 ml) was added cesium carbonate (7.7 g, 23.56 mmol, 1.5 eq) and sodium chlorodifluoroacetate (6 g, 39.26 mmol, 2.5 eq) and the reaction mixture was heated at 100 °C for 15 h. The reaction mixture was then extracted with water and ethyl acetate (3 × 50 ml). The combined organic layer was washed with water, brine and dried over sodium sulphate. The solvent was distilled off to afford the crude residue which was purified by column chromatography (60-120 silica gel, 10 % ethyl acetate in hexane) in 58 % yield (2.2 g).

### b) 2,6-Dichloro-4-(2-(difluoromethoxy)-4-fluorophenyl)pyridine

A solution of the compound of Intermediate Example 15(a) (2.2 g, 9.13 mmol) in 1,2-dimethoxyethane (50 ml) was degassed by N₂ bubbling for 5 min. 2,6-Dichloro-4-(4,4,5,5-tetramethyl-1,3,2-dioxaborolan-2-yl)pyridine (2.75 g, 10.04 mmol, 1.1 eq) was added and the mixture was degassed for another 5 min. Pd(dppf)Cl₂ (0.74 g, 0.9 mmol, 0.1 eq) and aqueous sodium carbonate (2.9 g, 27.3 mmol, 2.5 eq) were added sequentially using the procedure of Intermediate Example 1 and then heated at 110 °C for 4 h. The reaction mixture was then quenched and extracted as in Intermediate Example 1. The solvent was distilled off to afford the crude residue which was purified by column chromatography (60-120 silica gel, 10 % ethyl acetate in hexane) to afford the title product in 43 % yield (1.2 g). LC-MS (ESI): Calculated mass: 306.98; Observed mass: 307.85 [M+H]⁺ (rt: 2.0 min).

### c) N-(6-chloro-4-(2-(difluoromethoxy)-4-fluorophenyl)pyridin-2-yl)cyclo-propanesulfonamide

A solution of the compound of Intermediate Example 15(b) (0.5 g, 1.62 mmol) in dioxane (10 ml) was degassed by N₂ bubbling for 5 min. Cyclopropane sulfonamide (0.19 g, 1.62 mmol, 1 eq) was added and the mixture was degassed for another 5 min. Palladium acetate (18 mg, 0.08 mmol, 0.05 eq) and xantphos (46 mg, 0.08 mmol, 0.05 eq) and cesium carbonate (1.56 g, 4.8 mmol, 3.0 eq) were added sequentially and the reaction mixture was further degassed for 5 min and then heated at 100 °C for 5 h. The reaction mixture was filtered through celite pad and extracted with ethyl acetate (3 × 50 ml). The combined organic layer was washed with water, brine and dried over sodium sulphate. The solvent was distilled off to afford the crude residue which was purified by column chromatography (60-120 silica gel, 30 % ethyl acetate in hexane) in 47 % yield (0.3 g). LC-MS (ESI): Calculated mass: 392.02; Observed mass: 392.85 [M+H]⁺ (rt: 1.82 min).

### Intermediate Example 16.

### N-(6-chloro-4-(2,4-difluorophenyl)pyridin-2-yl)acetamide

A solution of the compound of Intermediate Example 5(a) (0.5 g, 1.92 mmol) in dioxane (20 ml) was degassed by N2 bubbling for 5 min. Acetamide (0.11 g, 1.92 mmol, 1 eq) was added and the mixture was degassed for another 5 min. Palladium acetate (43 mg, 0.19 mmol, 0.1 eq) and xantphos (222 mg, 0.38 mmol, 0.2 eq) and Cs₂CO₃ (1.88 g, 5.76 mmol, 3.0 eq) were added and the reaction mixture was further degassed for 5 min and then heated at 100 °C for 16 h. The reaction mixture was filtered through celite and extracted with ethyl acetate (3 × 50 ml). The combined organic layer was washed with water, brine and dried over sodium sulphate. The solvent was distilled off to afford the crude residue which was purified by column chromatography (60-120 silica gel, 30 % ethyl acetate in hexane) to afford the title product in 64.5 % yield (0.35 g). LC-MS (ESI): Calculated mass: 282.04; Observed mass: 283.0 [M+H]⁺ (rt: 1.60 min).

### Example 1.

### 4-(2,4-Difluorophenyl)-N-(1-methyl-1H-pyrazol-3-yl)-6-(5-(1-methyl-1H-pyrazol-4-yl)-1H-benzo[d]imidazol-1-yl) pyridin-2-amine

### a) 6-Chloro-4-(2, 4-difluorophenyl)-N-(4-(1-methyl-1H-pyrazol-4-yl)-2-nitro-phenyl) pyridin-2-amine

A solution of the compound of Intermediate Example 5(a) (0.8 g, 3.07 mmol) in toluene (5 ml) was degassed by N₂ bubbling for 5 min. The compound of Intermediate Example 1 (0.8 g, 3.69 mmol, 1.2 eq) was added and the mixture was degassed for another 5 min. Palladium acetate (0.027 g, 0.123 mmol), 0.04 eq) and BINAP (0.076 g, 0.123 mmol, 0.04 eq) and potassium *tert*-butoxide (0.69 g, 6.15 mmol, 2.0 eq) were added sequentially and the mixture was further degassed for 5 min and then heated at 100 °C for 5 h. The mixture was filtered through celite pad and extracted with ethyl acetate (3 × 50 ml). The combined organic layer was washed with water, brine and dried over sodium sulphate. The solvent was distilled off to afford the crude residue which was purified by column chromatography (60-120 silica gel, 30 % ethyl acetate in hexane) in 29.5 % yield (0.4 g). LC-MS (ESI): Calculated mass: 441.2; Observed mass: 442.0 [M+H]⁺ (rt: 1.84 min).

### b) 1-(6-Chloro-4-(2, 4-difluorophenyl) pyridin-2-yl)-5-(1-methyl-1H-pyrazol-4-yl)-1H-benzo[d]imidazole

To a solution of the compound of Example 1(a) (0.4 g, 0.907 mmol) in formic acid (5 ml), iron (0.5 g,9.07 mmol) was added and the mixture was heated at 100 °C for 16 h. The formic acid was distilled off and the crude product was dissolved in ethyl acetate. The ethyl acetate layer was washed with water, brine and dried over sodium sulphate. The solvent was distilled off to afford the title product in 47 % yield (0.18 g). LC-MS (ESI): Calculated mass: 421.2; Observed mass: 422.5 [M+H]⁺ (rt: 1.83 min).

### c) 4-(2, 4-Difluorophenyl)-N-(1-methyl-1H-pyrazol-3-yl)-6-(5-(1-methyl-1H-pyrazol-4-yl)-1H-benzo[d]imidazol-1-yl) pyridin-2-amine

A solution of the compound of Example 1(b) (0.07 g, 0.166 mmol) in toluene (5 ml) was degassed by N₂ bubbling for 5 min. 1-Methyl-1H-pyrazol-3-amine (0.024 g, 0.199 mmol, 1.2 eq) was added and the mixture was degassed for another 5 min. Pd₂(dba)₃ (0.007 g, 0.0083 mmol, 0.05 eq) and xantphos (0.005g, 0.0083 mmol), 0.05 eq) and Cs₂CO₃ (0.162 g, 0.4988 mmol, 3.0 eq) were added sequentially using the procedure of Example 1(a) and the mixture was heated at 100 °C for 16 h. The mixture was filtered and extracted using the procedure of Example 1(a). The solvent was distilled off to afford the crude residue which was purified by preparative HPLC to afford the title product in 15 % yield (12mg). ¹H NMR (400 MHz, CD₃OD): δ 8.83 (s, 1H), 8.24-8.22 (d, 1H), 8.03 (s, 1H), 7.90-7.89 (d, 2H), 7.77-7.71 (m, 1H), 7.62-7.60 (m, 1H), 7.51 (d, 1H), 7.30-7.25 (d, 2H), 7.20-7.13 (m, 2H), 6.43-6.42 (d, 1H), 3.97 (s, 3H), 3.86 (s, 3H); LC-MS (ESI): Calculated mass: 482.18; Observed mass: 483.55 [M+H]⁺ (rt: 1.57 min).

### Example 2.

### N-(4-(2,4-difluorophenyl)-6-(5-(1-methyl-1H-pyrazol-4-yl)-1H-benzo[d]-imidazol-1-yl)pyridin-2-yl)cyclopropanesulfonamide

A solution of the compound of Example 1(b) (42 mg, 0.1 mmol) in dioxane (1 ml) was degassed by N2 bubbling for 5 min. Cyclopropane sulfonamide (0.11 g, 0.1 mmol, 1 eq) was added and the mixture was degassed for another 5 min. Palladium acetate (2 mg, 0.008 mmol, 0.08 eq) and xantphos (5 mg, 0.008 mmol, 0.08 eq) and Cs₂CO₃ (100 mg, 0.3 mmol, 3.0 eq) were added and the mixture was further degassed for 5 min and then heated at 100 °C for 16 h. The mixture was filtered through celite and extracted with ethyl acetate (3 × 50 ml). The combined organic layer was washed with water, brine and dried over sodium sulphate. The solvent was distilled off to afford the crude residue which was purified by preparative HPLC to yield the title product in 20 % yield (10 mg). ¹H NMR (300 MHz, CD₃OD): δ 9.33 (s, 1H), 8.68 (d, 1H), 8.08 (s, 1H), 7.94-7.92 (m, 2H), 7.81-7.73 (m, 2H), 7.70 (s, 1H), 7.24-7.16 (m, 3H), 3.96 (s, 3H), 3.16-3.09 (m, 1H), 1.28-1.24 (m, 2H), 1.05-1.01 (m, 2H); LC-MS (ESI): Calculated mass: 506.1; Observed mass: 507.1 [M+H]⁺ (rt: 1.52 min).

### Example 2 (imido).

### Sodium salt of imido form of N-(4-(2,4-difluorophenyl)-6-(5-(1-methyl-1H-pyrazol-4-yl)-1H-benzo[d]imidazol-1-yl)pyridin-2-yl)cyclopropanesulfonamide

The compound (34 mg) was prepared using the procedure described in Example 17 (imido) starting from the title compound of Example 2. The desired compound was crystallized from ethanol. ¹H NMR (300 MHz, DMSO-*d*₆): δ 8.83 (s, 1H), 8.81-8.78 (d, 1H), 8.19 (s, 1H), 7.93 (s, 1H), 7.88 (d, 1H), 7.76-7.68 (m, 1H), 7.53-7.5 (d, 1H), 7.45-7.37 (dt, 1H), 7.27-7.20 (dt, 1H), 6.93 (s, 1H), 6.5 (s, 1H), 3.87 (s, 3H), 2.9 (m, 1H), 0.81-0.8 (m, 2H), 0.6-0.58 (m, 2H); LC-MS (ESI): Calculated mass: 506.1; Observed mass: 506.8 [M+H]⁺ (rt: 1.54 min).

### Example 3.

### N-(4-(2,4-difluorophenyl)-6-(5-(1-methyl-1H-pyrazol-4-yl)-1H-benzo[d]-imidazol-1-yl)pyridin-2-yl)acetamide

The compound was prepared from the compound of Example 1(b) using the procedure of Example 2. ¹H NMR (400 MHz, DMSO-*d*₆): δ 10.81 (s, 1H), 9.07 (s, 1H), 8.58 (d, 1H), 8.32-8.22 (m, 2H), 7.96-7.95 (m, 2H), 7.85-7.81 (m, 2H), 7.59 (d, 1H), 7.61-7.49 (m, 1H), 7.36-7.31 (m, 1H), 3.89 (s, 3H), 2.21 (s, 3H); LC-MS (ESI): Calculated mass: 444.15; Observed mass: 445.3 [M+H]⁺ (rt: 1.43 min).

### Example 4.

### N-(4-(2,4-difluorophenyl)-6-(5-(1-methyl-1H-pyrazol-4-yl)-1H-benzo[d]-imidazol-1-yl)pyridin-2-yl)methanesulfonamide

The compound was prepared from the compound of Example 1(b) using the procedure of Example 2. ¹H NMR (400 MHz, DMSO-*d*₆): δ 11.2 (s, 1H), 9.03 (s, 1H), 8.60 (d, 1H), 8.21 (s, 1H), 7.95 (s, 2H), 7.89-7.82 (m, 1H), 7.74 (s, 1H), 7.59-7.48 (m, 2H), 7.34-7.31 (m, 1H), 7.05 (s, 1H), 3.87 (s, 3H), 3.38 (s, 3H); LC-MS (ESI): Calculated mass: 480.12; Observed mass: 481.05 [M+H]⁺ (rt: 1.39 min).

### Example 5.

### N-(4-(2,4-difluorophenyl)-6-(5-(1-methyl-1H-pyrazol-4-yl)-1H-benzo[d]-imidazol-1-yl)pyridin-2-yl)ethanesulfonamide

The compound was prepared from the compound of Example 1(b) using the procedure of Example 2. ¹H NMR (400 MHz, DMSO-*d*₆): δ 11.1 (s, 1H), 9.02 (s, 1H), 8.61 (d, 1H), 8.22 (s, 1H), 7.95-7.94 (m, 2H), 7.89-7.83 (m, 1H), 7.74 (s, 1H), 7.60-7.56 (m, 1H), 7.54-7.48 (m, 1H), 7.36-7.31 (m, 1H), 7.06 (s, 1H), 3.87 (s, 3H), 3.54 (quartet, 2H), 1.26 (t, 3H); LC-MS (ESI): Calculated mass: 494.13; Observed mass: 494.8 [M+H]⁺ (rt: 1.5 min).

### Example 5 (imido).

### Sodium salt of imido form of N-(4-(2,4-difluorophenyl)-6-(5-(1-methyl-1H-pyrazol-4-yl)-1H-benzo[d]imidazol-1-yl)pyridin-2-yl)ethanesulfonamide

The compound (30 mg) was prepared using the procedure described in Example 17 (imido) starting from the title compound of Example 5. The desired compound was washed with diethylether and dried. ¹H NMR (300 MHz, DMSO-*d*₆): δ 8.83 (s, 1H), 8.8-8.7 (d, 1H), 8.19 (s, 1H), 7.93 (s, 1H), 7.88 (s, 1H), 7.73-7.69 (m, 1H), 7.52-7.49 (dd, 1H), 7.27-7.21 (t, 1H), 6.94 (s, 1H), 6.48 (s, 1H), 3.87 (s, 3H), 3.11-3.04 (q, 2H), 1.14-1.07 (t, 3H); LC-MS (ESI): Calculated mass: 494.13; Observed mass: 495.1 [M+H]⁺ (rt: 1.42 min).

### Example 6.

### N-(4-(2,4-difluorophenyl)-6-(5-(1-methyl-1H-pyrazol-4-yl)-1H-benzo[d]-imidazol-1-yl)pyridin-2-yl)propane-2-sulfonamide

The compound was prepared from the compound of Example 1(b) using the procedure of Example 2. ¹H NMR (400 MHz, DMSO-*d*₆): δ 11.1 (s, 1H), 9.04 (s, 1H), 8.67 (d, 1H), 8.22 (s, 1H), 7.96-7.94 (m, 2H), 7.87-7.85 (m, 1H), 7.73 (s, 1H), 7.61-7.52 (m, 2H), 7.34 (m, 1H), 7.06 (s, 1H), 3.95 (m, 1H), 3.87 (s, 3H), 1.33 (d, 6H); LC-MS (ESI): Calculated mass: 508.15; Observed mass: 509.05 [M+H]⁺ (rt: 1.65 min).

### Example 6 (imido).

### Imido form of N-(4-(2,4-difluorophenyl)-6-(5-(1-methyl-1H-pyrazol-4-yl)-1H-benzo[d]imidazol-1-yl)pyridin-2-yl)propane-2-sulfonamide

A slurry of the crude residue of the compound of Example 6 (12 g) was stirred at 65 °C with THF (166 ml) and filtered in hot condition. The solid material was dried under vacuum to afford 3.5 g of the title compound. ¹H NMR(400MHz, DMSO-d₆) δ 8.83-8.81 (m, 2H), 8.18 (s, 1H), 7.92 (s, 1H), 7.87 (s, 1H), 7.73-7.69 (m, 1H), 7.5-7.48 (d, 1H), 7.43-7.37 (dt, 1H), 7.25-7.21(dt, 1H), 6.93 (s, 1H), 6.49 (s, 1H), 3.87 (s, 3H), 3.75-3.65 (sep, 1H), 1.16-1.14 (d, 6H); LC-MS (ESI): Calculated mass: 508.15 (free base); Observed mass: 508.8 [M+H]⁺ (free base) (rt: 1.51 min).

### Example 7.

### N-(4-(2,4-difluorophenyl)-6-(5-(1-methyl-1H-pyrazol-4-yl)-1H-benzo[d]-imidazol-1-yl)pyridin-2-yl)pyridazin-3-amine

The compound was prepared from the compound of Example 1(b) using the procedure of Example 2. ¹H NMR (400 MHz, DMSO-*d*₆): δ 10.29 (s, 1H), 8.93 (s, 1H), 8.90-8.87 (m, 1H), 8.43-8.42 (m, 1H), 8.24-8.18 (m, 2H), 8.15-8.10 (m, 2H), 7.99 (s, 1H), 7.96 (s, 1H), 7.65-7.62 (m, 2H), 7.5-7.44 (m, 1H), 7.36-7.28 (m, 2H), 3.87 (s, 3H); LC-MS (ESI): Calculated mass: 480.16; Observed mass: 481.1 [M+H]⁺ (rt: 1.39 min).

### Example 8.

### N-(4-(2-fluorophenyl)-6-(5-(1-methyl-1H-pyrazol-4-yl)-1H-benzo[d]imidazol-1-yl)pyridin-2-yl)cyclopropanesulfonamide

### a) 2,6-Dichloro-4-(2-fluorophenyl)pyridine

A solution of 2,6-dichloro-4-iodopyridine (1.09 g, 4 mmol) in 1,2-dimethoxy-ethane (15 ml) was degassed by N₂ bubbling for 5 min. 2-Fluorophenylboronic acid (0.67 g, 4.8 mmol, 1.2 eq) was added and the mixture was degassed for another 5 min. Pd(dppf)Cl₂ (0.33 g, 0.4 mmol, 0.1 eq) and aqueous sodium carbonate (1.27 g, 12 mmol, 3 eq) were added sequentially using the procedure of Intermediate Example 1 and the mixture was then heated at 90 °C for 2 h. The reaction mixture was then quenched and extracted as in Intermediate Example 1. The solvent was distilled off to afford the crude residue which was purified by column chromatography (60-120 silica gel, 1 % ethyl acetate in hexane) to yield the title product in 100 % yield (0.97 g).

### b) 6-Chloro-4-(2-fluorophenyl)-N-(4-(1-methyl-1H-pyrazol-4-yl)-2-nitrophenyl) pyridin-2-amine

A solution of the compound of Example 8(a) (0.97 g, 4 mmol) in toluene (5 ml) was degassed by N₂ bubbling for 5 min. The compound of Intermediate Example 1 (0.96 g, 4.4 mmol, 1.1 eq) was added and the mixture was degassed for another 5 min. Palladium acetate (36 mg, 0.16 mmol, 0.04 eq) and BINAP (99 mg, 0.16 mmol, 0.04 eq) and potassium *tert*-butoxide (0.67 g, 6 mmol, 1.5 eq) were added sequentially following the procedure of Example 1(a). The crude residue of the product was purified by column chromatography (60-120 silica gel, 50 % ethyl acetate in hexane) in 36 % yield (0.6 g). LC-MS (ESI): Calculated mass: 423.09; Observed mass: 424.05 [M+H]⁺ (rt: 2.04 min).

### c) 1-(6-chloro-4-(2-fluorophenyl) pyridin-2-yl)-5-(1-methyl-1H-pyrazol-4-yl)-1H-benzo[d]imidazole

To a solution the compound of Example 8(b) (0.59 g, 1.4 mmol) in formic acid (5 ml), iron (0.78 g, 14 mmol) was added and heated at 100 °C for 16 h. The formic acid was distilled off and the crude product was dissolved in ethyl acetate. The ethyl acetate layer was washed with water, brine and dried over sodium sulphate. The solvent was distilled off to afford the title product in 53 % yield (0.3 g). LC-MS (ESI): Calculated mass: 403.1; Observed mass: 404.1 [M+H]⁺ (rt: 1.66 min).

### d) N-(4-(2-fluorophenyl)-6-(5-(1-methyl-1H-pyrazol-4-yl)-1H-benzo[d]imidazol-1-yl)pyridin-2-yl)cyclopropanesulfonamide

A solution of the compound of Example 8(c) (0.1 g, 0.25 mmol) in dioxane (1 ml) was degassed by N₂ bubbling for 5 min. Cyclopropane sulfonamide (30 mg, 0.25 mmol), 1 eq) was added and the mixture was degassed for another 5 min. Palladium acetate (5 mg, 0.02 mmol, 0.08 eq) and xantphos (12 mg, 0.02 mmol, 0.08 eq) and Cs₂CO₃ (0.24 g, 0.75 mmol, 3.0 eq) were added and the mixture was further degassed for 5 min and then heated at 100 °C for 16 h. The mixture was filtered through celite and extracted as in Example 1. The solvent was distilled off to afford the crude residue which was purified by preparative HPLC to afford the title product in 8 % yield (10 mg). ¹H NMR (400 MHz, CD₃OD): δ 8.91 (s, 1H), 8.62 (d, 1H), 8.05 (s, 1H), 7.91 (m, 2H), 7.73-7.67 (m, 3H), 7.58-7.52 (m, 1H), 7.41-7.33 (m, 2H), 7.21 (s, 1H), 3.97 (s, 3H), 3.17-3.15 (m, 1H), 1.30-1.26 (m, 2H), 1.06-1.04 (m, 2H); LC-MS (ESI): Calculated mass: 488.14; Observed mass: 489.2 [M+H]⁺ (rt: 1.46 min).

### Example 8 (imido).

### Sodium salt of imido form of N-(4-(2-fluorophenyl)-6-(5-(1-methyl-1H-pyrazol-4-yl)-1H-benzo[d]imidazol-1-yl)pyridin-2-yl)cyclopropanesulfonamide

The compound (45 mg) was prepared using the procedure described in Example 17 (imido) starting from the title compound of Example 8. ¹H NMR (300 MHz, DMSO-*d*₆): δ 8.82 (s, 1H), 8.79-8.76 (d, 1H), 8.18 (s, 1H), 7.91 (s, 1H), 7.87 (d, 1H), 7.67-7.62 (m, 1H), 7.52-7.42 (m, 2H), 7.37-7.31 (m, 2H), 6.93 (s, 1H), 6.53 (s, 1H), 3.86 (s, 3H), 2.89 (m, 1H), 0.82-0.79 (m, 2H), 0.61-0.57 (m, 2H); LC-MS (ESI): Calculated mass: 488.14; Observed mass: 489.4 [M+H]⁺ (rt: 1.49 min).

### Example 9.

### N-(4-(2-fluorophenyl)-6-(5-(1-ethyl-1H-pyrazol-4-yl)-1H-benzo[d]imidazol-1-yl)pyridin-2-yl)acetamide

The compound was prepared from the compound of Example 8(c) using the procedure of Example 8. ¹H NMR (300 MHz, CD₃OD): δ 8.88 (s, 1H), 8.37-8.35 (m, 2H), 8.01 (s, 1H), 7.87 (m, 2H), 7.69-7.61 (m, 3H), 7.59-7.49 (m, 1H), 7.36-7.34 (m, 2H), 3.95 (s, 3H), 2.26 (m, 3H); LC-MS (ESI): Calculated mass: 426.16; Observed mass: 427.25 [M+H]⁺ (rt: 1.4 min).

### Example 10.

### N-(4-(2-fluorophenyl)-6-(5-(1-methyl-1H-pyrazol-4-yl)-1H-benzo[d]imidazol-1-yl)pyridin-2-yl)methanesulfonamide

The compound was prepared from the compound of Example 8(c) using the procedure of Example 8. ¹H NMR (300 MHz, DMSO-*d*₆): δ 11.2 (s, 1H), 9.02 (s, 1H), 8.60 (d, 1H), 8.20 (s, 1H), 7.94 (s, 2H), 7.74 (m, 2H), 7.56 (m, 2H), 7.41 (m, 2H), 7.07 (s, 1H), 3.86 (s, 3H), 3.38 (s, 3H); LC-MS (ESI): Calculated mass: 462.13; Observed mass: 463.1 [M+H]⁺ (rt: 1.31 min).

### Example 11.

### N-(4-(2-fluorophenyl)-6-(5-(1-methyl-1H-pyrazol-4-yl)-1H-benzo[d]imidazol-1-yl)pyridin-2-yl)ethanesulfonamide

The compound was prepared from the compound of Example 8(c) using the procedure of Example 8(d). ¹H NMR (400 MHz, DMSO-*d*₆): δ 11.1 (s, 1H), 9.03 (s, 1H), 8.61 (d, 1H), 8.22 (s, 1H), 7.95-7.94 (m, 2H), 7.79-7.76 (m, 2H), 7.60-7.57 (m, 2H), 7.46-7.40 (m, 2H), 7.09 (s, 1H), 3.87 (s, 3H), 3.55 (quartet, 2H), 1.26 (t, 3H); LC-MS (ESI): Calculated mass: 476.14; Observed mass: 477.0 [M+H]⁺ (rt: 1.41 min).

### Example 11 (imido).

### Sodium salt of imido form of N-(4-(2-fluorophenyl)-6-(5-(1-methyl-1H-pyrazol-4-yl)-1H-benzo[d]imidazol-1-yl)pyridin-2-yl)ethanesulfonamide

The compound (34 mg) was prepared using the procedure described in Example 17 (imido) starting from the title compound of Example 11. The desired compound was washed with diethyl ether and dried. ¹H NMR (400 MHz, DMSO-*d*₆): δ 8.83 (s, 1H), 8.8-8.78 (d, 1H), 8.2 (s, 1H), 7.93 (s, 1H), 7.89-7.88 (d, 1H), 7.68-7.65 (dt, 1H), 7.52-7.46 (m, 1H), 7.38-7.33 (m, 2H), 6.96 (s, 1H), 6.51 (s, 1H), 3.22 (s, 3H), 3.11-3.06 (q, 2H), 1.14-1.08 (t, 3H); LC-MS (ESI): Calculated mass: 476.14; Observed mass: 477.0 [M+H]⁺ (rt: 1.41 min). Observed mass: 476.8 [M+H]⁺ (rt: 1.37 min).

### Example 12.

### N-(4-(2-fluorophenyl)-6-(5-(1-methyl-1H-pyrazol-4-yl)-1H-benzo[d]imidazol-1-yl)pyridin-2-yl)propane-2-sulfonamide

The compound was prepared from the compound of Example 8(c) using the procedure of Example 8. ¹H NMR (300 MHz, DMSO-*d*₆): δ 11.03 (s, 1H), 9.03 (s, 1H), 8.67 (d, 1H), 8.22 (s, 1H), 7.96-7.94 (m, 2H), 7.79-7.73 (m, 2H), 7.61-7.55 (m, 2H), 7.42-7.39 (m, 2H), 7.08 (s, 1H), 3.96-3.93 (m, 1H), 3.87 (s, 3H), 1.33 (d, 6H); LC-MS (ESI): Calculated mass: 490.16; Observed mass: 491.1 [M+H]⁺ (rt: 1.48 min).

### Example 12 (imido).

### Sodium salt of imido form of N-(4-(2-fluorophenyl)-6-(5-(1-methyl-1H-pyrazol-4-yl)-1H-benzo[d]imidazol-1-yl)pyridin-2-yl)propane-2-sulfonamide

The title compound (24 mg) was prepared using the procedure described in Example 17 (imido) starting from the title compound of Example 12. ¹H NMR (400 MHz, DMSO-*d*₆): δ 8.83-8.81 (m, 2H), 8.19 (s, 1H), 7.92 (s, 1H), 7.87 (d, 1H), 7.67-7.63 (dt, 1H), 7.51-7.46 (m, 2H), 7.37-7.32 (m, 2H), 6.94 (s, 1H), 6.51 (s, 1H), 3.87 (s, 3H), 3.75-3.65 (m, 1H), 1.16-1.14 (d, 6H); LC-MS (ESI): Calculated mass: 490.16; Observed mass: 491.4 [M+H]⁺ (rt: 1.5 min).

### Example 13.

### N-(4-(2-fluorophenyl)-6-(5-(1-methyl-1H-pyrazol-4-yl)-1H-benzo[d]imidazol-1-yl)pyridin-2-yl)pyridazin-3-amine

The compound was prepared from the compound of Example 8(c) using the procedure of Example 8. ¹H NMR (400 MHz, DMSO-*d*₆): δ 10.49 (s, 1H), 9.01 (s, 1H), 8.85-8.83 (m, 1H), 8.32-8.29 (m, 1H), 8.20 (s, 1H), 8.05 (s, 1H), 7.99-7.94 (m, 3H), 7.79-7.78 (m, 1H), 7.63-7.58 (m, 4H), 7.47-7.41 (m, 2H), 3.87 (s, 3H); LC-MS (ESI): Calculated mass: 462.17; Observed mass: 463.2 [M+H]⁺ (rt: 0.95 min).

### Example 14.

### N-(4-(4-fluorophenyl)-6-(5-(1-methyl-1H-pyrazol-4-yl)-1H-benzo[d]imidazol-1-yl)pyridin-2-yl)cyclopropanesulfonamide

### a) 2,6-Dichloro-4-(4-fluorophenyl)pyridine

A solution of 2,6-dichloro-4-iodopyridine (2.18 g, 8 mmol) in 1,2-dimethoxyethane (15 ml) was degassed by N₂ bubbling for 5 min. 4-Fluorophenylboronic acid (1.34 g, 9.6 mmol, 1.2 eq) was added and the mixture was degassed for another 5 min. Pd(dppf)Cl₂ (1.3 g, 1.6 mmol, 0.2 eq) and aqueous sodium carbonate (2.54 g, 24 mmol, 3 eq) were added sequentially using the procedure of Intermediate Example 1 and the mixture was then heated at 90 °C for 2 h. The reaction mixture was quenched and extracted as in Intermediate Example 1. The solvent was distilled off to afford the crude residue which was purified by column chromatography (60-120 silica gel, 1 % ethyl acetate in hexane) to yield the title product in 77 % yield (1.5 g). LC-MS (ESI): Calculated mass: 240.99; Observed mass: 242.0 [M+H]⁺ (rt: 1.95 min).

### b) 6-Chloro-4-(4-fluorophenyl)-N-(4-(1-methyl-1H-pyrazol-4-yl)-2-nitrophenyl) pyridin-2-amine

A solution of the compound of Example 14(a) (0.97 g, 4 mmol) in toluene (5 ml) was degassed by N₂ bubbling for 5 min. The compound of Intermediate Example 1 (0.96 g, 4.4 mmol, 1.1 eq) was added and the mixture was degassed for another 5 min. Palladium acetate (36 mg, 0.16 mmol, 0.04 eq) and BINAP (99 mg, 0.16 mmol, 0.04 eq) and potassium *tert*-butoxide (0.67 g, 6 mmol, 1.5 eq) were added sequentially following the procedure of Example 1(a). The crude residue of the product was purified by column chromatography (60-120 silica gel, 50 % ethyl acetate in hexane) in 15 % yield (0.25 g).

### c) N1-(6-chloro-4-(4-fluorophenyl)pyridin-2-yl)-4-(1-methyl-1H-pyrazol-4-yl)-benzene-1,2-diamine

To a solution of the compound of Example 14(b) (0.25 g, 0.6 mmol) in THF (10 ml) were added a solution of ammonium chloride (0.26 g, 4.8 mmol, 8 eq) in water (2 ml) and zinc (0.31 g, 4.8 mmol, 8 eq). The mixture was stirred at RT for 6 h and filtered. The filtrate was diluted with water and extracted as in Intermediate Example 1. The solvent was distilled off to afford the title product in 100 % yield (0.24 g). LC-MS (ESI): Calculated mass: 393.12; Observed mass: 394.5 [M+H]⁺ (rt: 1.59 min).

### d) 1-(6-chloro-4-(4-fluorophenyl) pyridin-2-yl)-5-(1-methyl-1H-pyrazol-4-yl)-1H-benzo[d]imidazole

A solution of the compound of Example 14(c) (0.24 g, 0.6 mmol) and formic acid (5 ml) was heated at 100 °C for 16 h. The formic acid was distilled off and the crude product was extracted as in Example 8(c). The solvent was distilled off to afford the title product in 38 % yield (90 mg). LC-MS (ESI): Calculated mass: 403.1; Observed mass: 404.2 [M+H]⁺ (rt: 1.68 min).

### e) N-(4-(4-fluorophenyl)-6-(5-(1-methyl-1H-pyrazol-4-yl)-1H-benzo[d]imidazol-1-yl)pyridin-2-yl)cyclopropanesulfonamide

A solution the compound of Example 14(d) (40 mg, 01 mmol) in dioxane (1 ml) was degassed by N₂ bubbling for 5 min. Cyclopropane sulfonamide (12 mg, 0.1 mmol, 1 eq) was added and the mixture was degassed for another 5 min. Palladium acetate (2 mg, 0.008 mmol, 0.08 eq) and xantphos (5 mg, 0.008 mmol, 0.08 eq) and Cs₂CO₃ (0.1 g, 0.3 mmol, 3.0 eq) were added and the mixture was further degassed for 5 min and then heated at 100 °C for 16 h. The mixture was filtered through celite and extracted as in Example 1. The solvent was distilled off to afford the crude residue which was purified by preparative HPLC to afford the title product in 20 % yield (10 mg). ¹H NMR (400 MHz, CD₃OD): δ 8.97 (s, 1H), 8.68 (d, 1H), 8.06 (s, 1H), 7.92-7.89 (m, 4H), 7.73 (m, 1H), 7.69-7.67 (m, 1H), 7.34-7.29 (m, 2H), 7.21 (m, 1H), 3.97 (s, 3H), 3.16-3.09 (m, 1H), 1.30-1.26 (m, 2H), 1.05-1.03 (m, 2H); LC-MS (ESI): Calculated mass: 488.14; Observed mass: 489.1 [M+H]⁺ (rt: 1.5 min).

### Example 15.

### N-(4-(4-fluorophenyl)-6-(5-(1-methyl-1H-pyrazol-4-yl)-1H-benzo[d]imidazol-1-yl)pyridin-2-yl)acetamide

The compound was prepared from the compound of Example 14(d) using the procedure of Example 14. ¹H NMR (400 MHz, DMSO-*d*₆): δ 10.75 (s, 1H), 9.13 (s, 1H), 8.59 (d, 1H), 8.29 (d, 1H), 7.93-7.89 (m, 5H), 7.56 (d, 2H), 7.43-7.4 (m, 2H), 3.86 (s, 3H), 2.19 (m, 3H); LC-MS (ESI): Calculated mass: 426.16; Observed mass: 427.5 [M+H]⁺ (rt: 1.47 min).

### Example 16.

### N-(4-(4-fluorophenyl)-6-(5-(1-methyl-1H-pyrazol-4-yl)-1H-benzo[d]imidazol-1-yl)pyridin-2-yl)methanesulfonamide

The compound was prepared from the compound of Example 14(d) using the procedure of Example 14. ¹H NMR (400 MHz, DMSO-*d*₆): δ 11.2 (s, 1H), 9.12 (s, 1H), 8.63 (d, 1H), 8.22 (s, 1H), 7.96-7.92 (m, 4H), 7.86 (s, 1H), 7.59-7.57 (m, 1H), 7.46-7.42 (m, 2H), 7.11 (s, 1H), 3.88 (s, 3H), 3.38 (s, 3H); LC-MS (ESI): Calculated mass: 462.13; Observed mass: 462.8 [M+H]⁺ (rt: 1.43 min).

### Example 17.

### N-(4-(4-fluorophenyl)-6-(5-(1-methyl-1H-pyrazol-4-yl)-1H-benzo[d]imidazol-1-yl)pyridin-2-yl)propane-2-sulfonamide

The compound was prepared from the compound of Example 14(d) using the procedure of Example 14(e). ¹H NMR (300 MHz, DMSO-*d*₆): δ 11.0 (s, 1H), 9.13 (s, 1H), 8.68 (d, 1H), 8.22 (s, 1H), 7.96-7.87 (m, 5H), 7.62-7.58 (m, 1H), 7.47-7.41 (m, 2H), 7.12(s, 1H), 3.99-3.93 (m, 1H), 3.87 (s, 3H), 1.34 (d, 6H); LC-MS (ESI): Calculated mass: 490.16; Observed mass: 491.05 [M+H]⁺ (rt: 1.58 min).

### Example 17 (imido).

### Sodium salt of imido form of N-(4-(4-fluorophenyl)-6-(5-(1-methyl-1H-pyrazol-4-yl)-1H-benzo[d]imidazol-1-yl)pyridin-2-yl)propane-2-sulfonamide

To the compound of Example 17 (37 mg) isopropyl alcohol (1 ml) and sodium *tert-*butoxide (7 mg) were added and after refluxing overnight the mixture was evaporated to yield 32 mg of the title compound. ¹H NMR (300 MHz, DMSO-*d*₆): δ 8.9 (s, 1H), 8.87-8.84 (d, 1H), 7.9 (s, 1H), 7.85-7.8 (m, 3H), 7.49-7.46 (d, 1H), 7.35-7.29 (t, 2H), 7.08 (s, 1H), 6.6 (s, 1H), 3.86(s, 3H), 3.73-3.69 (m, 1H), 1.15-1.31 (d, 6H): Calculated mass: 490.16; Observed mass: 491.45 [M+H]⁺ (rt: 1.52min).

### Example 18.

### N-(3-fluoro-6'-(5-(1=methyl-1H-pyrazol-4-yl)-1H-benzo[d]imidazol-1-yl)-[2,4'-bipyridin]-2'-yl)cyclopropanesulfonamide

### a) 2,6-Dichloro-4-(4,4,5,5-tetramethyl-1,3,2-dioxaborolan-2-yl)pyridine

A solution of 2,6-dichloro-4-iodopyridine (15 g, 54.9 mmol) in DMF (150 ml) was degassed by N₂ bubbling for 5 min. Bispinacolato diborane (17.63 g, 82.4 mmol, 1.5 eq) was added and the mixture was degassed for another 5 min. Pd(dppf)Cl₂ (2.24 g, 2.7 mmol, 0.05 eq) and potassium acetate (8.07 g, 82.4 mmol, 1.5 eq) were added sequentially using the procedure of Intermediate Example 1 and the mixture was then heated at 90 °C for 3 h. The reaction mixture was then quenched and extracted as in Intermediate Example 1. The solvent was distilled off to afford the crude residue which was purified by column chromatography (60-120 silica gel, 50 % ethyl acetate in hexane) to afford the title product in 67 % yield (10 g). ¹H NMR (300 MHz, CDCl₃): δ 7.59 (s, 2H), 1.35 (s, 6H), 1.26 (s, 6H).

### b) 2',6'-Dichloro-3-fluoro-2,4'-bipyridine

A solution of 2-bromo-3-fluoropyridine (3 g, 17 mmol) in 1,2-dimethoxyethane (30 mL) was degassed by N₂ bubbling for 5 min. The compound of Example 18(a) (9.3 g, 34 mmol, 2 eq) was added and the mixture was degassed for another 5 min. Pd(dppf)Cl₂ (1.39 g, 1.7 mmol, 0.1 eq) and aqueous sodium carbonate (4.5 g, 42 mmol, 2.5 eq) were added sequentially using the procedure of Intermediate Example 1 and the mixture was then heated at 110 °C for 4 h. The reaction mixture was then quenched and extracted as in Intermediate Example 1. The solvent was distilled off to afford the crude residue which was purified by column chromatography (60-120 silica gel, 10 % ethyl acetate in hexane) to afford the title product in 30 % yield (1.2 g). ¹H NMR (300 MHz, CDCl₃): δ 7.61-7.55 (m, 2H), 7.42-7.31 (m, 3H).

### c) 6'-chloro-3-fluoro-N-(4-(1-methyl-1H-pyrazol-4-yl)-2-nitrophenyl)-[2,4'-bi-pyridin]-2'-amine

A solution of the compound of Example 18(b) (1.2 g, 5 mmol) in dioxane (12 ml) was degassed by N₂ bubbling for 5 min. The compound of Intermediate Example 1 (1.29 g, 6 mmol, 1.2 eq) was added and the mixture was degassed for another 5 min. Palladium acetate (110 mg, 0.5 mmol, 0.1 eq) and BINAP (610 mg, 1 mmol, 0.2 eq) and cesium carbonate (4.07 g, 12.4 mmol, 2.5 eq) were added sequentially following the procedure of Example 1(a). The crude residue of the product was purified by column chromatography (60-120 silica gel, 70 % ethyl acetate in hexane) to yield the title product in 38 % yield (0.8 g).

### d) N1-(6'-chloro-3-fluoro-[2,4'-bipyridin]-2'-yl)-4-(1-methyl-1H-pyrazol-4-yl)-benzene-1,2-diamine

To a solution of the compound of Example 18(c) (0.8 g, 1.9 mmol) in THF (15 ml) were added a solution of ammonium chloride (0.8 g, 15.1 mmol, 8 eq) in water (5 ml) and zinc (0.97 g, 15.1 mmol, 8 eq). The mixture was stirred at RT for 6 h and filtered. The filtrate was diluted with water and extracted as in Intermediate Example 1. The solvent was distilled off to afford the title product in 100 % yield (0.8 g). LC-MS (ESI): Calculated mass: 394.11; Observed mass: 395.0 [M+H] (rt: 1.34 min).

### e) 1-(6'-chloro-3-fluoro-[2,4'-bipyridin]-2'-yl)-5-(1-methyl-1H-pyrazol-4-yl)-1H-benzo[d]imidazole

A solution of the compound of Example 18(d) (0.4 g, 1 mmol) and formic acid (5 ml) was heated at 110°C for 12 h. The formic acid was distilled off and the crude was extracted as in Example 8(c). The solvent was distilled off to afford the title product in 100 % yield (0.4 g). ¹H NMR (300 MHz, CD₃OD): δ 8.95 (s, 1H), 8.64 (m, 1H), 8.38 (s, 1H), 8.29-8.27 (m, 2H), 8.03-8.02 (m, 2H), 7.92-7.81 (m, 2H), 7.69-7.64 (m, 2H), 3.95 (s, 3H).

### f) N-(3-fluoro-6'-(5-(1-methyl-1H-pyrazol-4-yl)-1H-benzo[d]imidazol-1-yl)-[2,4'-bipyridin]-2'-yl)cyclopropanesulfonamide

A solution of the compound of Example 18(e) (40 mg, 0.1 mmol) in dioxane (1 ml) was degassed by N₂ bubbling for 5 min. Cyclopropane sulfonamide (12 mg, 0.1 mmol, 1 eq) was added and the mixture was degassed for another 5 min. Pd₂(dba)₃ (9 mg, 0.01 mmol, 0.1 eq) and xantphos (6 mg, 0.01 mmol, 0.1 eq) and Cs₂CO₃ (80 mg, 0.25 mmol, 2.5 eq) were added and the mixture was further degassed for 5 min and then heated at 110 °C for 16 h. The mixture was filtered through celite and extracted as in Example 1. The solvent was distilled off to afford the crude residue which was purified by column chromatography (60-120 silica gel, 10 % methanol in CHCl₃) to afford the title product in 50 % yield (15 mg). ¹H NMR (300 MHz, DMSO-*d*₆): δ 11.22 (s, 1H), 9.0 (s, 1H), 8.69-8.63 (m, 2H), 8.24 (s, 1H), 8.04-7.98 (m, 4H), 7.71-7.6 (m, 2H), 7.49 (s, 1H), 3.88 (s, 3H), 3.17-3.15 (m, 1H), 1.13-1.12 (m, 2H), 1.05-1.02 (m, 2H); LC-MS (ESI): Calculated mass: 489.14; Observed mass: 490.01 [M+H]⁺ (rt: 0.57 min).

### Example 19.

### N-(3-fluoro-6'-(5-(1-methyl-1H-pyrazol-4-yl)-1H-benzo[d]imidazol-1-yl)-[2,4'-bipyridin]-2'-yl)acetamide

The compound was prepared from the compound of Example 18(e) using the procedure of Example 18. ¹H NMR (300 MHz, DMSO-*d*₆): δ 11.1 (s, 1H), 9.34 (s, 1H), 8.84 (s, 1H), 8.62 (d, 1H), 8.45 (d, 1H), 8.12 (s, 1H), 8.07 (s, 1H), 7.94-7.92 (m, 2H), 7.86-7.74 (m, 2H), 7.63-7.57 (m, 1H), 3.89 (s, 3H), 2.22 (m, 3H); LC-MS (ESI): Calculated mass: 427.16; Observed mass: 428.3 [M+H]⁺ (rt: 0.7 min).

### Example 20.

### N-(3-fluoro-6'-(6-(1-methyl-1H-pyrazol-4-yl)-3H-imidazo[4,5-b]pyridin-3-yl)-[2,4'-bipyridin]-2'-yl)cyclopropanesulfonamide

### a) 6'-Chloro-3-fluoro-N-(5-(1-methyl-1H-pyrazol-4-yl)-3-nitropyridin-2-yl)-[2,4'-bipyridin]-2'-amine

A solution of 2',6'-dichloro-3-fluoro-2,4'-bipyridine (0.5 g, 2.1 mmol) in dioxane (12 ml) was degassed by N₂ bubbling for 5 min. The compound of Intermediate Example 7 (0.55 g, 2.5 mmol, 1.2 eq) was added and the mixture was degassed for another 5 min. Pd₂(dba)₃ (0.19 g, 0.21 mmol, 0.1 eq) and xantphos (0.24 g, 0.42 mmol, 0.2 eq) and cesium carbonate (1.68 g, 5.2 mmol, 2.5 eq) were added sequentially following the procedure of Example 1(a). The crude residue of the product was purified by column chromatography (60-120 silica gel, 70 % ethyl acetate in hexane) to yield the title product in 32 % yield (0.28 g). LC-MS (ESI): Calculated mass: 425.08; Observed mass: 426.3 [M+H]⁺ (rt: 1.72 min).

### b) N2-(6'-chloro-3-fluoro-[2,4'-bipyridin]-2'-yl)-5-(1-methyl-1H-pyrazol-4-yl)pyridine-2,3-diamine

To a solution of the compound of Example 20(a) (0.28 g, 0.7 mmol) in THF (10 ml) were added a solution of ammonium chloride (0.28 g, 5.6 mmol, 8 eq) in water (5 ml) and zinc (0.33 g, 5.6 mmol, 8 eq). The mixture was stirred at RT for 2 h and filtered. The filtrate was diluted with water and extracted as in Intermediate Example 1. The solvent was distilled off to afford the title product in 72 % yield (0.2 g).

### c) 3-(6'-Chloro-3-fluoro-[2,4'-bipyridin]-2'-yl)-6-(1-methyl-1H-pyrazol-4-yl)-3H-imidazo[4,5-b]pyridine

A solution the compound of Example 20(b) (0.2 g, 0.5 mmol) and formic acid (5 ml) was heated at 100°C for 16 h. The formic acid was distilled off and the crude was extracted as in Example 8(c). The solvent was distilled off to afford the title product in 75 % yield (0.15 g).

### d) N-(3-fluoro-6'-(6-(1-methyl-1H-pyrazol-4-yl)-3H-imidazo[4,5-b]pyridin-3-yl)-[2,4'-bipyridin]-2'-yl)cyclopropanesulfonamide

A solution of the compound of Example 20(c) (40 mg, 0.1 mmol) in dioxane (1 ml) was degassed by N₂ bubbling for 5 min. Cyclopropane sulfonamide (14 mg, 0.12 mmol, 1.2 eq) was added and the mixture was degassed for another 5 min. Pd₂(dba)₃ (9 mg, 0.01 mmol, 0.1 eq) and xantphos (6 mg, 0.01 mmol, 0.1 eq) and Cs₂CO₃ (80 mg, 0.25 mmol, 2.5 eq) were added and the mixture was further degassed for 5 min and then heated at 110 °C for 16 h. The mixture was filtered through celite and extracted as in Example 1. The solvent was distilled off to afford the crude residue which was purified by column chromatography (60-120 silica gel, 10 % methanol in CHCl₃) to afford the title product in 8 % yield (5 mg). ¹H NMR (300 MHz, CDCl₃): δ 9.24 (s, 1H), 9.16 (s, 1H), 8.69 (s, 1H), 8.64 (m, 1H), 8.3 (s, 1H), 7.98 (s, 1H), 7.85 (s, 1H), 7.73 (s, 1H), 7.61-7.58 (m, 2H), 7.48-7.44 (m, 1H), 4.0 (s, 3H), 2.88 (m, 1H), 1.40-1.31 (m, 2H), 1.08-1.1 (m, 2H); LC-MS (ESI): Calculated mass: 490.13; Observed mass: 491.0 [M+H]⁺ (rt: 1.16 min).

### Example 21.

### N-(4-(2,4-difluorophenyl)-6-(5-(1-(2-(dimethylamino)ethyl)-1H-pyrazol-4-yl)-1H-benzo[d]imidazol-1-yl)pyridin-2-yl)cyclopropanesulfonamide

### a) 6-Chloro-4-(2,4-difluorophenyl)-N-(4-(1-(2-(dimethylamino)ethyl)-1H-pyrazol-4-yl)-2-nitrophenyl)pyridin-2-amine

A solution of the compound of Intermediate Example 5(a) (0.2 g, 0.8 mmol) in dioxane (15 ml) was degassed by N₂ bubbling for 5 min. The compound Intermediate Example 3 (0.23 g, 0.9 mmol, 1.1 eq) was added and the mixture was degassed for another 5 min. Palladium acetate (0.017 g, 0.08 mmol, 0.1 eq) and BINAP (0.1 g, 0.16 mmol, 0.2 eq) and cesium carbonate (0.65 g, 2 mmol, 2.0 eq) were added sequentially and the mixture was further degassed for 5 min and then heated at 110 °C for 12 h. The mixture was filtered through celite and extracted as in Example 1. The solvent was distilled off to afford the crude residue which was purified by column chromatography (60-120 silica gel, 10 % methanol in CH₂Cl₂) to afford the title product in 55 % yield (0.1 g). LC-MS (ESI): Calculated mass: 498.14; Observed mass: 499.7 [M+H]⁺ (rt: 1.41 min).

### b) N1-(6-chloro-4-(2,4-difluorophenyl)pyridin-2-yl)-4-(1-(2-(dimethylamino)-ethyl)-1H-pyrazol-4-yl)benzene-1,2-diamine

To a solution of the compound of Example 21(a) (0.1 g, 0.2 mmol) in THF (10 ml) were added a solution of ammonium chloride (85 mg, 1.6 mmol, 8 eq) in water (2 ml) and zinc (0.1 g, 1.6 mmol, 8 eq). The mixture was stirred at RT for 12 h and filtered. The filtrate was diluted with water and extracted as in Intermediate Example 1. The solvent was distilled off to afford the title product in 100 % yield (93 mg).

### c) 2-(4-(1-(6-chloro-4-(2,4-difluorophenyl)pyridin-2-yl)-1H-benzo[d]imidazol-5-yl)-1H-pyrazol-1-yl)-N,N-dimethylethanamine

A solution the compound of Example 21(b) (93 mg, 0.2 mmol) and formic acid (5 ml) was heated at 100 °C for 12 h. The formic acid was distilled off and the crude was extracted as in Example 8(c). The solvent was distilled off to afford desired title product in 83 % yield (80 mg). LC-MS (ESI): Calculated mass: 478.15; Observed mass: 479.45 [M+H]⁺ (rt: 1.36 min).

### d) N-(4-(2,4-difluorophenyl)-6-(5-(1-(2-(dimethylamino)ethyl)-1H-pyrazol-4-yl)-1H-benzo[d]imidazol-1-yl)pyridin-2-yl)cyclopropanesulfonamide

A solution of the compound of Example 21(c) (80 mg, 0.2 mmol) in dioxane (5 ml) was degassed by N₂ bubbling for 5 min. Cyclopropane sulfonamide (29 mg, 0.24 mmol, 1.2 eq) was added and the mixture was degassed for another 5 min. Pd(OAc)₂ (5 mg, 0.02 mmol, 0.1 eq) and xantphos (23 mg, 0.04 mmol, 0.2 eq) and Cs₂CO₃ (162 mg, 0.5 mmol, 2.5 eq) were added and the mixture was further degassed for 5 min and then heated at 110°C for 12 h. The mixture was filtered through celite and extracted as in Example 1. The solvent was distilled off to afford the crude residue which was purified by column chromatography (60-120 silica gel, 10 % methanol in CH₂Cl₂) to afford the desired title product in 35 % yield (20 mg). ¹H NMR (400 MHz, CDCl₃):δ 8.7 (s, ¹H), 8.04 (m, 2H), 7.82 (d, 2H), 7.56(m, 2H), 7.41 (m, 2H), 7.07-7.02 (m, 2H), 4.29 (m, 2H), 2.89-2.84 (m, 3H), 2.33 (s, 6H), 1.39 (m, 2H), 1.08 (m, 2H); LC-MS (ESI): Calculated mass: 563.19; Observed mass: 564.25 [M+H]⁺ (rt: 0.59 min).

### Example 22.

### N-(4-(2-fluorophenyl)-6-(5-(1-methyl-1H-pyrazol-4-yl)-1H-benzo[d]imidazol-1-yl)pyrimidin-2-yl)cyclopropanesulfonamide

### a) 4-Chloro-6-(2-fluorophenyl)pyrimidin-2-amine

A solution of 4,6-dichloropyrimidin-2-amine (5 g, 30.6 mmol) in 1,2-dimethoxyethane (50 ml) was degassed by N₂ bubbling for 5 min. 2-Fluprophenylboronic acid (4.27 g, 30.6 mmol, 1.2 eq) was added and the mixture was degassed for another 5 min. Pd(dppf)Cl₂ (1.25 g, 1.53 mmol, 0.05 eq) and aqueous sodium carbonate (8.12 g, 76.6 mmol, 2.5 eq) were added sequentially using the procedure of Intermediate Example 1 and then heated at 90 °C for 3 h. The reaction mixture was then quenched and extracted as in Intermediate Example 1. The solvent was distilled off to afford the crude residue which was purified by column chromatography (60-120 silica gel, 30 % ethyl acetate in hexane) to afford the title product in 15 % yield (1 g). LC-MS (ESI): Calculated mass: 223.03; Observed mass: 224.0 [M+H]⁺ (rt: 1.53 min).

### b) N-(4-chloro-6-(2-fluorophenyl)pyrimidin-2-yl)cyclopropanesulfonamide

To an icecold solution of the compound of Example 22(a) (1 g, 4.48 mmol) in DMF (50 ml) was added NaH (0.16 g, 6.72 mmol, 1.5 eq). The mixture was stirred for 10 min and cyclopropylsulfonyl chloride (0.76 g, 5.38 mmol, 1.2 eq) was added and the mixture was stirred at RT for 24 h: The mixture was then quenched with water and extracted with ethyl acetate (3 x 100 ml). The combined organic layer was washed with water, brine and dried over sodium sulphate. The solvent was distilled off to afford the crude product which was purified by column chromatography (60-120 silica gel, 5 % ethyl acetate in hexane) to afford the title product in 34 % yield (0.5 g). LC-MS (ESI): Calculated mass: 327.02; Observed mass: 327.8 [M+H]⁺ (rt: 1.61 min).

### c) N-(4-(2-fluorophenyl)-6-(5-(1-methyl-1H-pyrazol-4-yl)-1H-benzo[d]imidazol-1-yl)pyrimidin-2-yl)cyclopropanesulfonamide

To an icecold solution of the compound of Example 22(b) (0.2 g, 0.61 mmol) in DMF (50 ml) in a sealed tube was added NaH (22 mg, 0.9 mmol, 1.5 eq). The mixture was stirred for 10 min and the compound of Intermediate Example 6 (0.12 g, 0.61 mmol, 1 eq) was added. The mixture was stirred at RT for 24 h and then quenched and extracted with ethyl acetate (3 x 100 ml). The combined organic layer was washed with water, brine and dried over sodium sulphate. The solvent was distilled off to afford a crude product mixture from which the title compound was isolated by preparative TLC in 0.01% yield (3 mg). ¹H NMR (400 MHz, CDCl₃): δ 8.75 (s, 1H), 8.36 (d, 1H), 8.24-8.21 (m,2H), 7.99 (s, 1H), 7.85-7.81 (m, 2H), 7.69 (s, 1H), 7.63-7.55 (m, 3H), 7.39-7.37 (m, 1H), 3.99 (s, 3H), 3.3 (m, 1H), 1.12-1.10 (m, 2H), 0.88-0.84 (m, 2H); LC-MS (ESI): Calculated mass: 489.14; Observed mass: 490.4 [M+H]⁺ (rt: 1.43 min).

### Example 23.

### N-(6-(5-(H-pyrazol-1-yl)-1H-benzo[d]imidazol-1-yl)-4-(2,4-difluorophenyl)-pyridin-2-yl)cyclopropanesulfonamide

A solution of the title compound of Intermediate Example 5 (50 mg, 0.144 mmol) in dioxane (5 ml) was degassed by N₂ bubbling for 5 min. The compound of Intermediate Example 4 (26 mg, 0.144 mmol, 1 eq) was added and the mixture was degassed for another 5 min. CuI (1.3 mg, 0.0072 mmol, 0.05 eq), N,N-dimethyl glycine (1.4 mg, 0.0144 mmol, 0.1 eq) and Cs₂CO₃ (141 mg, 0.434 mmol, 3.0 eq) were added and the reaction mixture was further degassed for 5 min and then heated at 100 °C for 24 h. The reaction mixture was filtered through celite and washed with ethyl acetate. The solvent was distilled off to afford a crude product mixture from which the title compound was isolated by preparative HPLC to afford the title compound in 14 % yield (10 mg). ¹H NMR (400 MHz, DMSO-*d*₆): δ 11.2 (s, 1H), 9.18 (d, 1H), 8.86 (d, 1H), 8.66 (d, 1H), 8.23 (d, 1H), 7.96-7.86 (m, 2H), 7.83-7.78 (m, 2H); 7.59-7.51 (m, 1H), 7.39-7.34 (m, 1H), 7.16 (s, 1H), 6.59 (s, 1H), 3.19-3.14 (m, 1H), 1.24-1.12 (m, 2H), 1.06-1.03 (m, 2H); LC-MS (ESI): Calculated mass: 492.12; Observed mass: 493.4 [M+H]⁺ (rt: 1.71 min).

### Example 24.

### N-(4-(2,4-difluorophenyl)-6-(6-(1-methyl-1H-pyrazol-4-yl)-3H-imidazo[4,5-b]pyridin-3-yl)pyridin-2-yl)cyclopropanesulfonamide

### a) 6-Chloro-4-(2,4-difluorophenyl)-N-(5-(1-methyl-1H-pyrazol-4-yl)-3-nitropyridin-2-yl)pyridin-2-amine

A solution of the compound of Intermediate Example 5(a) (0.5 g, 1.92 mmol) in dioxane (5 ml) was degassed by N₂ bubbling for 5 min. The compound of Intermediate Example 7 (0.42 g, 1.92 mmol, 1 eq) was added and the mixture was degassed for another 5 min. Pd₂(dba)₃ (0.087 g, 0.09 mmol, 0.05 eq) and xantphos (0.11 g, 0.192 mmol, 0.1 eq) and cesium carbonate (1.56 g, 4.8 mmol, 2.5 eq) were added sequentially following the procedure of Example 1(a) and the mixture was then heated at 110 °C for 16 h. The mixture was filtered through celite and extracted as in Example 1. The solvent was distilled off to afford the crude residue which was purified by column chromatography (60-120 silica gel, 20 % ethyl acetate in hexane) to afford the title product in 46 % yield (0.4 g).

### b) N2-(6-chloro-4-(2,4-difluorophenyl)pyridin-2-yl)-5-(1-methyl-1H-pyrazol-4-yl)pyridine-2,3-diamine

To a solution of the compound of Example 24(a) (0.39 g, 0.88 mmol) in THF (10 ml) were added a solution of ammonium chloride (0.37 g, 7 mmol, 8 eq) in water (2 ml) and zinc (0.46 g, 7 mmol, 8 eq). The mixture was stirred at RT for 6 h and filtered. The filtrate was diluted with water and extracted as in Intermediate Example 1. The solvent was distilled off to afford the title product in 88 % yield (0.32 g).

### c) 3-(6-Chloro-4-(2,4-difluorophenyl)pyridin-2-yl)-6-(1-methyl-1H-pyrazol-4-yl)-3H-imidazo[4,5-b]pyridine

A solution the compound of Example 24(b) (0.32 g, 0.77 mmol) and formic acid (10 ml) was heated at 100 °C for 16 h. The formic acid was distilled off and the crude was extracted as in Example 8(c). The solvent was distilled off to afford the title product in 70 % yield (0.23 g). LC-MS (ESI): Calculated mass: 422.09; Observed mass: 423.2 [M+H] ⁺ (rt: 1.74 min).

### d) N-(4-(2,4-difluorophenyl)-6-(6-(1-methyl-1H-pyrazol-4-yl)-3H-imidazo[4,5-b]pyridin-3-yl)pyridin-2-yl)cyclopropanesulfonamide

A solution of the compound of Example 24(c) (150 mg, 0.35 mmol) in dioxane (5 ml) was degassed by N₂ bubbling for 5 min. Cyclopropane sulfonamide (55 mg, 0.46 mmol, 1.3 eq) was added and the mixture was degassed for another 5 min. Pd(OAc)₂ (4 mg, 0.017 mmol, 0.05 eq) and xantphos (20 mg, 0.03 mmol, 0.1 eq) and Cs₂CO₃ (288 mg, 0.88 mmol, 2.5 eq) were added and the mixture was further degassed for 5 min and then heated at 110°C for 12 h. The mixture was filtered through celite and extracted as in Example 1. The solvent was distilled off to afford the crude residue which was purified by column chromatography (60-120 silica gel, 3 % methanol in CH₂Cl₂) to afford the title product in 27 % yield (48 mg). ¹H NMR (300 MHz, DMSO-*d*₆): δ 11.07 (s, 1H), 9.09 (s, 1H), 8.80 (d, 1H), 8.49-8.45 (m, 2H), 8.30 (s, 1H), 8.05 (d, 1H), 7.81-7.73 (m, 1H), 7.57-7.49 (m, 1H),7.38-7.32 (m, 1H), 7.11(s, 1H), 3.89 (s, 3H), 3.41-3.37 (m, 1H), 1.13-1.10 (m, 4H); LC-MS (ESI): Calculated mass: 507.13; Observed mass: 508.1 [M+H]⁺ (rt: 1.54 min).

### Example 25.

### N-(3,5-difluoro-6'-(5-(1-methyl-1H-pyrazol-4-yl)-1H-benzo[d]imidazol-1-yl)-[2,4'-bipyridin]-2'-yl)cyclopropanesulfonamide

### a) 2',6'-Dichloro-3,5-difluoro-2,4'-bipyridine

A solution of 2-bromo-3,5-difluoropyridine (1.6 g, 5.84 mmol) in 1,2-dimethoxyethane (30 ml) was degassed by N₂ bubbling for 5 min. The compound of Example 18(a) (1.36 g, 7 mmol, 2 eq) was added and the mixture was degassed for another 5 min. Pd(dppf)Cl₂ (0.47 g, 0.58 mmol, 0.1 eq) and aqueous sodium carbonate (1.85 g, 17.5 mmol, 3 eq) were added sequentially using the procedure of Intermediate Example 1 and the mixture was then heated at 90 °C for 2 h. The reaction mixture was quenched and extracted as in Intermediate Example 1. The solvent was distilled off to afford the crude residue which was purified by column chromatography (60-120 silica gel, 5 % ethyl acetate in hexane) to afford the title product in 72 % yield (1.1 g).

### b) 6'-Chloro-3,5-difluoro-N-(4-(1-methyl-1H-pyrazol-4-yl)-2-nitrophenyl)-[2,4'-bipyridin]-2'-amine

A solution of the compound of Example 25(a) (0.4 g, 1.5 mmol) in dioxane (12 ml) was degassed by N₂ bubbling for 5 min. The compound of Intermediate Example 1 (0.4 g, 1.84 mmol, 1.2 eq) was added and the mixture was degassed for another 5 min. Palladium acetate (17 mg, 0.08 mmol, 0.05 eq) and BINAP (47 mg, 0.08 mmol, 0.05 eq) and potassium *tert*-butoxide (0.26 g, 2.29 mmol, 1.5 eq) were added sequentially following the procedure of Example 1(a) and the mixture was then heated at 110 °C for 72 h. The crude residue of the product was purified by column chromatography (60-120 silica gel, 50 % ethyl acetate in hexane) to afford the title product in 18 % yield (0.12 g). LC-MS (ESI): Calculated mass: 442.08; Observed mass: 443.05 [M+H]⁺ (rt: 1.98 min).

### c) N1-(6'-chloro-3,5-difluoro-[2,4'-bipyridin]-2'-yl)-4-(1-methyl-1H-pyrazol-4-yl)benzene-1,2-diamine

To a solution of the compound of Example 25(b) (0.12 g, 0.27 mmol) in THF (15 ml) were added a solution of ammonium chloride (0.15 g, 2.7 mmol, 8 eq) in water (2 ml) and zinc (0.18 g, 2.7 mmol, 8 eq). The mixture was stirred at RT for 6 h and filtered. The filtrate was diluted with water and extracted as in Intermediate Example 1. The solvent was distilled off to afford the title product in 90 % yield (0.1 g).

### d) 1-(6'-Chloro-3,5-difluoro-[2,4'-bipyridin]-2'-yl)-5-(1-methyl-1H-pyrazol-4-yl)-1 H-benzo [d] imidazole

A solution the compound of Example 25(c) (0.1 g, 0.242 mmol) and formic acid (10 ml) was heated at 100°C for 16 h. The formic acid was distilled off and the crude product was extracted as in Example 8(c). The solvent was distilled off to afford the title product in 78 % yield (80 mg). LC-MS (ESI): Calculated mass: 422.09; Observed mass: 422.8 [M+H]⁺ (rt: 1.69 min).

### e) N-(3,5-difluoro-6'-(5-(1-methyl-1H-pyrazol-4-yl)-1H-benzo[d]imidazol-1-yl)-[2,4'-bipyridin]-2'-yl)cyclopropanesulfonamide

A solution of the compound of Example 25(d) (80 mg, 0.19 mmol) in dioxane (5 ml) was degassed by N₂ bubbling for 5 min. Cyclopropane sulfonamide (28 mg, 0.23 mmol, 1.2 eq) was added and the mixture was degassed for another 5 min. Pd(OAc)₂ (4.2 mg, 0.019 mmol, 0.1 eq) and xantphos (22 mg, 0.04 mmol, 0.2 eq) and Cs₂CO₃ (185 mg, 0.57 mmol, 3 eq) were added and the mixture was further degassed for 5 min and then heated at 110 °C for 12 h. The mixture was filtered through celite and extracted as in Example 1. The solvent was distilled off to afford the crude residue which was purified by preparative TLC to afford the title product in 5 % yield (5 mg). ¹H NMR (300 MHz, DMSO-*d*₆): δ 8.98 (s, 1H), 8.76 (d, 1H), 8.61 (d, 1H), 8.25-8.18 (m, 2H), 7.96 (m, 3H), 7.62-7.59 (m, 1H), 7.47 (s, 1H), 3.88 (s, 3H), 3.12 (m, 1H), 1.14-1.12 (m, 2H), 1.04-1.02 (m, 2H); LC-MS (ESI): Calculated mass: 507.13; Observed mass: 508.1 [M+H]⁺ (rt: 1.27 min).

### Example 26.

### N-(3,5-difluoro-6'-(5-(1-methyl-1H-pyrazol-4-yl)-1H-benzo[d]imidazol-1-yl)-[2,4'-bipyridin]-2'-yl)acetamide

The compound was prepared from the compound of Example 25(d) using the procedure of Example 28. ¹H NMR (400 MHz, DMSO-*d*₆): δ 10.85 (s, 1H), 9.0 (s, 1H), 8.76 (d, 1H), 8.58 (s, 1H), 8.50 (d, 1H), 8.23-8.21 (m, 2H), 8.0 (s, 1H), 7.95 (m, 2H), 7.62-7.59 (m, 1H), 3.88 (s, 3H), 2.21 (s, 3H); LC-MS (ESI): Calculated mass: 445.15; Observed mass: 446.1 [M+H]⁺ (rt: 1.08 min).

### Example 27.

### N-(6-(5-(1-methyl-1H-pyrazol-4-yl)-1H-benzo[d]imidazol-1-yl)-4-(1H-pyrrol-1-yl)pyridin-2-yl)acetamide

### a) 2,6-Dichloro-4-(1H-pyrrol-1-yl)pyridine

A solution 2,6-dichloropyridin-4-amine (2 g, 12.3 mmol) and 2,5-dimethoxy-furan (1.94 g, 14.7 mmol, 1.2 eq) in acetic acid (10 ml) was heated at 90 °C for 2 h. The mixture was quenched with water and extracted with EtOAc (3 x 50 ml). The combined organic layer was washed with water, brine and dried over sodium sulphate. The solvent was distilled off to afford the title product in 80 % yield (2.1 g).

### b) 6-Chloro-N-(4-(1-methyl-1H-pyrazol-4-yl)-2-nitrophenyl)-4-(1H-pyrrol-1-yl)-pyridin-2-amine

To an icecold solution of the compound of Example 27(a) (1 g, 4.58 mmol) in DMSO (20 ml) was added NaH (0.13 g, 5.5 mmol, 1.2 eq). The mixture was stirred for 10 min and the compound of Intermediate Example 1 (1.1 g, 5.5 mmol, 1.2 eq) was added. The mixture was stirred at RT for 16 h and then quenched with water and extracted with ethyl acetate (3 × 50 ml). The combined organic layer was washed with water, brine and dried over sodium sulphate. The solvent was distilled off to afford the crude product which was purified by column chromatography (60-120 silica gel, 50 % ethyl acetate in hexane) to afford the title product in 25 % yield (0.45 g). LC-MS (ESI): Calculated mass: 394.09; Observed mass: 394.8 [M+H]⁺ (rt: 1.87 min).

### c) N1-(6-chloro-4-(1H-pyrrol-1-yl)pyridin-2-yl)-4-(1-methyl-1H-pyrazol-4-yl)benzene-1,2-diamine

To a solution of the compound of Example 27(b) (0.43 g, 1.1 mmol) in THF (30 ml) were added a solution of ammonium chloride (0.58 g, 10.9 mmol, 10 eq) in water (5 ml) and zinc (0.71 g, 10.9 mmol, 10 eq). The mixture was stirred at RT for 6 h and filtered. The filtrate was diluted with water and extracted as in Intermediate Example 1. The solvent was distilled off to afford the product in 90 % yield (0.36 g). LC-MS (ESI): Calculated mass: 364.12; Observed mass: 365.0 [M+H]⁺ (rt: 1.47 min).

### d) 1-(6-Chloro-4-(1H-pyrrol-1-yl)pyridin-2-yl)-5-(1-methyl-1H-pyrazol-4-yl)-1H-benzo[d]imidazole

A solution of the compound of Example 27(c) (0.35 g, 0.96 mmol) and formic acid (10 ml) was heated at 100°C for 16 h. The formic acid was distilled off and the crude was extracted as in Example 8(c). The solvent was distilled off to afford the title product in 97 % yield (350 mg). LC-MS (ESI): Calculated mass: 374.10; Observed mass: 375.1 [M+H]⁺ (rt: 1.59 min).

### e) N-(6-(5-(1-methyl-1H-pyrazol-4-yl)-1H-benzo[d]imidazol-1-yl)-4-(1H-pyrrol-1-yl)pyridin-2-yl)acetamide

A solution of the compound of Example 27(d) (100 mg, 0.27 mmol) in dioxane (5 ml) was degassed by N₂ bubbling for 5 min. Acetamide (19 mg, 0.32 mmol, 1.2 eq) was added and the mixture was degassed for another 5 min. Pd(OAc)₂ (3 mg, 0.013 mmol, 0.05 eq) and xantphos (15 mg, 0.026 mmol, 0.1 eq) and Cs₂CO₃ (261 mg, 0.8 mmol, 3 eq) were added and the mixture was further degassed for 5 min and then heated at 110 °C for 12 h. The mixture was filtered through celite and extracted as in Example 1. The solvent was distilled off to afford the crude residue which was purified by preparative HPLC to afford the title product in 75 % yield (80 mg). ¹H NMR (400 MHz, DMSO-*d₆*): δ 10.79 (s, 1H), 9.13 (s, 1H), 8.63 (d, 1H), 8.21 (s, 2H), 7.95-7.93 (m, 2H), 7.84 (d, 1H), 7.60-7.57 (m, 3H), 6.41-6.40 (m, 2H), 3.88 (s, 3H), 2.21 (s, 3H); LC-MS (ESI): Calculated mass: 397.17; Observed mass: 398.1 [M+H]⁺ (rt: 1.24 min).

### Example 28.

### N-(4-(2-chlorophenyl)-6-(5-(1-methyl-1H-pyrazol-4-yl)-1H-benzo[d]imidazol-1-yl)pyridin-2-yl)cyclopropanesulfonamide

### a) 2,6-Dichloro-4-(2-chlorophenyl)pyridine

A solution of 2,6-dichloro-4-iodopyridine (1 g, 3.65 mmol) in 1,2-dimethoxyethane (15 ml) was degassed by N₂ bubbling for 5 min. 2-Chlorophenylboronic acid (0.68 g, 4.38 mmol, 1.2 eq) was added and the mixture was degassed for another 5 min. Pd(dppf)Cl₂ (0.3 g, 0.37 mmol, 0.1 eq) and aqueous sodium carbonate (1.16 g, 10.9 mmol, 3 eq) were added sequentially using the procedure of Intermediate Example 1 and the mixture was heated at 90 °C for 2 h. The reaction mixture was then quenched and extracted as in Intermediate Example 1. The solvent was distilled off to afford the crude residue which was purified by column chromatography (60-120 silica gel, 5 % ethyl acetate in hexane) to afford the title product in 74 % yield (0.7 g). ¹H NMR (300 MHz, CDCl₃): δ 7.53-7.49 (m, 1H), 7.41-7.35 (m, 3H), 7.32-7.29 (m, 1H), 7.26 (m, 1H).

### b) 6-Chloro-4-(2-chlorophenyl)-N-(4-(1-methyl-1H-pyrazol-4-yl)-2-nitrophenyl)-pyridin-2-amine

A solution of the compound of Example 28(a) (0.7 g, 2.7 mmol) in toluene (10 ml) was degassed by N₂ bubbling for 5 min. The compound of Intermediate Example 1 (0.6 g, 2.7 mmol, 1 eq) was added and the mixture was degassed for another 5 min. Palladium acetate (24 mg, 0.11 mmol, 0.04 eq) and BINAP (67 mg, 0.11 mmol, 0.04 eq) and potassium *tert*-butoxide (0.3 g, 2.7 mmol, 1 eq) were added sequentially following the procedure of Example 1(a) and the mixture was heated at 100 °C overnight. The crude residue of the product was purified by column chromatography (60-120 silica gel, 30 % ethyl acetate in hexane) to afford the title product in 71 % yield (0.5 g). LC-MS (ESI): Calculated mass: 439.06; Observed mass: 439.95 [M+H]⁺ (rt: 2.02 min).

### c) 1-(6-Chloro-4-(2-chlorophenyl)pyridin-2-yl)-5-(1-methyl-1H-pyrazol-4-yl)-1H-benzo[d]imidazole

A solution of the compound of Example 28(b) (0.5 g, 1.13 mmol) in formic acid (10 ml), iron (0.63 g, 11.4 mmol) was added and heated at 100 °C for 16 h. The formic acid was distilled off and the crude was dissolved in ethyl acetate. The ethyl acetate layer was washed with water, brine and dried over sodium sulphate. The solvent was distilled off to afford the title product in 63 % yield (0.3 g). LC-MS (ESI): Calculated mass: 419.07; Observed mass: 421.8 [M+H] ⁺ (rt: 1.84 min).

### d) N-(4-(2-chlorophenyl)-6-(5-(1-methyl-1H-pyrazol-4-yl)-1H-benzo[d]-imidazol-1-yl)pyridin-2-yl)cyclopropanesulfonamide

A solution of the compound of Example 28(c) (200 mg, 0.47 mmol) in dioxane (5 ml) was degassed by N₂ bubbling for 5 min. Cyclopropanesulfonamide (63 mg, 0.52 mmol, 1.1 eq) was added and the mixture was degassed for another 5 min. Pd(OAc)₂ (5 mg, 0.023 mmol, 0.05 eq) and xantphos (15 mg, 0.023 mmol, 0.05 eq) and Cs₂CO₃ (450 mg, 1.41 mmol, 3 eq) were added and the mixture was further degassed for 5 min and then heated at 110°C for 12 h. The:mixture was filtered through celite and extracted as in Example 1. The solvent was distilled off to afford the crude residue which was purified by column chromatography (2 % methanol in CHCl₃) to afford the title product in 15 % yield (30 mg). ¹H NMR (400 MHz, DMSO-*d*₆): δ 11.17 (s, 1H), 9.0 (s, 1H), 8.71 (d, 1H), 8.23 (s, 1H), 7.96-7.94 (m, 2H), 7.67-7.65 (m, 2H), 7.62-7.59 (m, 2H), 7.54-7.52 (m, 2H), 6.98 (s, 1H), 3.87 (s, 3H), 3.16 (m, 1H), 1.12-1.11 (m, 2H), 1.04-1.02 (m, 2H); LC-MS (ESI): Calculated mass: 504.11; Observed mass: 504.7 [M+H]⁺ (rt: 1.59 min).

### Example 29.

### N-(3-chloro-6'-(5-(1-methyl-1H-pyrazol-4-yl)-1H-benzo[d]imidazol-1-yl)-[2,4'-bipyridin]-2'-yl)cyclopropanesulfonamide

### a) 2',3,6'-Trichloro-2,4'-bipyridine

A solution of (2,6-dichloropyridin-4-yl)boronic acid (0.76 g, 4 mmol) in 1,2-dimethoxyethane (15 ml) was degassed by N₂ bubbling for 5 min. 2-Bromo-3-chloropyridine (0.7 g, 3.63 mmol, 1.2 eq) was added and the mixture was degassed for another 5 min. Pd(dppf)Cl₂ (0.3 g, 0.36 mmol, 0.1 eq) and aqueous sodium carbonate (1.15 g, 10.9 mmol, 3 eq) were added sequentially using the procedure of Intermediate Example 1 and then heated at 90 °C for 2 h. The reaction mixture was then quenched and extracted as in Intermediate Example 1. The solvent was distilled off to afford the crude residue which was purified by column chromatography (60-120 silica gel, 10 % ethyl acetate in hexane) to afford the title product in 74 % yield (0.7 g). ¹H NMR (300 MHz, CDCl₃): δ 8.63 (dd, 1H), 7.86 (m, 1H), 7.68 (s, 2H), 7.37 (dd, 1H).

### b) 3,6'-Dichloro-N-(4-(1-methyl-1H-pyrazol-4-yl)-2-nitrophenyl)-[2,4'-bi-pyridin]-2'-amine

A solution of the compound of Example 29(a) (0.69 g, 3.17 mmol) in toluene (10 ml) was degassed by N₂ bubbling for 5 min. The compound of Intermediate Example 1 (0.69 g, 3.17 mmol, 1.1 eq) was added and the mixture was degassed for another 5 min. Palladium acetate (25 mg, 0.115 mmol, 0.04 eq) and BINAP (71 mg, 0.115 mmol, 0.04 eq) and potassium *tert*-butoxide (0.38 g, 3.46 mmol, 1.2 eq) were added sequentially following the procedure of Example 1(a) and the mixture was heated at 100 °C overnight. The crude residue of the product was purified by column chromatography (60-120 silica gel, 30 % ethyl acetate in hexane) to afford the title product in 43 % yield (0.3 g). ¹H NMR (300 MHz, CDCl₃): δ 10.25 (s, 1H), 8.77 (d, 1H), 8.65-8.63 (m, 1H), 8.30 (d, 1H), 7.88-7.85 (m, 1H), 7.79-7.73 (m, 2H), 7.67 (s, 1H), 7.37-7.33 (m, 2H), 7.22 (m, 1H), 3.97 (s, 3H):

### c) 1-(3,6'-Dichloro-[2,4'-bipyridin]-2'-yl)-5-(1-methyl-1H-pyrazol-4-yl)-1H-benzo[d]imidazole

A solution of the compound of Example 29(b) (0.3 g, 0.68 mmol) in formic acid (10 ml), iron (0.38 g, 6.8 mmol) was added and heated at 100 °C for 16 h. The formic acid was distilled off and the crude was dissolved in ethyl acetate. The ethyl acetate layer was washed with water, brine and dried over sodium sulphate. The solvent was distilled off to afford the title product in 64 % yield (0.18 g). LC-MS (ESI): Calculated mass: 420.07; Observed mass: 421.2 [M+H]⁺ (rt: 1.56 min).

### d) N-(3-chloro-6'-(5-(1-methyl-1H-pyrazol-4-yl)-1H-benzo[d]imidazol-1-yl)-[2,4'-bipyridin]-2'-yl)cyclopropanesulfonamide

A solution of the compound of Example 29(c) (100 mg, 0.24 mmol) in dioxane (5 ml) was degassed by N₂ bubbling for 5 min. Cyclopropanesulfonamide (34 mg, 0.28 mmol, 1.2 eq) was added and the mixture was degassed for another 5 min. Pd(OAc)₂ (3 mg, 0.011 mmol, 0.05 eq) and xantphos (6 mg, 0.011 mmol, 0.05 eq) and Cs₂CO₃ (230 mg, 0.71 mmol, 3 eq) were added and the mixture was further degassed for 5 min and then heated at 110 °C for 12h. The mixture was filtered through celite and extracted as in Example 1. The solvent was distilled off to afford the crude residue which was purified by column chromatography (2 % methanol in CHCl₃) to afford the title product in 15 % yield (17 mg). ¹H NMR (300 MHz, DMSO-*d*₆): δ 11.22 (s, 1H), 8.98 (s, 1H), 8.74-8.67 (m, 2H), 8.23 (s, 1H), 8.19-8.16 (m, 1H), 7.97-7.95 (m, 2H), 7.85 (m, 1H), 7.62-7.58 (m, 2H), 7.19 (s, 1H), 3.87 (s, 3H), 3.16 (m, 1H), 1.13-1.12 (m, 2H), 1.06-1.02 (m, 2H); LC-MS (ESI): Calculated mass: 505.11; Observed mass: 506.00 [M+H]⁺ (rt: 1.52 min).

### Example 30.

### N-(5-fluoro-6'-(5-(1-methyl-1H-pyrazol-4-yl)-1H-benzo[d]imidazol-1-yl)-[2,4'-bipyridin]-2'-yl)cyclopropanesulfonamide

### a) 2',6'-Dichloro-5-fluoro-2,4'-bipyridine

A solution of 2-bromo-5-fluoropyridine (2 g, 11 mmol) in 1,2-dimethoxyethane (30 ml) was degassed by N₂ bubbling for 5 min. The compound of Example 18(a) (3.11 g, 11 mmol, 1 eq) was added and the mixture was degassed for another 5 min. Pd(PPh₃)₄ (1.31 g, 0.011 mmol, 0.1 eq) and aqueous sodium carbonate (9.28 g, 28.5 mmol, 2.5 eq) were added sequentially using the procedure of Intermediate Example 1 and then heated at 90 °C for 2 h. The reaction mixture was then quenched and extracted as in Intermediate Example 1. The solvent was distilled off to afford the crude residue which was purified by column chromatography (60-120 silica gel, 5 % ethyl acetate in hexane) to afford the title product in 39 % yield (1 g).

### b) 6'-Chloro-5-fluoro-N-(4-(1-methyl-1H-pyrazol-4-yl)-2-nitrophenyl)-[2,4'-bipyridin]-2'-amine

A solution of the compound of Example 30(a) (0.2 g, 0.82 mmol) in toluene (12 ml) was degassed by N₂ bubbling for 5 min. The compound of Intermediate Example 1 (0.2 g, 0.9 mmol, 1.1 eq) was added and the mixture was degassed for another 5 min. Palladium acetate (14.7 mg, .065 mmol, 0.08 eq) and BINAP (40 mg, 0.065 mmol, 0.08 eq) and potassium *tert*-butoxide (0.23 g, 2.06 mmol, 2.5 eq) were added sequentially following the procedure of Example 1(a) and then heated at 110 °C for 16 h. The crude residue of the product was purified by column chromatography (60-120 silica gel, 50 % ethyl acetate in hexane) to afford the title product in 29 % yield (0.1 g).

### c) N1-(6'-chloro-5-fluoro-[2,4'-bipyridin]-2'-yl)-4-(1-methyl-1H-pyrazol-4-yl)benzene-1,2-diamine

To a solution of the compound of Example 30(b) (0.28 g, 0.66 mmol) in THF (10 ml) were added a solution of ammonium chloride (0.29 g, 5.28 mmol, 8 eq) in water (2 ml) and zinc (0.34 g, 5.28 mmol, 8 eq). The mixture was stirred at RT for 1 h and filtered. The filtrate was diluted with water and extracted as in Intermediate Example 1. The solvent was distilled off to afford the title product in 96 % yield (0.25 g). LC-MS (ESI): Calculated mass: 394.11; Observed mass: 395.1 [M+H]⁺ (rt: 1.42 min).

### d) 1-(6'-Chloro-5-fluoro-[2,4'-bipyridin]-2'-yl)-5-(1-methyl-1H-pyrazol-4-yl)-1H-benzo[d]imidazole

A solution of the compound of Example 30(c) (0.25 g, 0.63 mmol) and formic acid (10 ml) was heated at 100 °C for 4 h. The formic acid was distilled off and the crude was extracted as in Example 8(c). The solvent was distilled off to afford the title product in 55 % yield (150 mg). LC-MS (ESI): Calculated mass: 404.1; Observed mass: 404.8 [M+H]⁺ (rt: 1.7 min).

### e) N-(5-fluoro-6'-(5-(1-methyl-1H-pyrazol-4-yl)-1H-benzo[d]imidazol-1-yl)-[2,4'-bipyridin]-2'-yl)cyclopropanesulfonamide

A solution of the compound of Example 30(d) (50 mg, 0.123 mmol) in dioxane (5 ml) was degassed by N₂ bubbling for 5 min. Cyclopropanesulfonamide (15 mg, 0.123 mmol, 1 eq) was added and the mixture was degassed for another 5 min. Pd(OAc)₂ (2 mg, 0.009 mmol, 0.08 eq) and xantphos (5.7 mg, 0.008 mmol, 0.08 eq) and Cs₂CO₃ (120 mg, 0.37 mmol, 3 eq) were added and the mixture was further degassed for 5 min and then heated at 100°C for 24 h. The mixture was filtered through celite and extracted as in Example 1. The solvent was distilled off to afford the crude residue which was purified by preparative TLC to afford the title product in 25 % yield (15 mg). ¹H NMR (400 MHz, DMSO-*d*₆): δ 11.16 (s, 1H), 9.14 (s, 1H), 8.81 (d, 1H), 8.73 (d, 1H), 8.40-8.37 (m, 1H), 8.23 (s, 1H), 8.15 (s, 1H), 8.05-8.0 (m, 1H), 7.97-7.95 (m, 2H), 7.66 (s, 1H), 7.62-7.59 (m, 1H), 3.89 (s, 3H), 3.16-3.12 (m, 1H), 1.13-1.10 (m, 2H), 1.03-1.0 (m, 2H); LC-MS (ESI): Calculated mass: 489.14; Observed mass: 490.4 [M+H]⁺ (rt: 1.23 min).

### Example 31.

### N-(5-fluoro-6'-(5-(1-methyl-1H-pyrazol-4-yl)-1H-benzo[d]imidazol-1-yl)-[2,4'-bipyridin]-2'-yl)acetamide

The compound was prepared from the compound of Example 30(d). ¹H NMR (400 MHz, DMSO-*d*₆): δ 10.78 (s, 1H), 9.16 (s, 1H), 8.82-8.78 (m,2H), 8.62 (d, 1H), 8.39-8.37 (m, 1H), 8.22-8.20 (m, 2H), 8.04-7.99 (m, 1H), 7.96 (s, 2H), 7.62-7.59 (m, 1H), 3.89 (s, 3H), 2.23 (m, 3H); LC-MS (ESI): Calculated mass: 427.16; Observed mass: 428.3 [M+H]⁺ (rt: 1.91 min).

### Example 32.

### N-(6-(5-(1H-imidazol-1-yl)-1H-benzo[d]imidazol-1-yl)-4-(2,4-difluorophenyl)-pyridin-2-yl)cyclopropanesulfonamide

### a) N-(4-(1H-imidazol-1-yl)-2-nitrophenyl)-6-chloro-4-(2,4-difluorophenyl)-pyridin-2-amine

A solution of the compound of Intermediate Example 5(a) (0.6 g, 2.94 mmol) in toluene (5 ml) was degassed by N₂ bubbling for 5 min. The compound of Intermediate Example 9 (0.76 g, 2.94 mmol, 1 eq) was added and the mixture was degassed for another 5 min. Palladium acetate (32 mg, 0.147 mmol, 0.05 eq) and BINAP (182 mg, 0.294 mmol, 0.1 eq) and potassium *tert*-butoxide (0.9 g, 7.35 mmol, 2.5 eq) were added sequentially and the mixture was further degassed for 5 min and then heated at 100 °C for 12 h. The mixture was filtered through celite and extracted as in Example 1. The solvent was distilled off to afford the crude residue which was purified by column chromatography (60-120 silica gel, 50 % ethyl acetate in hexane) to afford the title product in 12 % yield (150 mg).

### b) N1-(6-chloro-4-(2,4-difluorophenyl)pyridin-2-yl)-4-(1H-imidazol-1-yl)-benzene-1,2-diamine

To a solution of the compound of Example 32(a) (0.15 g, 0.35 mmol) in THF (10 ml) were added a solution of ammonium chloride (0.15 g, 2.81 mmol, 8 eq) in water (2 ml) and zinc (0.18 g, 2.81 mmol, 8 eq). The mixture was stirred at RT for 1 h and filtered. The filtrate was diluted with water and extracted as in Intermediate Example 1. The solvent was distilled off to afford the title product in 72 % yield (0.1 g).

### c) 1-(6-Chloro-4-(2,4-difluorophenyl)pyridin-2-yl)-5-(1H-imidazol-1-yl)-1H-benzo[d]imidazole

A solution of the compound of Example 32(b) (0.1 g, 0.25 mmol) and formic acid (2 ml) was heated at 90 °C for 4 h. The formic acid was distilled off and the crude was extracted as in Example 8(c). The solvent was distilled off to afford the title product in 50 % yield (50 mg).

### d) N-(6-(5-(1H-imidazol-1-yl)-1H-benzo[d]imidazol-1-yl)-4-(2,4-difluoro-phenyl)pyridin-2-yl)cyclopropanesulfonamide

A solution of the compound of Example 32(c) (50 mg, 0.122 mmol) in dioxane (5 ml) was degassed by N₂ bubbling for 5 min. Cyclopropanesulfonamide (15 mg, 0.122 mmol, 1 eq) was added and the mixture was degassed for another 5 min. Pd(OAc)₂ (2 mg, 0.009 mmol, 0.08 eq) and xantphos (5.7 mg, 0.008 mmol, 0.08 eq) and Cs₂CO₃ (120 mg, 0.37 mmol, 3 eq) were added and the mixture was further degassed for 5 min and then heated at 100 °C for 12 h. The mixture was filtered through celite and extracted as in Example 1. The solvent was distilled off to afford the crude residue which was purified by preparative TLC to afford the title product in 33 % yield (20 mg). ¹H NMR (400 MHz, DMSO-*d*₆): δ 11.22 (s, 1H), 9.12 (s, 1H), 8.67 (d, 1H), 8.38 (s, 1H), 8.08 (d, 1H), 7.91-7.85 (m, 2H), 7.79 (s, 1H), 7.71-7.69 (m, 1H), 7.56-7.51 (m, 1H), 7.38-7.33 (m, 1H), 7.13 (s, 2H), 3.16-3.11 (m, 1H), 1.12-1.08 (m, 2H), 1.03-1.01 (m, 2H); LC-MS (ESI): Calculated mass: 492.12; Observed mass: 493.1 [M+H]⁺ (rt: 0.30 min).

### Example 33.

### N-(4-(2,4-difluorophenyl)-6-(5-(1-(2-morpholinoethyl)-1H-pyrazol-4-yl)-1H-benzo[d]imidazol-1-yl)pyridin-2-yl)cyclopropanesulfonamide

### a) 6-Chloro-4-(2,4-difluorophenyl)-N-(4-(1-(2-morpholinoethyl)-1H-pyrazol-4-yl)-2-nitrophenyl)pyridin-2-amine

A solution of the compound of Intermediate Example 5(a) (0.49 g, 1.89 mmol) in dioxane (5 ml) was degassed by N₂ bubbling for 5 min. The compound of Intermediate Example 8 (0.6 g, 1.89 mmol, 1 eq) was added and the mixture was degassed for another 5 min. Palladium acetate (34 mg, 0.15 mmol, 0.08 eq) and BINAP (94 mg, 0.15 mmol, 0.08 eq) and potassium *tert*-butoxide (0.53 g, 4.73 mmol, 2.5 eq) were added sequentially and the mixture was further degassed for 5 min and then heated at 100 °C for 12 h. The mixture was filtered through celite and extracted as in Example 1. The solvent was distilled off to afford the crude residue which was purified by column chromatography (60-120 silica gel, 50 % ethyl acetate in hexane) to afford the title product in 20 % yield (200 mg).

### b) N1-(6-chloro-4-(2,4-difluorophenyl)pyridin-2-yl)-4-(1-(2-morpholinoethyl)-1H-pyrazol-4-yl)benzene-1,2-diamine

To a solution of the compound of Example 33(a) (0.2 g, 0.37 mmol) in THF (10 ml) were added a solution of ammonium chloride (0.16 g, 2.96 mmol, 8 eq) in water (2 ml) and zinc (0.19 g, 2.96 mmol, 8 eq). The mixture was stirred at RT for 1 h and filtered. The filtrate was diluted with water and extracted as in Intermediate Example 1. The solvent was distilled off to afford the title product in 79 % yield (0.15 g). LC-MS (ESI): Calculated mass: 510.17; Observed mass: 511.1 [M+H]⁺ (rt: 0.66 min).

### c) 4-(2-(4-(1-(6-Chloro-4-(2,4-difluorophenyl)pyridin-2-yl)-1H-benzo[d]-imidazol-5-yl)-1H-pyrazol-1-yl)ethyl)morpholine

A solution the compound of Example 33(b) (0.15 g, 0.29 mmol) and formic acid (2 ml) was heated at 90°C for 12 h. The formic acid was distilled off and the crude was extracted as in Example 8(c). The solvent was distilled off to afford the title product in 50 % yield (75 mg). LC-MS (ESI): Calculated mass: 520.16; Observed mass: 521.2 [M+H]⁺ (rt: 1.03 min).

### d) N-(4-(2,4-difluorophenyl)-6-(5-(1-(2-morpholinoethyl)-1H-pyrazol-4-yl)-1H-benzo[d]imidazol-1-yl)pyridin-2-yl)cyclopropanesulfonamide

A solution of the compound of Example 33(c) (75 mg, 0.144 mmol) in dioxane (5 ml) was degassed by N₂ bubbling for 5 min. Cyclopropanesulfonamide (17 mg, 0.144 mmol, 1 eq) was added and the mixture was degassed for another 5 min. Pd(OAc)₂ (2.5 mg, 0.011 mmol, 0.08 eq) and xantphos (8.3 mg, 0.0144 mmol, 0.1 eq) and Cs₂CO₃ (117 mg, 0.36 mmol, 2.5 eq) were added and the mixture was further degassed for 5 min and then heated at 100 °C for 12 h. The mixture was filtered through celite and extracted as in Example 1. The solvent was distilled off to afford the crude residue which was purified by preparative TLC to afford the title product in 29 % yield (25 mg). ¹H NMR (400 MHz, DMSO-*d*₆): δ 11.16 (s, 1H), 9.05 (s, 1H), 8.69 (d, 1H), 8.29 (s, 1H), 7.98-7.96 (m, 2H), 7.88-7.85 (m, 1H), 7.75 (s, 1H), 7.62-7.59 (m, 1H), 7.56-7.49 (m, 1H), 7.37-7.32 (m, 1H), 7.09 (s, 1H), 4.27-4.24 (m, 2H), 3.58-3.55 (m, 4H), 3.18-3.13 (m, 1H), 2.78-2.74 (m, 2H), 2.53-2.49 (m, 4H), 1.15-1.07 (m, 2H), 1.05-1.01 (m, 2H); LC-MS (ESI): Calculated mass: 605.2; Observed mass: 605.8 [M+H]⁺ (rt: 0.44 min).

### Example 34.

### N-(4-(2,4-difluorophenyl)-6-(5-(1-(pyrrolidin-3-yl)-1H-pyrazol-4-yl)-1H-benzo[d]imidazol-1-yl)pyridin-2-yl)cyclopropanesulfonamide

### a) tert-Butyl 3-(4-(1-(6-(cyclopropanesulfonamido)-4-(2,4-difluorophenyl)-pyridin-2-yl)-1H-benzo[d]imidazol-5-yl)-1H-pyrazol-1-yl)pyrrolidine-1-carboxylate

A solution of the compound of Intermediate Example 5 (113mg, 0.347 mmol) in dioxane (5 ml) was degassed by N₂ bubbling for 5 min. The compound of Intermediate Example 2 (120 mg, 0.347 mmol, 1 eq) was added and the mixture was degassed for another 5 min. CuI (3.3 mg, 0.0174 mmol, 0.05 eq), N,N-dimethyl glycine (1.7 mg, 0.0174 mmol, 0.05 eq) and Cs₂CO₃ (282 mg, 0.87 mmol, 2.5 eq) were added and the mixture was further degassed for 5 min and then heated at 100 °C for 24 h. The mixture was filtered through celite and washed with ethyl acetate. The solvent was distilled off to afford the crude product mixture which was purified by preparative TLC to afford the title product in 10 % yield (20 mg). LC-MS (ESI): Calculated mass: 661.23; Observed mass: 662.6 [M+H]⁺ (rt: 1.71 min).

### b) N-(4-(2,4-difluorophenyl)-6-(5-(1-(pyrrolidin-3-yl)-1H-pyrazol-4-yl)-1H-benzo[d]imidazol-1-yl)pyridin-2-yl)cyclopropanesulfonamide

To a solution of the compound of Example 34(a) (15 mg, 0.028 mmol) in 1,4-dioxane (5 ml) at 0°C was added HCl in dioxane and stirred at RT for 1 h. The solvent was distilled off and the residue was washed several times with diethyl ether to give the title product in 78 % yield (10 mg). ¹H NMR (400 MHz, DMSO-*d*₆): δ 11.23 (s, 1H), 9.38 (s, 1H), 9.28 (s, 1H), 9.22 (s, 1H), 8.76 (d, 1H), 8.49 (s, 1H), 8.16 (s, 1H), 8.03 (s, 1H), 7.91-7.85 (m, 1H), 7.79 (s, 1H), 7.71-7.69 (m, 1H), 7.57-7.52 (m, 1H), 7.37-7.34 (m, 1H), 7.14 (s, 1H), 5.20-5.18 (m, 1H), 3.62-3.37 (m, 3H), 3.19-3.15 (m, 2H), 2.43-2.33 (m, 2H), 1.19-1.12 (m, 2H), 1.05-1.02 (m, 2H); LC-MS (ESI): Calculated mass: 561.18; Observed mass: 562.6 [M+H]⁺ (RT: 0.40 min).

### Example 35.

### N-(4-(2,4-difluorophenyl)-6-(5-(1-ethyl-1H-1,2,3-triazol-4-yl)-1H-benzo[d]-imidazol-1-yl)pyridin-2-yl)cyclopropanesulfonamide

A solution of the compound of Intermediate Example 5 (48 mg, 0.14 mmol) in dioxane (5 ml) was degassed by N2 bubbling for 5 min. The compound of Intermediate Example 10 (30 mg, 0.14 mmol, 1 eq) was added and the mixture was degassed for another 5 min. CuI (2 mg, 0.014 mmol, 0.1 eq), N,N-dimethyl glycine (1 mg, 0.014 mmol, 0.1 eq) and Cs₂CO₃ (130 mg, 0.42 mmol, 3.0 eq) were added and the mixture was further degassed for 5 min and then heated at 100 °C for 24 h. The mixture was filtered through celite and washed with ethyl acetate. The solvent was distilled off to afford the crude product mixture which was purified by preparative TLC to afford the title product in 33 % yield (24 mg). ¹H NMR (400 MHz, DMSO-*d*₆): δ 11.18 (s, 1H), 9.11 (s, 1H), 8.79 (d, 1H), 8.71 (s, 1H), 8.22 (s, 1H), 7.92-7.85 (m, 2H), 7.79 (s, 1H), 7.56-7.50 (m, 1H), 7.38-7.33 (m, 1H), 7.13 (s, 1H), 4.44 (quartet, 2H), 3.18-3.13 (m, 1H), 1.51 (t, 3H), 1.14-1.09 (m, 2H), 1.07-1.02 (m, 2H); LC-MS (ESI): Calculated mass: 521.14; Observed mass: 522.1 [M+H]⁺ (rt: 1.52 min).

### Example 36.

### N-(4-(2,4-difluorophenyl)-6-(5-(1-methyl-1H-1,2,3-triazol-4-yl)-1H-benzo[d]-imidazol-1-yl)pyridin-2-yl)cyclopropanesulfonamide

### a) N-(4-(2,4-difluorophenyl)-6-(5-ethynyl-1H-benzo[d]imidazol-1-yl)pyridin-2-yl)cyclopropanesulfonamide

A solution of the compound of Intermediate Example 5 (0.17 g, 0.51 mmol, 1.1 eq) in dioxane (5 ml) was degassed by N₂ bubbling for 5 min. The compound of Intermediate Example 12 (100 mg, 0.46 mmol) was added and the mixture was degassed for another 5 min. CuI (8 mg, 0.04 mmol, 0.1 eq), N,N-dimethyl glycine (4 mg, 0.04 mmol, 0.1 eq) and Cs₂CO₃ (450 mg, 1.4 mmol, 3.0 eq) were added and the mixture was further degassed for 5 min and then heated at 100 °C for 24 h. The mixture was filtered through celite and washed with 3 % methanol/chloroform to afford crude product mixture which was recrystallized from diethyl ether to afford the product mixture in 40 % yield (80 mg) which was directly taken for the next step. LC-MS (ESI): Calculated mass: 450.1; Observed mass: 451.3 [M+H]⁺ (rt: 1.65 min).

### b) N-(4-(2,4-difluorophenyl)-6-(5-(1-methyl-1H-1,2,3-triazol-4-yl)-1H-benzo[d]-imidazol-1-yl)pyridin-2-yl)cyclopropanesulfonamide

A mixture of Example 36(a) (0.1 g, 0.22 mmol), sodium azide (28 mg, 0.44 mmol, 2.0 eq), methyl iodide (31 mg, 0.22 mmol, 1.0 eq), sodium ascorbate (43 mg, 0.022 mmol, 0.1 eq) and copper sulfate pentahydrate (5 mg, 0.022 mmol, 0.1 eq) in DMSO and water (1:0.5, 3 ml) was stirred for 12 h at RT. The mixture was quenched with water and the precipitate formed was filtered and dried. The crude product mixture was purified by by column chromatography (60-120 silica gel, 2 % methanol in CHCl₃) to give the title product in 6.3 % yield (7 mg). ¹H NMR (400 MHz, DMSO-*d*₆): δ 11.18 (s, 1H), 9.11 (s, 1H), 8.79 (d, 1H), 8.62 (s, 1H), 8.21 (d, 1H), 7.90-7.85 (m, 2H), 7.79 (s, 1H), 7.56-7.50 (m, 1H), 7.38-7.33 (m, 1H), 7.13 (s, 1H), 4.12 (s, 3H), 3.34-3.13 (m, 1H), 1.16-1.12 (m, 2H), 1.06-0.94 (m, 2H); LC-MS (ESI): Calculated mass: 507.13; Observed mass: 508.1 [M+H]⁺ (rt: 1.52 min).

### Example 37.

### N-(4-(2,4-difluorophenyl)-6-(5-(1-methyl-1H-imidazol-4-yl)-1H-benzo[d]-imidazol-1-yl)pyridin-2-yl)cyclopropanesulfonamide

A solution of the compound of Intermediate Example 5 (34 mg, 0.1 mmol) in DMF (5 ml) was degassed by N₂ bubbling for 5 min. The compound of Intermediate Example 11 (20 mg, 0.1 mmol, 1 eq) was added and the mixture was degassed for another 5 min. CuI (2 mg, 0.01 mmol), 0.1 eq), N,N-dimethyl glycine (0.52 mg, 0.005 mmol, 0.05 eq) and Cs₂CO₃ (82 mg, 0.25 mmol, 2.5 eq) were added and the mixture was further degassed for 5 min and then heated art 100 °C for 24 h. The mixture was filtered through celite and washed with ethyl acetate. The solvent was distilled off to afford the crude product mixture which was purified by preparative TLC to yield the title compound in 10 % yield (5 mg). ¹H NMR (400 MHz, DMSO-*d*₆): δ 11.11 (s, 1H), 9.02 (s, 1H), 8.66 (d, 1H), 8.09 (d, 1H), 7.89-7.84 (m, 1H), 7.81-7.75 (m, 2H), 7.68-7.64 (m, 2H), 7.54-7.48 (m, 1H), 7.37-7.32 (m, 1H), 7.13 (s, 1H), 3.71 (s, 3H), 3.18-3.14 (m, 1H), 1.15-1.12 (m,2H), 1.06-1.02 (m, 2H); LC-MS (ESI): Calculated mass: 506.13; Observed mass: 507.35 [M+H]⁺ (rt: 0.18 min).

### Example 38.

### N-(4-(2,4-difluorophenyl)-6-(5-(1-methyl-1H-pyrazol-4-yl)-1H-benzo[d]-imidazol-1-yl) pyrimidin-2-yl) acetamide

### a) N-(4, 6-dichloropyrimidin-2-yl) acetamide

To a solution of 4, 6-dichloropyrimidin-2-amine (5 g, 30.48 mmol) in toluene (50 ml) was added acetic anhydride (15 ml, 152.43 mmol) and the mixture was heated at 120 °C for 16 h. The solvent was evaporated and the crude product was taken in hexane (50 ml) and dichloromethane (6 ml) and filtered to afford the title product in 80 % yield (5 g). LC-MS (ESI): Calculated mass: 206.0; Observed mass: 208.0 [M+H]⁺ (rt: 0.245 min).

### b) N-(4-((2-amino-4-(1-methyl-1H-pyrazol-4-yl) phenyl) amino)-6-chloro-pyrimidin-2-yl) acetamide

A solution of the compound of Intermediate Example 13 (1.4 g, 7.44 mmol), the compound of Example 38(a) (1.53 g, 7.44 mmol,) and sodium bicarbonate (1.56 g, 18.6 mmol, 2.5 eq) in ethanol was heated at 80 °C for 16 h. The mixture was quenched with water and extracted with ethyl acetate (3 × 100 ml). The combined organic layer was washed with water, brine and dried over sodium sulphate. The solvent was distilled off to afford the crude residue which was purified by column chromatography (60-120 silica gel, 3 % methanol in DCM) to afford the the title product in 19.2 % yield (0.5 g). LC-MS (ESI): Calculated mass: 357.11; Observed mass: 358.1 [M+H]⁺ (rt: 0.123 min).

### c) N-(4-chloro-6-(5-(1-methyl-1H-pyrazol-4-yl)-1H-benzo[d]imidazol-1-yl) pyrimidin-2-yl) acetamide

A mixture of the compound of Example 38(b) (0.15 g, 0.42 mmol) and formic acid (2 ml) was heated at 80 °C for 2 h. The formic acid was distilled off and the crude was dissolved in ethyl acetate. The ethyl acetate layer was washed with water, brine and dried over sodium sulphate. The solvent was distilled off to afford the title product in 97 % yield (0.15 g). LC-MS (ESI): Calculated mass: 367.07; Observed mass: 368.1 [M+H] ⁺ (rt: 0.318 min).

### d) N-(4-(2,4-difluorophenyl)-6-(5-(1-methyl-1H-pyrazol-4-yl)-1H-benzo[d]-imidazol-1-yl) pyrimidin-2-yl) acetamide

A solution of the compound of Example 38(c) (0.08 g, 0.217 mmol) in dioxane (5 ml) was degassed by N₂ bubbling for 5 min. 2,4-Difluorophenylboronic acid (0.04 g, 0.261 mmol, 1.2 eq) was added and the mixture was degassed for another 5 min. Pd(PPh₃)₄ (0.025 g, 0.021mmol), 0.1 eq) and aqueous cesium carbonate (0.106 g, 0.326mmol, 1.5 eq) were added sequentially and the mixture was further degassed for 5 min and then heated at 100 °C for 2 h. The reaction mixture was quenched with water and extracted with ethyl acetate (3 × 30 ml). The combined organic layer was washed with water, brine and dried over sodium sulphate. The solvent was distilled off to afford the crude residue which was purified by preparative HPLC to afford the title product in 10.4 % yield (10 mg). ¹H NMR (400 MHz, DMSO- *d*₆): δ 11.1 (s, 1H), 9.25 (s, 1H), 9.12 (d, 1H), 8.23 (s, 1H), 8.11-8.07 (m, 1H), 8.03 (s, 1H), 7.96 (d, 2H), 7.63 (d, 1H), 7.54 (t, 1H), 7.36 (t, 1H), 3.88 (s, 3H), 2.27 (s, 3H); LC-MS (ESI): Calculated mass: 445.15; Observed mass: 445.9 [M+H]⁺ (rt: 1.33 min).

### Example 39.

### Ethyl 1-(1-(6-(cyclopropanesulfonamido)-4-(2,4-difluorophenyl)pyridin-2-yl)-1H-benzo[d]imidazol-5-yl)-1H-1,2,3-triazole-4-carboxylate

A solution of the compound of Intermediate Example 5 (200 mg, 0.58 mmol) in DMF (5 ml) was degassed by N₂ bubbling for 5 min. The compound of Intermediate Example 14 (149 mg, 0.58 mmol, 1 eq) was added and the mixture was degassed for another 5 min. CuI (11 mg, 0.05 mmol, 0.1 eq), N,N-dimethyl glycine (8 mg, 0.05 mmol, 0.1 eq) and Cs₂CO₃ (570 mg, 1.74 mmol, 3 eq) were added and the mixture was further degassed for 5 min and then heated at 100 °C for 24 h. The mixture was filtered through celite and washed with ethyl acetate. The solvent was distilled off to afford the crude product mixture which was purified by preparative HPLC to afford the title product in 5 % yield (18 mg). ¹H NMR (400 MHz, DMSO-*d*₆): δ 11.11 (s, 1H), 9.59 (d, 1H), 9.26 (s, 1H), 8.95 (d, 1H), 8.38 (s, 1H), 8.02-8.00 (m, 1H), 7.92-7.83 (m, 2H), 7.56-7.51 (m, 1H), 7.51-7.34 (m, 1H), 7.17 (s, 1H), 4.38 (quartet, 2H), 3.17-3.13 (m, 1H), 1.36 (t, 3H), 1.13-1.12 (m, 2H), 1.04-1.02 (m, 2H); LC-MS (ESI): Calculated mass: 565.13; Observed mass: 566.2 [M+H]⁺ (rt: 1.63 min).

### Example 40.

### N-(4-(2-(difluoromethoxy)-4-fluorophenyl)-6-(5-(1-methyl-1H-pyrazol-4-yl)-1H-benzo [d]imidazol-1-yl)pyridin-2-yl)cyclopropanesulfonamide

A solution of the compound of Intermediate Example 15 (300 mg, 0.75 mmol) in DMF (10 ml) was degassed by N₂ bubbling for 5 min. The compound of Intermediate Example 6 (149 mg, 0.75 mmol, 1 eq) was added and the mixture was degassed for another 5 min. CuI (14 mg, 0.075 mmol, 0.1 eq), N,N-dimethyl glycine (8 mg, 0.075 mmol, 0.1 eq) and Cs₂CO₃ (730 mg, 2.25 mmol, 3 eq) were added and the reaction mixture was further degassed for 5 min and then heated at 100 °C for 2 days. The reaction mixture was filtered through celite and washed with ethyl acetate. The solvent was distilled off to afford the crude product mixture which was purified by column chromatography (60-120 silica gel, 2 % methanol in chloroform) to afford the title product in 2 % yield (8 mg). ¹H NMR (400 MHz, CD₃OD): δ 8.83 (s, 1H), 8.56 (d, 1H), 8.02 (s, 1H), 7.89-7.87 (m, 2H), 7.69-7.63 (m, 2H), 7.57 (s, 1H), 7.22-7.15 (m, 2H), 7.15 (s, 1H), 6.97 (s, 0.5 H), 6.79 (s, 0.5 H), 3.94 (s, 3H), 3.15-3.05 (m, 1H), 1.28-1.15 (m, 2H), 1.00-0.87 (m, 2H); LC-MS (ESI): Calculated mass: 554.13; Observed mass: 555.5 [M+H]⁺ (rt: 1.66 min).

### Example 41.

### N-(4-(2,4-difluorophenyl)-6-(5-(1-methyl-1H-pyrazol-4-yl)-1H-benzo[d]-imidazol-1-yl)pyrimidin-2-yl)cyclopropanesulfonamide

### a) 4-Chloro-6-(2,4-difluorophenyl)pyrimidin-2-amine

A solution of 4,6-dichloropyrimidin-2-amine (5 g, 30 mmol) in 1,2-dimethoxyethane (50 ml) was degassed by N₂ bubbling for 5 min. 2,4-Difluorophenylboronic acid (4.38 g, 27 mmol, 0.9 eq) was added and the mixture was degassed for another 5 min. Pd(dppf)Cl₂ (1.38 g, 1.5 mmol, 0.05 eq) and aqueous sodium carbonate (4.9 g, 46 mmol, 1.5 eq) were added sequentially using the procedure of Intermediate Example 1 and heated at 90 °C for 6 h. The reaction mixture was quenched and extracted as in Intermediate Example 1. The solvent was distilled off to afford the crude residue which was purified by column chromatography (60-120 silica gel, 70 % ethyl acetate in hexane) to afford the title product in 70 % yield (4 g). LC-MS (ESI): Calculated mass: 241.02; Observed mass: 242.05 [M+H]⁺ (rt: 1.58 min).

### b) 6-(2,4-Difluorophenyl)-N4-(4-(1-methyl-1H-pyrazol-4-yl)-2-nitrophenyl)-pyrimidine-2,4-diamine

A solution of the compound of Example 41 (a) (0.5 g, 2.1 mmol) in dioxane (5 ml) was degassed by N₂ bubbling for 5 min. The compound of Intermediate Example 1 (0.5 g, 2.3 mmol, 1.1 eq) was added and the mixture was degassed for another 5 min. Pd₂dba₃ (0.19 g, 0.2 mmol, 0.1 eq) and xantphos (0.24 g, 0.4 mmol, 0.2 eq) and cesium carbonate (1.68 g, 5.2 mmol, 2.5 eq) were added sequentially and the mixture was further degassed for 5 min and then heated at 110 °C for 16 h. The mixture was filtered through celite pad and extracted with ethyl acetate (3 × 50 ml). The combined organic layer was washed with water, brine and dried over sodium sulphate. The solvent was distilled off to afford the crude residue which was purified by column chromatography (60-120 silica gel, 70 % ethyl acetate in hexane) in 22.5 % yield (0.2 g). LC-MS (ESI): Calculated mass: 423.13; Observed mass: 423.9 [M+H]⁺ (rt: 0.26 min).

### c) N4-(2-amino-4-(1-methyl-1H-pyrazol-4-yl)phenyl)-6-(2,4-difluorophenyl)-pyrimidine-2,4-diamine

To a solution of the compound of Example 41(b) (0.12 g, 0.3 mmol) in THF (15 ml) were added a solution of ammonium chloride (0.12 g, 2.3 mmol, 8 eq) in water (5 ml) and zinc (0.145 g, 2.3 mmol, 8 eq). The mixture was stirred at RT for 2 h and filtered. The filtrate was diluted with water and extracted with ethyl acetate (3 × 100 ml). The combined organic layer was washed with water, brine and dried over sodium sulphate. The solvent was distilled off to afford the crude product in 83 % yield (0.1 g). LC-MS (ESI): Calculated mass: 393.15; Observed mass: 394.3 [M+H]⁺ (rt: 0.14 min).

### d) 4-(2,4-difluorophenyl)-6-(5-(1-methyl-1H-pyrazol-4-yl)-1H-benzo[d]-imidazol-1-yl)pyrimidin-2-amine

A solution of the compound of Example 41(c) (0.1 g, 0.2 mmol) in formic acid (5 ml) was heated at 100°C for 16 h. The formic acid was distilled off and the crude product was dissolved in ethyl acetate. The ethyl acetate layer was washed with water, brine and dried over sodium sulphate. The solvent was distilled off to afford the title product in 24 % yield (20 mg).

### e) N-(4-(2,4-difluorophenyl)-6-(5-(1-methyl-1H-pyrazol-4-yl)-1H-benzo[d]-imidazol-1-yl)pyrimidin-2-yl)cyclopropanesulfonamide

To an icecold solution of the compound of Example 41 (d) (50 mg, 0.1 mmol) in DMF (50 ml) was added NaH (4 mg, 0.2 mmol, 2 eq). The mixture was stirred for 10 min and cyclopropylsulfonyl chloride (26 mg, 0.2 mmol, 2 eq) was added and the mixture was stirred at RT for 12 h. The mixture was then quenched with water and extracted with ethyl acetate (3 × 100 ml). The combined organic layer was washed with water, brine and dried over sodium sulphate. The solvent was distilled off to afford the crude product which was purified by preparative HPLC to afford the title product in 6 % yield (3 mg). ¹H NMR (400 MHz, CDCl₃): δ 8.73 (s, 1H), 8.39 (d, 1H), 8.36-8.27 (m, 1H), 8.09 (s, 1H), 7.99 (s, 1H), 7.93 (d, 1H), 7.87 (d, 1H), 7.74 (d, 1H), 7.67-7.64 (m, 1H), 7.20-7.01 (m, 2H), 3.83 (s, 3H), 3.30-3.26 (m, 1H), 1.51-1.42 (m, 2H), 1.16-1.12 (m, 2H); LC-MS (ESI): Calculated mass: 507.13; Observed mass: 508.2 [M+H]⁺ (rt: 1.48min).

### Example 42.

### Ethyl 1-(1-(6-acetamido-4-(2,4-difluorophenyl)pyridin-2-yl)-1H-benzo[d]-imidazol-5-yl)-1H-1,2,3-triazole-4-carboxylate

A solution of the compound of Intermediate Example 16 (150 mg, 0.53 mmol) in DMF (5 ml) was degassed by N₂ bubbling for 5 min. The compound of Intermediate Example 6 (137 mg, 0.53 mmol, 1 eq) was added and the mixture was degassed for another 5 min. CuI ( 10 mg, 0.05 mmol, 0.1 eq), N,N-dimethyl glycine (7 mg, 0.05 mmol, 0.1 eq) and Cs₂CO₃ (0.52 g, 1.59 mmol, 3 eq) were added and the mixture was further degassed for 5 min and then heated at 110 °C for 16 h. The mixture was filtered through celite and washed with ethyl acetate. The solvent was distilled off to afford the crude product mixture which was purified by preparative HPLC to yield the title product in 4.5 % yield (16 mg). ¹H NMR (400 MHz, DMSO-*d*₆): δ 10.95 (s, 1H), 9.58 (d, 1H), 9.22 (s, 1H), 8.98 (d, 1H), 8.39 (s, 1H), 8.03-7.96 (m, 2H), 7.89-7.83 (m, 2H), 7.55-7.49 (m, 1H), 7.47-7.33 (m, 1H), 4.39 (quartet, 2H), 2.22 (s, 1H), 1.36 (t, 3H); LC-MS (ESI): Calculated mass: 503.15; Observed mass: 504.1 [M+H]⁺ (rt: 1.63 min).

### Example 43.

### N-(6-(2, 4-difluorophenyl)-4-(5-(1-methyl-1H-pyrazol-4-yl)-1H-benzo[d]-imidazol-1-yl) pyridin-2-yl) cyclopropanesulfonamide

### a) 4-(1-methyl-1H-pyrazol-4-yl)-2-nitroaniline

A solution of 4-bromo-2-nitroaniline (6 g, 27.6 mmol) in 1, 2-dimethoxyethane (15 ml) was degassed by N₂ bubbling for 5 min. 1-Methyl-4-(4,4,5,5-tetramethyl-1,3,2-dioxaborolan-2-yl)-1H-pyrazole (6.90 g, 33.1 mmol, 1.2 eq.) was added and the mixture was degassed for another 5 min. Pd(dppf)Cl₂ (2.25 g, 27.6 mmol, 0.1 eq) and aqueous sodium carbonate (8.79 g, 82.9 mmol, 3.0 eq) were added sequentially and the mixture was further degassed for 5 min and then heated at 90 °C for 2 h. The mixture was quenched with water and extracted with ethyl acetate (3 × 50 ml). The combined organic layer was washed with water, brine and dried over sodium sulphate. The solvent was distilled off under reduced pressure to afford the crude residue which was purified by column chromatography (60-120 silica gel, 40 % ethyl acetate in hexane) to yield the title product in 75 % yield (4.5 g). LC-MS (ESI): Calculated mass: 218.21; Observed mass: 218.9 [M+H]⁺ (rt: 0.390 min). ¹H NMR (400 MHz, DMSO- *d*₆): δ 8.10-8.08 (m, 2H), 7.81 (s, 1H), 7.66-7.63 (m,1H), 7.44(s, 2H), 7.05-7.03(d, 1H), 3.84 (s, 3H).

### b) 2,6-dichloro-N-(4-(1-methyl-1H-pyrazol-4-yl)-2-nitrophenyl)pyridin-4-amine

A solution of 2,6-dichloro-4-iodopyridine (1 g, 3.66 mmol) in toluene (15 ml) was degassed by N₂ bubbling for 5 min. The compound of Example 43(a) (0.958 g, 4.39 mmol, 1.2 eq.) was added and the mixture was degassed for another 5 min. Palladium acetate (0.032 g, 0.146 mmol, 0.04 eq) and BINAP (0.091 g, 0.146 mmol, 0.04 eq.) and potassium tert-butoxide (0.616 g, 5.49 mmol, 1.5 eq) were added sequentially and the mixture was further degassed for 5 min and then heated at 100°C for 5 h. The mixture was filtered through celite and extracted with ethyl acetate (3 × 100 ml). The combined organic layer was washed with water, brine and dried over sodium sulphate. The solvent was distilled off under reduced pressure to afford the crude residue which was purified by column chromatography (60-120 silica gel, 25 % ethyl acetate in hexane) in 42.3 % yield (550 mg). LC-MS (ESI): Calculated mass: 363.04; Observed mass: 364.0 [M+H]⁺ (rt: 1.578 min).

### c) 1-(2, 6-dichloropyridin-4-yl)-5-(1-methyl-1H-pyrazol-4-yl)-1H-benzo[d]-imidazole

A solution of the compound of Example 43(b) (0.55 g, 1.51 mmol) in formic acid (10 ml) and iron (0.843 g, 15.1 mmol) was heated at 100°C for 16 h. The formic acid was distilled off under reduced pressure and the crude product was dissolved in ethyl acetate. The ethyl acetate layer was washed with water, brine and dried over sodium sulphate. The solvent was distilled off under reduced pressure to afford the title compound in 77 % yield (0.4 g). LC-MS (ESI): Calculated mass: 343.04; Observed mass: 344.05 [M+H] ⁺ (rt: 1.169 min).

### d) N-(6-chloro-4-(5-(1-methyl-1H-pyrazol-4-yl)-1H-benzo[d]imidazol-1-yl) pyridin-2-yl) cyclopropanesulfonamide

A solution of the compound of Example 43(c) (0.54 g, 1.56 mmol) in dioxane (15 ml) was degassed by N₂ bubbling for 5 min. Cyclopropane sulfonamide (0.189g, 1.56 mmol, 1 eq) was added and the mixture was degassed for another 5 min. Palladium acetate (0.028 g, 0.125 mmol, 0.08 eq) and xantphos (0.072 g, 0.125 mmol, 0.08 eq) and Cs₂CO₃ (1.53 g, 4.7 mmol, 3.0 eq) were added and the mixture was further degassed for 5 min and then heated at100°C for 16 h. The mixture was filtered through celite and extracted with ethyl acetate (3 × 50 ml). The combined organic layer was washed with water, brine and dried over sodium sulphate. The solvent was distilled off under reduced pressure to afford the crude residue which was purified by column chromatography (60-120 silica gel, 3 % methanol in hexane) to yield the title product in 37 % yield (250 mg). LC-MS (ESI): Calculated mass: 428.08; Observed mass: 429.2 [M+H]⁺ (rt: 0.854 min).

### e) N-(6-(2,4-difluorophenyl)-4-(5-(1-methyl-1H-pyrazol-4-yl)-1H-benzo[d]-imidazol-1-yl) pyridin-2-yl) cyclopropanesulfonamide

A solution of the compound of Example 43(d) (0.15 g, 0.350 mmol) in DME (4 ml) was degassed by N₂ bubbling for 5 min. 2,4-Difluorophenylboronic acid (0.06 g, 0.385 mmol, 1.1 eq) was added and the mixture was degassed for another 5 min. Pd(PPh₃)₄ (0.039 g, 0.035mmol, 0.1 eq) and aqueous cesium carbonate (0.341 g, 1.051 mmol, 3 eq) were added sequentially and the mixture was further degassed for 5 min and then heated at 100 °C for 16 h. The reaction mixture was quenched with water and extracted with ethyl acetate (3 × 30 ml). The combined organic layer was washed with water, brine and dried over sodium sulphate. The solvent was distilled off under reduced pressure to afford the crude residue which was purified by preparative TLC to yield the title product in 11.2 % yield (20mg). ¹H NMR (400 MHz, DMSO- *d*₆): δ 11.0 (s, 1H), 8.78 (s, 1H), 8.21 (s, 1H), 8.08-8.04 (m, 1H), 8.00 (s, 1H), 7.95 (s, 1H), 7.78-7.76 (d, 2H), 7.65-7.63 (d, 1H), 7.49-7.43 (m, 1H), 7.35-7.29 (m, 2H), 3.87 (s, 3H), 3.23 (m,1H), 1.13-1.06 (m,4H). LC-MS (ESI): Calculated mass: 506.13; Observed mass: 507.5 [M+H]⁺ (rt: 1.583 min).

### Abbreviations:

RT - Room temperature
rt - Retention time
BINAP - 2,2'-bis(diphenylphosphino)-1,1'-binaphthyl
DMF - *N*,*N*-dimethylformamide
THF - Tetrahydrofuran
TEA - Triethyl amine
TLC - Thin layer chromatography
DCM - Dichloromethane
DME - Dimethoxyethane
DMSO - Dimethylsulfoxide
EDC - 1-Ethyl-3-(3-dimethylaminopropyl) carbodiimide hydrochloride
HATU - 2-(1H-7-azabenzotriazol-1-yl)-1,1,3,3-tetramethyl uronium hexafluorophosphate methanaminium
HOBt - Hydroxybenzotriazole
DIPEA - *N*,*N*-diisopropylethylamine
TBAF - tetra-n-butylammonium fluoride
Pd(dppf)Cl₂ - 1,1'-Bis(diphenylphosphino)ferrocene-palladium(II)dichloride
Pd(PPh₃)₄ - Tetrakis(triphenylphosphine)palladium(0)
Pd₂(dba)₃ - Tris(dibenzylideneacetone)dipalladium(0)

## Claims

1. A compound which is of formula (I) 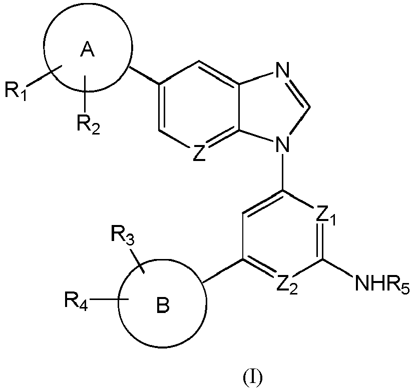 wherein
Z₁ is N and Z₂ is CH, or
Z₁ is CH and Z₂ is N, or
Z₁ and Z₂ is N;
Z is CH or N;
A is a phenyl ring or a 5 -12 membered heterocyclic ring;
R₁ is H, C₁₋₇ alkyl, C₃₋₇ cycloalkyl, C₃₋₇ cycloalkyl C₁₋₇ alkyl, C₁₋₇ alkoxy, C₁₋₇ alkyl carbonyl, amino, hydroxy, hydroxy C₁₋₇ alkyl, halo C₁₋₇ alkyl, C₁₋₇ alkylamino C₁₋₇ alkyl, -R₁₆-C(O)-R₁₇ or -E-R₆;
R₂ is H, halogen or C₁₋₇ alkyl;
B is a 5-12 membered carbocyclic ring and R₃ is halogen, C₁₋₇ alkyl, C₁₋₇ alkoxy, halo C₁₋₇ alkyl or halo C₁₋₇ alkoxy; or B is a 5-12 membered heterocyclic ring and R₃ is H, halogen, C₁₋₇ alkyl, C₁₋₇ alkoxy, halo C₁₋₇ alkyl or halo C₁₋₇ alkoxy;
R₄ is H, halogen, C₁₋₇ alkyl or oxo;
R₅ is H, -C(O)R₇, -SO₂R₈ or an optionally substituted 5-6 membered heterocyclic ring;
R₆ is an optionally substituted 5-6 membered heterocyclic ring;
R₇ is C₁₋₇ alkyl, C₂₋₇ alkenyl, C₁₋₇ alkoxy, C₁₋₇ alkoxy C₁₋₇ alkyl, carboxy C₁₋₇ alkyl, C₁₋₇ alkoxy carbonyl C₁₋₇ alkyl, C₁₋₇ alkylamino C₁₋₇ alkyl, -NH-R₁₀ or -NH-X₁-R₁₁;
R₈ is C₁₋₇ alkyl, C₂₋₇ alkenyl, C₃₋₇ cycloalkyl, hydroxy C₁₋₇ alkyl, -NR₁₃R₁₄, -NH-X₂-R₁₅, phenyl or an optionally substituted 5-6 membered heterocyclic ring;
R₁₀ is C₁₋₇ alkyl or C₃₋₇ cycloalkyl;
R₁₁ is phenyl or an optionally substituted 5-6 membered heterocyclic ring;
R₁₃ and R₁₄ are, independently, H, C₁₋₇ alkyl or C₃₋₇ cycloalkyl;
R₁₅ is phenyl or an optionally substituted 5-6 membered heterocyclic ring;
R₁₆ is a bond or a C₁₋₇ alkyl;
R₁₇ is C₁₋₇ alkyl, C₁₋₇ alkoxy, C₁₋₇ alkylamino, amino or hydroxy;
E is a bond or a C₁₋₇ alkyl;
X₁ and X₂ are, independently, a bond or C₁₋₇ alkyl;
or is a pharmaceutically acceptable salt thereof.

2. A compound according to claim 1, wherein Z is CH.

3. A compound according to claim 1 or 2, wherein Z₁ is N and Z₂ is CH.

4. A compound according to to claim 1 or 2, wherein Z₁ is CH and Z₂ is N.

5. A compound according to to claim 1 or 2, wherein Z₁ and Z₂ is N.

6. A compound according to any of claims 1 to 5, wherein ring A is any one of the following groups or tautomers thereof

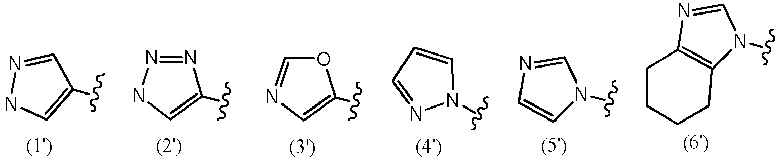

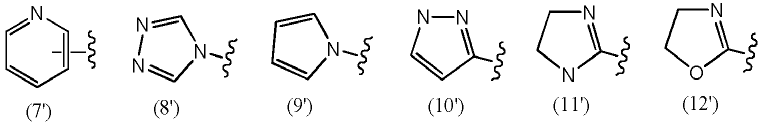

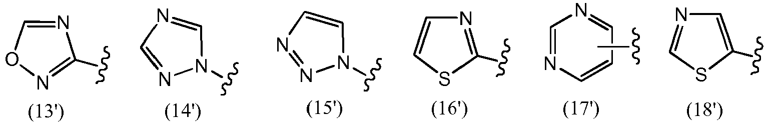

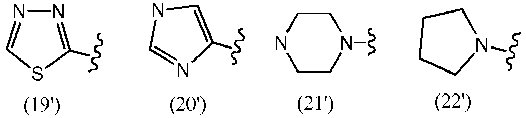

7. A compound according to any of claims 1 to 6, wherein ring B is any one of the following groups or tautomers thereof

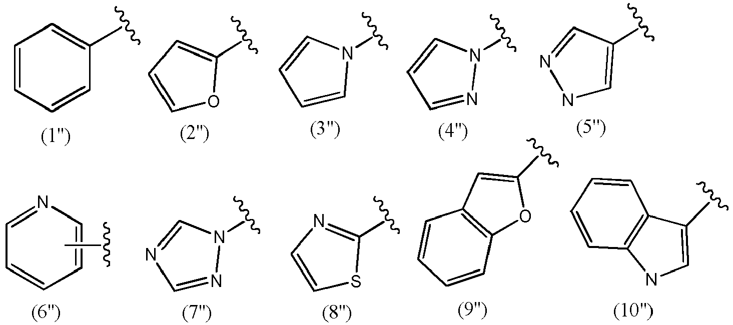

8. A compound according to any of claims 1 to 7, wherein
A is a ring of formula (1'), (2'), (3'), (4'), (5'), (7'), (14'), (16') or (20');
R₁ is H, C₁₋₇ alkyl, C₁₋₇ alkoxy, hydroxy C₁₋₇ alkyl, C₁₋₇ alkylamino C₁₋₇ alkyl or -E-R₆;
R₂ is H;
Z is CH;
B is a ring of formula (1 "), (2"), (3"), (4") or (6");
E is a bond or C₁₋₇ alkyl;
R₆ is any of the following groups 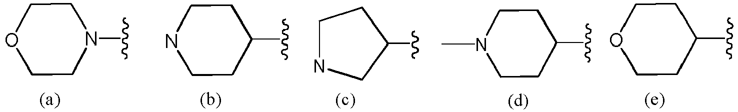 R₃ is H, halogen, C₁₋₇ alkyl, C₁₋₇ alkoxy;
R₄ is H or halogen;
R₅ is -C(O)R₇ or -SO₂R₈ or any one of the following groups 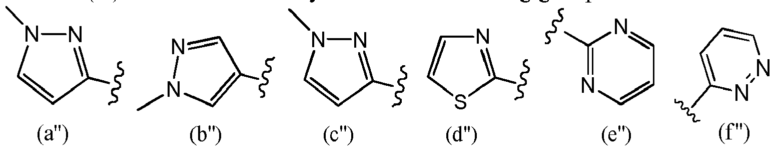 R₇ is C₁₋₇ alkyl, C₂₋₇ alkenyl or -NH-R₁₀;
R₈ is C₁₋₇ alkyl, C₂₋₇ alkenyl, C₃₋₇ cycloalkyl, hydroxy C₁₋₇ alkyl or -NR₁₃R₁₄; and
R₁₀ is C₁₋₇ alkyl or C₃₋₇ cycloalkyl.

9. A compound according to any of claims 1 to 8, wherein B is a ring of formula (1") (3 ") or (6").

10. A compound according to any of claims 1 to 9, wherein A is a ring of formula (1'), (2'), (4'), (5') or (20").

11. A compound according to any of claims 1 to 10, wherein R₅ is -SO₂R₈.

12. A compound according to any of claims 1 to 11, wherein Z is CH, Z₁ is N and Z₂ is CH, A is a ring of formula (1'), B is a ring of formula (1"), R₁ is C₁₋₇ alkyl, R₂ is H, R₃ is halogen, R₄ is H or halogen, R₅ is -SO₂R₈ and R₈ is C₁₋₇ alkyl or
C₃₋₇ cycloalkyl.

13. A compound according to claim 1, wherein B is a 5-12 membered heterocyclic ring.

14. A compound according to claim 1, wherein R₃ is halogen, C₁₋₇ alkyl, C₁₋₇ alkoxy, halo C₁₋₇ alkyl or halo C₁₋₇ alkoxy.

15. A compound according to any of claims 1 to 12, which is
4-(2, 4-Difluorophenyl)-N-(1-methyl-1H-pyrazol-3-yl)-6-(5-(1-methyl-1H-pyrazol-4-yl)-1H-benzo[d]imidazol-1-yl) pyridin-2-amine;
N-(4-(2,4-difluorophenyl)-6-(5-(1-methyl-1H-pyrazol-4-yl)-1H-benzo[d]imidazol-1-yl)pyridin-2-yl)cyclopropanesulfonamide;
Sodium salt of imido form of N-(4-(2,4-difluorophenyl)-6-(5-(1-methyl-1H-pyrazol-4-yl)-1H-benzo[d]imidazol-1-yl)pyridin-2-yl)cyclopropanesulfonamide;
N-(4-(2,4-difluorophenyl)-6-(5-(1-methyl-1H-pyrazol-4-yl)-1H-benzo[d]imidazol-1-yl)pyridin-2-yl)methanesulfonamide;
N-(4-(2,4-difluorophenyl)-6-(5-(1-methyl-1H-pyrazol-4-yl)-1H-benzo[d]imidazol-1-yl)pyridin-2-yl)ethanesulfonamide;
Sodium salt of imido form of N-(4-(2,4-difluorophenyl)-6-(5-(1-methyl-1H-pyrazol-4-yl)-1H-benzo[d]imidazol-1-yl)pyridin-2-yl)ethanesulfonamide;
N-(4-(2,4-difluorophenyl)-6-(5-(1-methyl-1H-pyrazol-4-yl)-1H-benzo[d]imidazol-1-yl)pyridin-2-yl)propane-2-sulfonamide;
Imido form of N-(4-(2,4-difluorophenyl)-6-(5-(1-methyl-1H-pyrazol-4-yl)-1H-benzo[d]-imidazol-1-yl)pyridin-2-yl)propane-2-sulfonamide;
N-(4-(2-fluorophenyl)-6-(5-(1-methyl-1H-pyrazol-4-yl)-1H-benzo[d]imidazol-1-yl)pyridin-2-yl)cyclopropanesulfonamide;
Sodium salt of imido form of N-(4-(2-fluorophenyl)-6-(5-(1-methyl-1H-pyrazol-4-yl)-1H-benzo[d]imidazol-1-yl)pyridin-2-yl)cyclopropanesulfonamide;
N-(4-(2-fluorophenyl)-6-(5-(1-methyl-1H-pyrazol-4-yl)-1H-benzo[d]imidazol-1-yl)pyridin-2-yl)methanesulfonamide;
N-(4-(2-fluorophenyl)-6-(5-(1-methyl-1H-pyrazol-4-yl)-1H-benzo[d]imidazol-1-yl)pyridin-2-yl)ethanesulfonamide;
Sodium salt of imido form of N-(4-(2-fluorophenyl)-6-(5-(1-methyl-1H-pyrazol-4-yl)-1H-benzo[d]imidazol-1-yl)pyridin-2-yl)ethanesulfonamide;
N-(4-(2-fluorophenyl)-6-(5-(1-methyl-1H-pyrazol-4-yl)-1H-benzo[d]imidazol-1-yl)pyridin-2-yl)propane-2-sulfonamide;
Sodium salt of imido form of N-(4-(2-fluorophenyl)-6-(5-(1-methyl-1H-pyrazol-4-yl)-1H-benzo[d]imidazol-1-yl)pyridin-2-yl)propane-2-sulfonamide;
N-(4-(4-fluorophenyl)-6-(5-(1-methyl-1H-pyrazol-4-yl)-1H-benzo[d]imidazol-1-yl)pyridin-2-yl)cyclopropanesulfonamide;
N-(4-(4-fluorophenyl)-6-(5-(1-methyl-1H-pyrazol-4-yl)-1H-benzo[d]imidazol-1-yl)pyridin-2-yl)methanesulfonamide;
N-(4-(4-fluorophenyl)-6-(5-(1-methyl-1H-pyrazol-4-yl)-1H-benzo[d]imidazol-1-yl)pyridin-2-yl)propane-2-sulfonamide;
Sodium salt of imido form of N-(4-(4-fluorophenyl)-6-(5-(1-methyl-1H-pyrazol-4-yl)-1H-benzo[d]imidazol-1-yl)pyridin-2-yl)propane-2-sulfonamide;
N-(3-fluoro-6'-(5-(1-methyl-1H-pyrazol-4-yl)-1H-benzo[d]imidazol-1-yl)-[2,4'-bipyridin]-2'-yl)cyclopropanesulfonamide;
N-(3-fluoro-6'-(5-(1-methyl-1H-pyrazol-4-yl)-1H-benzo[d]imidazol-1-yl)-[2,4'-bipyridin]-2'-yl)acetamide;
N-(4-(2,4-difluorophenyl)-6-(5-(1-(2-(dimethylamino)ethyl)-1H-pyrazol-4-yl)-1H-benzo[d]imidazol-1-yl)pyridin-2-yl)cyclopropanesulfonamide;
N-(4-(2-fluorophenyl)-6-(5-(1-methyl-1H-pyrazol-4-yl)-1H-benzo[d]imidazol-1-yl)pyrimidin-2-yl)cyclopropanesulfonamide;
N-(6-(5-(1H-pyrazol-1-yl)-1H-benzo[d]imidazol-1-yl)-4-(2,4-difluorophenyl)pyridin-2-yl)cyclopropanesulfonamide;
N-(3,5-difluoro-6'-(5-(1-methyl-1H-pyrazol-4-yl)-1H-benzo[d]imidazol-1-yl)-[2,4'-bipyridin]-2'-yl)cyclopropanesulfonamide;
N-(3,5-difluoro-6'-(5-(1-methyl-1H-pyrazol-4-yl)-1H-benzo[d]imidazol-1-yl)-[2,4'-bipyridin]-2'-yl)acetamide;
N-(4-(2-chlorophenyl)-6-(5-(1-methyl-1H-pyrazol-4-yl)-1H-benzo[d]imidazol-1-yl)pyridin-2-yl)cyclopropanesulfonamide;
N-(3-chloro-6'-(5-(1-methyl-1H-pyrazol-4-yl)-1H-benzo[d]imidazol-1-yl)-[2,4'-bipyridin]-2'-yl)cyclopropanesulfonamide;
N-(5-fluoro-6'-(5-(1-methyl-1H-pyrazol-4-yl)-1H-benzo[d]imidazol-1-yl)-[2,4'-bipyridin]-2'-yl)cyclopropanesulfonamide;
N-(6-(5-(1H-imidazol-1-yl)-1H-benzo[d]imidazol-1-yl)-4-(2,4-difluorophenyl)pyridin-2-yl)cyclopropanesulfonamide;
N-(4-(2,4-difluorophenyl)-6-(5-(1-(2-morpholinoethyl)-1H-pyrazol-4-yl)-1H-benzo[d]imidazol-1-yl)pyridin-2-yl)cyclopropanesulfonamide;
N-(4-(2,4-difluorophenyl)-6-(5-(1-(pyrrolidin-3-yl)-1H-pyrazol-4-yl)-1H-benzo[d]-imidazol-1-yl)pyridin-2-yl)cyclopropanesulfonamide;
N-(4-(2,4-difluorophenyl)-6-(5-(1-ethyl-1H-1,2,3-triazol-4-yl)-1H-benzo[d]imidazol-1-yl)pyridin-2-yl)cyclopropanesulfonamide;
N-(4-(2,4-difluorophenyl)-6-(5-(1-methyl-1H-1,2,3-triazol-4-yl)-1H-benzo[d]imidazol-1-yl)pyridin-2-yl)cyclopropanesulfonamide;
N-(4-(2,4-difluorophenyl)-6-(5-(1-methyl-1H-imidazol-4-yl)-1H-benzo[d]imidazol-1-yl)pyridin-2-yl)cyclopropanesulfonamide;
N-(4-(2, 4-difluorophenyl)-6-(5-(1-methyl-1H-pyrazol-4-yl)-1H-benzo[d]imidazol-1-yl) pyrimidin-2-yl) acetamide;
Ethyl 1-(1-(6-(cyclopropanesulfonamido)-4-(2,4-difluorophenyl)pyridin-2-yl)-1H-benzo[d]imidazol-5-yl)-1H-1,2,3-triazole-4-carboxylate;
N-(4-(2-(difluoromethoxy)-4-fluorophenyl)-6-(5-(1-methyl-1H-pyrazol-4-yl)-1H-benzo[d]imidazol-1-yl)pyridin-2-yl)cyclopropanesulfonamide;
N-(4-(2,4-difluorophenyl)-6-(5-(1-methyl-1H-pyrazol-4-yl)-1H-benzo[d]imidazol-1-yl)pyrimidin-2-yl)cyclopropanesulfonamide;
N-(6-(2, 4-difluorophenyl)-4-(5-(1-methyl-1H-pyrazol-4-yl)-1H-benzo[d]imidazol-1-yl) pyridin-2-yl) cyclopropanesulfonamide;
or a pharmaceutically acceptable salt or tautomer thereof.

16. A pharmaceutical composition comprising a compound as defined in any one of claims 1 to 15 with a pharmaceutically acceptable carrier.

17. A compound as defined in any one of claims 1 to 15 for use in a method of treatment of a condition, where FGFR kinase inhibition is desired.

18. A compound as defined in any one of claims 1 to 15 for use in a method of treatment of cancer.

## Patentansprüche

1. Verbindung der Formel (I) 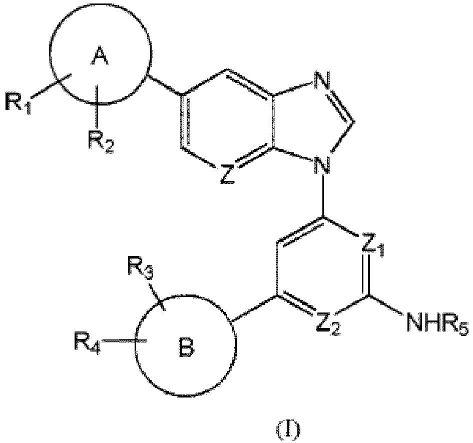 wobei
Z₁ N ist und Z₂ CH ist oder
Z₁ CH ist und Z₂ N ist oder
Z₁ und Z₂ N sind;
Z CH oder N ist;
A ein Phenylring oder ein 5-12-gliedriger heterocyclischer Ring ist;
R₁ Folgendes ist: H, C₁₋₇-Alkyl, C₃₋₇-Cycloalkyl, C₃₋₇-Cycloalkyl, C₁₋₇-Alkyl, C₁₋₇-Alkoxy, C₁₋₇-Alkylcarbonyl, Amino, Hydroxy, Hydroxy-C₁₋₇-Alkyl, Halogen, C₁₋₇-Alkylamino, C₁₋₇-Alkyl, -R₁₆-C(O)-R₁₇ oder -E-R₆;
R₂ H, Halogen oder C₁₋₇-Alkyl ist;
B ein 5- bis 12-gliedriger carbocyclischer Ring ist und R₃ Halogen, C₁₋₇-Alkyl, C₁₋₇-Alkoxy, Halogen-C₁₋₇-Alkyl oder Halogen-C₁₋₇-Alkoxy ist; oder B ein 5- bis 12-gliedriger heterocyclischer Ring ist und R₃ H, Halogen, C₁₋₇-Alkyl, C₁₋₇-Alkoxy, Halogen-C₁₋₇-Alkyl oder Halogen-C₁₋₇-Alkoxy ist;
R₄ H, Halogen, C₁₋₇-Alkyl oder Oxo ist;
R₅ H, -C(O)R₇, -SO₂R₈ oder ein optional substituierter 5-6-gliedriger heterocyclischer Ring ist;
R₆ ein optional substituierter 5-6-gliedriger heterocyclischer Ring ist;
R₇ C₁₋₇-Alkyl, C₂₋₇-Alkenyl, C₁₋₇-Alkoxy, C₁₋₇-Alkoxy-C₁₋₇-Alkyl, Carboxy-C₁₋₇-Alkyl, C₁₋₇-Alkoxycarbonyl-C₁₋₇-Alkyl, C₁₋₇-Alkylamino-C₁₋₇Alkyl, -NH-R₁₀ oder -NH-X₁-R₁₁ ist;
R₈ C₁₋₇-Alkyl, C₂₋₇-Alkenyl, C₃₋₇-Cycloalkyl, Hydroxy-C₁₋₇-Alkyl, -NR₁₃R₁₄, -NH-X₂-R₁₅, Phenyl oder einen optional substituierter 5-6-gliedriger heterocyclischer Ring ist;
R₁₀ C₁₋₇-Alkyl oder C₃₋₇-Cycloalkyl ist;
R₁₁ Phenyl oder ein optional substituierter 5-6-gliedriger heterocyclischer Ring ist;
R₁₃ und R₁₄ unabhängig voneinander H, C₁₋₇-Alkyl oder C₃₋₇-Cycloalkyl sind;
R₁₅ Phenyl oder ein optional substituierter 5-6-gliedriger heterocyclischer Ring ist;
R₁₆ eine Bindung oder ein C₁₋₇-Alkyl ist;
R₁₇ C₁₋₇-Alkyl, C₁₋₇-Alkoxy, C₁₋₇-Alkylamino, Amino oder Hydroxy ist;
E eine Bindung oder ein C₁₋₇-Alkyl ist;
X₁ und X₂ unabhängig voneinander eine Bindung oder C₁₋₇-Alkyl sind;
oder ein pharmazeutisch annehmbares Salz davon ist.

2. Verbindung nach Anspruch 1, wobei Z CH ist.

3. Verbindung nach Anspruch 1 oder 2, wobei Z₁ N ist und Z₂ CH ist.

4. Verbindung nach Anspruch 1 oder 2, wobei Z₁ CH ist und Z₂ N ist.

5. Verbindung nach Anspruch 1 oder 2, wobei Z₁ und Z₂ N sind.

6. Verbindung nach einem der Ansprüche 1 bis 5, wobei der Ring A eine der folgenden Gruppen oder Tautomere davon ist:

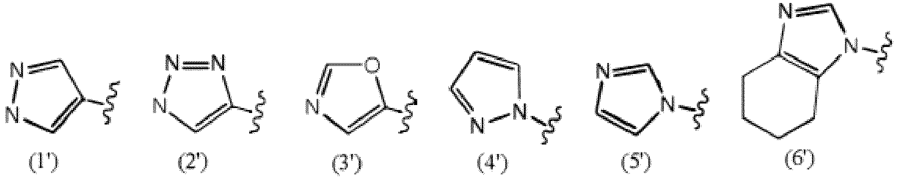

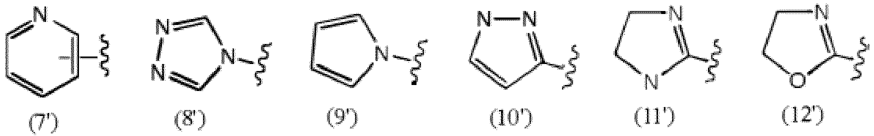

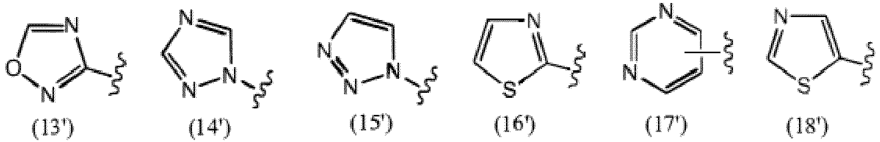

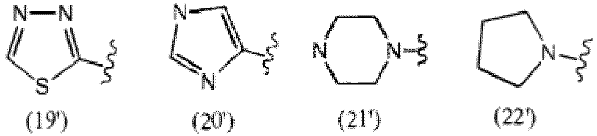

7. Verbindung nach einem der Ansprüche 1 bis 6, wobei der Ring B eine der folgenden Gruppen oder Tautomere davon ist:

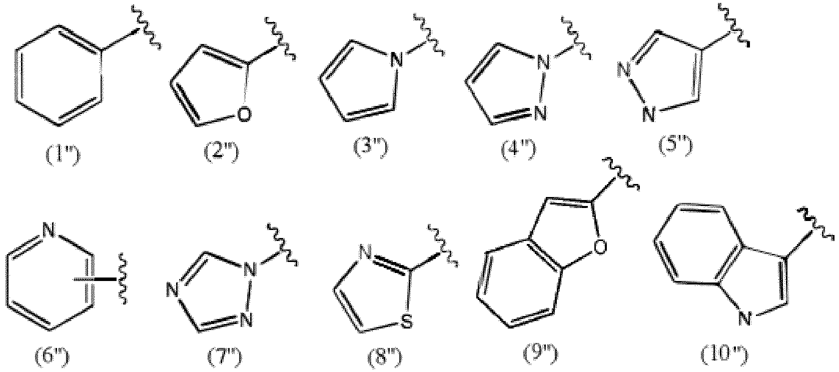

8. Verbindung nach einem der Ansprüche 1 bis 7, wobei
A ein Ring der Formel (1'), (2'), (3'), (4'), (5'), (7'), (14'), (16') oder (20') ist;
R₁ H, C₁₋₇-Alkyl, C₁₋₇-Alkoxy, Hydroxy-C₁₋₇-Alkyl, C₁₋₇-Alkylamino-C₁₋₇-alkyl oder-E-R₆ ist;
R₂ H ist;
Z CH ist;
B ein Ring der Formel (1"), (2"), (3"), (4") oder (6") ist; E eine Bindung oder ein C₁₋₇-Alkyl ist;
R₆ eine der folgenden Gruppen ist: 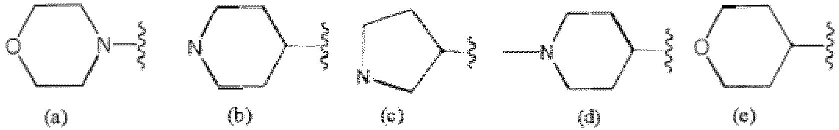 R₃ H, Halogen, C₁₋₇-Alkyl, C₁₋₇-Alkoxy ist;
R₄ H oder Halogen ist;
R₅ -C(O)R₇ oder -SO₂R₈ oder eine der folgenden Gruppen ist: 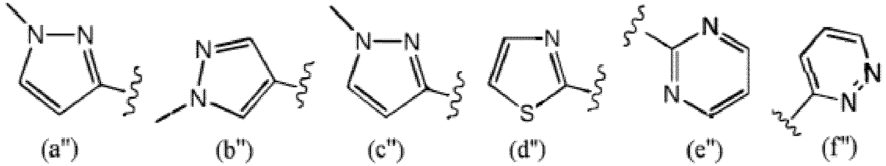 R₇ C₁₋₇-Alkyl, C₂₋₇-Alkenyl oder -NH-R₁₀ ist;
R₈ C₁₋₇-Alkyl, C₂₋₇-Alkenyl, C₃₋₇-Cycloalkyl, Hydroxy-C₁₋₇-Alkyl oder -NR₁₃R₁₄ ist und
R₁₀ C₁₋₇-Alkyl oder C₃₋₇-Cycloalkyl ist.

9. Verbindung nach einem der Ansprüche 1 bis 8, wobei B ein Ring der Formel (1"), (3") oder (6") ist.

10. Verbindung nach einem der Ansprüche 1 bis 9, wobei A ein Ring der Formel (1'), (2'), (4'), (5') oder (20") ist;

11. Verbindung nach einem der Ansprüche 1 bis 10, wobei R₅ -SO₂R₈ ist.

12. Verbindung nach einem der Ansprüche 1 bis 11, wobei Z CH ist, Z₁ N ist und Z₂ CH ist, A ein Ring der Formel (1') ist, B ein Ring der Formel (1") ist, R₁ C₁₋₇-Alkyl ist, R₂ H ist, R₃ Halogen ist, R₄ H oder Halogen ist, R₅ -SO₂R₈ ist und R₈ C₁₋₇-Alkyl oder C₃₋₇-Cycloalkyl ist.

13. Verbindung nach Anspruch 1, wobei B ein 5-12-gliedriger heterocyclischer Ring ist.

14. Verbindung nach Anspruch 1, wobei R₃ Halogen, C₁₋₇-Alkyl, C₁₋₇-Alkoxy, Halogen-C₁₋₇-Alkyl oder Halogen-C₁₋₇-Alkoxy ist.

15. Verbindung nach einem der Ansprüche 1 bis 12, die Folgendes ist:
4-(2, 4-Difluorphenyl)-N-(1-methyl-1H-pyrazol-3-yl)-6-(5-(1-methyl-1H-pyrazol-4-yl)-1H-benzo[d]imidazol-1-yl) pyridin-2-amin;
N-(4-(2,4-Difluorphenyl)-6-(5-(1-methyl-1H-pyrazol-4-yl)-1H-benzo [d]imidazol-1-yl)pyridin-2-yl)cyclopropansulfonamid;
Natriumsalz der Imidform von N-(4-(2,4-Difluorphenyl)-6-(5-(1-methyl-1H-pyrazol-4-yl)-1H-benzo[d]imidazol-1-yl)pyridin-2-yl)cyclopropansulfonamid;
N-(4-(2,4-Difluorphenyl)-6-(5-(1-methyl-1H-pyrazol-4-yl)-1H-benzo[d]imidazol-1-yl)pyridin-2-yl)methansulfonamid;
N-(4-(2,4-Difluorphenyl)-6-(5-(1-methyl-1H-pyrazol-4-yl)-1H-benzo[d]imidazol-1-yl)pyridin-2-yl)ethansulfonamid;
Natriumsalz der Imidform von N-(4-(2,4-Difluorphenyl)-6-(5-(1-methyl-1H-pyrazol-4-yl)-1H-benzo[d]imidazol-1-yl)pyridin-2-yl)ethansulfonamid;
N-(4-(2,4-Difluorphenyl)-6-(5-(1-methyl-1H-pyrazol-4-yl)-1H-benzo[d]imidazol-1-yl)pyridin-2-yl)propan-2-sulfonamid;
Imidform von N-(4-(2,4-Difluorphenyl)-6-(5-(1-methyl-1H-pyrazol-4-yl)-1H-benzo[d]-imidazol-1-yl)pyridin-2-yl)propan-2-sulfonamid;
N-(4-(2-Fluorphenyl)-6-(5-(1-methyl-1H-pyrazol-4-yl)-1H-benzo[d]imidazol-1-yl)pyridin-2-yl)cyclopropansulfonamid;
Natriumsalz der Imidform von N-(4-(2-Fluorphenyl)-6-(5-(1-methyl-1H-pyrazol-4-yl)-1H-benzo[d]imidazol-1-yl)pyridin-2-yl)cyclopropansulfonamid;
N-(4-(2-Fluorphenyl)-6-(5-(1-methyl-1H-pyrazol-4-yl)-1H-benzo[d]imidazol-1-yl)pyridin-2-yl)methansulfonamid;
N-(4-(2-Fluorphenyl)-6-(5-(1-methyl-1H-pyrazol-4-yl)-1H-benzo [d]imidazol-1-yl)pyridin-2-yl)ethansulfonamid;
Natriumsalz der Imidform von N-(4-(2-Fluorphenyl)-6-(5-(1-methyl-1H-pyrazol-4-yl)-1H-benzo [d]imidazol-1-yl)pyridin-2-yl)ethansulfonamid;
N-(4-(2-Fluorphenyl)-6-(5-(1-methyl-1H-pyrazol-4-yl)-1H-benzo[d]imidazol-1-yl)pyridin-2-yl)propan-2-sulfonamid;
Natriumsalz der Imidform von N-(4-(2-Fluorphenyl)-6-(5-(1-methyl-1H-pyrazol-4-yl)-1H-benzo[d]imidazol-1-yl)pyridin-2-yl)propan-2-sulfonamid;
N-(4-(4-Fluorphenyl)-6-(5-(1-methyl-1H-pyrazol-4-yl)-1H-benzo[d]imidazol-1-yl)pyridin-2-yl)cyclopropansulfonamid;
N-(4-(4-Fluorphenyl)-6-(5-(1-methyl-1H-pyrazol-4-yl)-1H-benzo[d]imidazo 1-1-yl)pyridin-2-yl)methansulfonamid;
N-(4-(4-Fluorphenyl)-6-(5-(1-methyl-1H-pyrazol-4-yl)-1H-benzo[d]imidazol-1-yl)pyridin-2-yl)propan-2-sulfonamid;
Natriumsalz der Imidform von N-(4-(4-Fluorphenyl)-6-(5-(1-methyl-1H-pyrazol-4-yl)-1H-benzo [d]imidazol-1-yl)pyridin-2-yl)propan-2-sulfonamid;
N-(3-Fluor-6'-(5-(1-methyl-1H-pyrazol-4-yl)-1H-benzo[d]imidazol-1-yl)-[2,4'-bipyridin]-2'-yl)cyclopropansulfonamid;
N-(3-Fluor-6'-(5-(1-methyl-1H-pyrazol-4-yl)-1H-benzo[d]imidazol-1-yl)-[2,4'-bipyridin]-2'-yl)acetamid;
N-(4-(2,4-Difluorphenyl)-6-(5-(1-(2-(dimethylamino)ethyl)-1H-pyrazol-4-yl)-1H-benzo[d]imidazol-1-yl)pyridin-2-yl)cyclopropansulfonamid;
N-(4-(2-Fluorphenyl)-6-(5-(1-methyl-1H-pyrazol-4-yl)-1H-benzo[d]imidazol-1-yl)pyrimidin-2-yl)cyclopropansulfonamid;
N-(6-(5-(1H-Pyrazol-1-yl)-1H-benzo[d]imidazol-1-yl)-4-(2,4-difluorphenyl)pyridin-2-yl)cyclopropansulfonamid;
N-(3,5-Difluor-6'-(5-(1-methyl-1H-pyrazol-4-yl)-1H-benzo[d]imidazol-1-yl)-[2,4'-bipyridin]-2'-yl)cyclopropansulfonamid;
N-(3,5-Difluor-6'-(5-(1-methyl-1H-pyrazol-4-yl)-1H-benzo[d]imidazol-1-yl)-[2,4'-bipyridin]-2'-yl)acetamid;
N-(4-(2-Chlorphenyl)-6-(5-(1-methyl-1H-pyrazol-4-yl)-1H-benzo[d]imidazol-1-yl)pyridin-2-yl)cyclopropansulfonamid;
N-(3-Chlor-6'-(5-(1-methyl-1H-pyrazol-4-yl)-1H-benzo[d]imidazol-1-yl)-[2,4'-bipyridin]-2'-yl)cyclopropansulfonamid;
N-(5-Fluor-6'-(5-(1-methyl-1H-pyrazol-4-yl)-1H-benzo[d]imidazol-1-yl)-[2,4'-bipyridin]-2'-yl)cyclopropansulfonamid;
N-(6-(5-(1H-Imidazol-1-yl)-1H-benzo[d]imidazol-1-yl)-4-(2,4-difluorphenyl)pyridin-2-yl)cyclopropansulfonamid;
N-(4-(2,4-Difluorphenyl)-6-(5-(1-(2-morpholinoethyl)-1H-pyrazol-4-yl)-1H-benzo[d]imidazol-1-yl)pyridin-2-yl)cyclopropansulfonamid;
N-(4-(2,4-Difluorphenyl)-6-(5-(1-(pyrrolidin-3-yl)-1H-pyrazol-4-yl)-1H-benzo[d]-imidazol-1-yl)pyridin-2-yl)cyclopropansulfonamid;
N-(4-(2,4-Difluorphenyl)-6-(5-(1-ethyl-1H-1,2,3-triazol-4-yl)-1H-benzo[d]imidazol-1-yl)pyridin-2-yl)cyclopropansulfonamid;
N-(4-(2,4-Difluorphenyl)-6-(5-(1-methyl-1H-1,2,3-triazol-4-yl)-1H-benzo[d]imidazol-1-yl)pyridin-2-yl)cyclopropansulfonamid;
N-(4-(2,4-Difluorphenyl)-6-(5-(1-methyl-1H-imidazol-4-yl)-1H-benzo[d]imidazol-1-yl)pyridin-2-yl)cyclopropansulfonamid;
N-(4-(2,4-Difluorphenyl)-6-(5-(1-methyl-1H-pyrazol-4-yl)-1H-benzo[d]imidazol-1-yl)pyrimidin-2-yl)acetamid;
Ethyl1-(1-(6-(cyclopropansulfonamid)-4-(2,4-difluorphenyl)pyridin-2-yl)-1H-benzo[d]imidazol-5-yl)-1H-1,2,3-triazol-4-carboxylat;
N-(4-(2-(Difluormethoxy)-4-fluorphenyl)-6-(5-(1-methyl-1H-pyrazol-4-yl)-1H-benzo[d]imidazol-1-yl)pyridin-2-yl)cyclopropansulfonamid;
N-(4-(2,4-Difluorphenyl)-6-(5-(1-methyl-1H-pyrazol-4-yl)-1H-benzo[d]imidazol-1-yl)pyrimidin-2-yl)cyclopropansulfonamid;
N-(6-(2,4-Difluorphenyl)-4-(5-(1-methyl-1H-pyrazol-4-yl)-1H-benzo[d]imidazol-1-yl) pyridin-2-yl) cyclopropansulfonamid;
oder ein pharmazeutisch verträgliches Salz oder Tautomer davon.

16. Pharmazeutische Zusammensetzung, die eine Verbindung nach einem der Ansprüche 1 bis 15 mit einen pharmazeutisch verträglichen Träger umfasst.

17. Verbindung nach einem der Ansprüche 1 bis 15, zur Verwendung in einem Verfahren zur Behandlung einer Erkrankung, bei dem eine Hemmung der FGFR-Kinase erwünscht ist.

18. Verbindung nach einem der Ansprüche 1 bis 15, zur Verwendung in einem Verfahren zur Behandlung von Krebs.

## Revendications

1. Composé qui est de la formule (I) 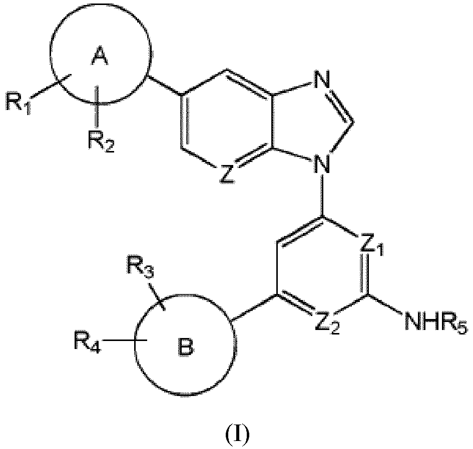 dans lequel
Z₁ est N et Z₂ est CH, ou
Z₁ est CH et Z₂ est N, ou
Z₁ et Z₂ est N ;
Z est CH ou N ;
A est un anneau phényle ou un anneau hétérocyclique à 5 à 12 membres ;
R₁ est H, alkyle C₁₋₇, cycloalkyle C₃₋₇, cycloalkyle C₃₋₇ alkyle C₁₋₇, alkoxy C₁₋₇, alkyle C₁₋₇ carbonyle, amino, hydroxy, hydroxy alkyle C₁₋₇, halo alkyle C₁₋₇, alkylamino C₁₋₇ alkyle C₁₋₇, -R₁₆-C(O)-R₁₇ ou -E-R₆ ;
R₂ est H, halogène ou alkyle C₁₋₇;
B est un anneau carbocyclique à 5 à 12 membres et R₃ est halogène, alkyle C₁₋₇, alkoxy C₁₋₇, halo alkyle C₁₋₇ ou halo alkoxy C₁₋₇; ou B est un anneau hétérocyclique à 5 à 12 membres et R₃ est H, halogène, alkyle C₁₋₇, alkoxy C₁₋₇, halo alkyle C₁₋₇ ou halo alkoxy C₁₋₇;
R₄ est H, halogène, alkyle C₁₋₇ ou oxo ;
R₅ est H, -C(O)R₇, -SO₂R₈ ou un anneau hétérocyclique à 5 à 6 membres, optionnellement substitué ;
R₆ est un anneau hétérocyclique à 5 à 6 membres, optionnellement substitué ;
R₇ est alkyle C₁₋₇, alkényle C₂₋₇, alkoxy C₁₋₇, alkoxy C₁₋₇ alkyle C₁₋₇, carboxy alkyle C₁₋₇, alkoxy C₁₋₇ carbonyle alkyle C₁₋₇, alkylamino C₁₋₇ alkyle C₁₋₇, -NH-R₁₀ ou -NH-X₁-R₁₁ ;
R₈ est alkyle C₁₋₇, alkényle C₂₋₇, cycloalkyle C₃₋₇, hydroxy alkyle C₁₋₇, -NR₁₃R₁₄, -NH-X₂-R₁₅, phényle ou un anneau hétérocyclique à 5 à 6 membres, optionnellement substitué ;
R₁₀ est alkyle C₁₋₇ ou cycloalkyle C₃₋₇ ;
R₁₁ est phényle ou un anneau hétérocyclique à 5 à 6 membres, optionnellement substitué ;
R₁₃ et R₁₄ sont, indépendamment, H, alkyle C₁₋₇ ou cycloalkyle C₃₋₇ ;
R₁₅ est phényle ou un anneau hétérocyclique à 5 à 6 membres, optionnellement substitué ;
R₁₆ est une liaison ou un alkyle C₁₋₇ ;
R₁₇ est alkyle C₁₋₇, alkoxy C₁₋₇, alkylamino C₁₋₇, amino ou hydroxy ;
E est une liaison ou un alkyle C₁₋₇;
X₁ et X₂ sont, indépendamment, une liaison ou alkyle C_{1-7;} ou
est un sel pharmaceutiquement acceptable de celui-ci.

2. Composé selon la revendication 1, dans lequel Z est CH.

3. Composé selon la revendication 1 ou 2, dans lequel Z₁ est N et Z₂ est CH.

4. Composé selon la revendication 1 ou 2, dans lequel Z₁ est CH et Z₂ est N.

5. Composé selon la revendication 1 ou 2, dans lequel Z₁ et Z₂ est N.

6. Composé selon l'une quelconque des revendications 1 à 5, dans lequel l'anneau A est l'un quelconque des groupes suivants ou tautomères de ceux-ci :

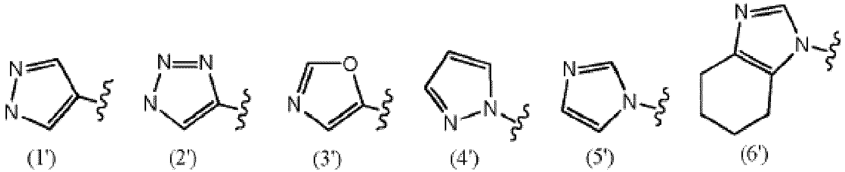

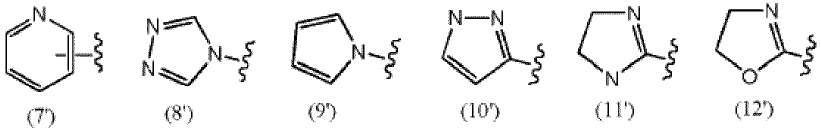

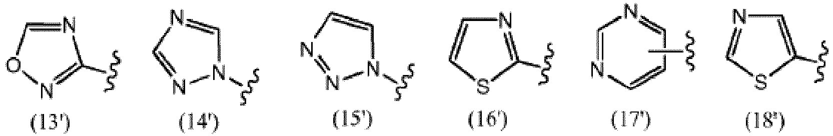

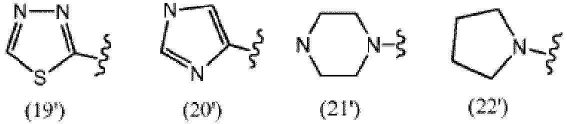

7. Composé selon l'une quelconque des revendications 1 à 6, dans lequel l'anneau B est l'un quelconque des groupes suivants ou tautomères de ceux-ci :

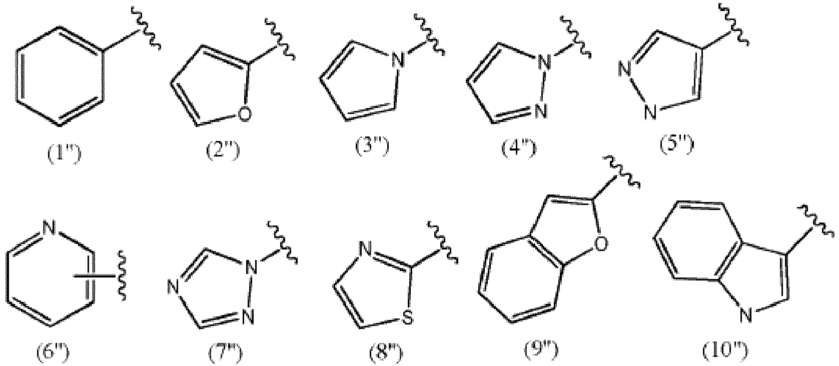

8. Composé selon l'une quelconque des revendications 1 à 7, dans lequel
A est un anneau de la formule (1'), (2'), (3'), (4'), (5'), (7'), (14'), (16') ou (20') ;
R₁ est H, alkyle C₁₋₇, alkoxy C₁₋₇, hydroxy alkyle C₁₋₇, alkylamino C₁₋₇ alkyle C₁₋₇ ou-E-R₆;
R₂ est H ;
Z est CH ;
B est un anneau de la formule (1"), (2 "), (3"), (4") ou (6") ; E est une liaison ou alkyle C₁₋₇;
R₆ est l'un quelconque des groupes suivants : 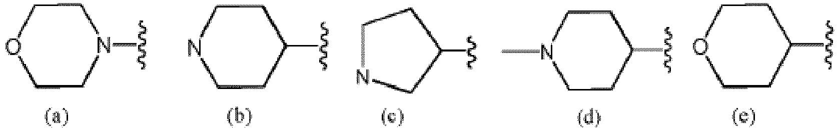 R₃ est H, halogène, alkyle C₁₋₇, alkoxy C₁₋₇ ;
R₄ est H ou halogène ;
R₅ est -C(O)R₇ ou -SO₂R₈ ou l'un quelconque des groupes suivants : 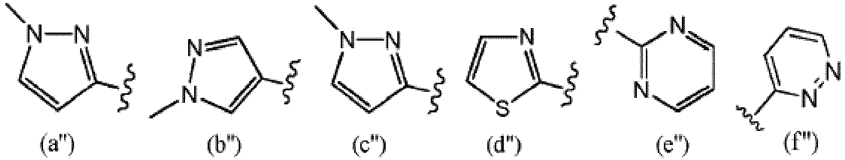 R₇ est alkyle C₁₋₇, alkényle C₂₋₇ ou -NH-R₁₀ ;
R₈ est alkyle C₁₋₇, alkényle C₂₋₇, cycloalkyle C₃₋₇, hydroxy alkyle C₁₋₇ ou -NR₁₃R₁₄ ; et R₁₀ est alkyle C₁₋₇ ou cycloalkyle C₃₋₇.

9. Composé selon l'une quelconque des revendications 1 à 8, dans lequel B est un anneau de la formule (1"), (3 ") ou (6").

10. Composé selon l'une quelconque des revendications 1 à 9, dans lequel A est un anneau de la formule (1'), (2'), (4'), (5') ou (20").

11. Composé selon l'une quelconque des revendications 1 à 10, dans lequel R₅ est - SO₂R₈.

12. Composé selon l'une quelconque des revendications 1 à 11, dans lequel Z est CH, Z₁ est N et Z₂ est CH, A est un anneau de la formule (1'), B est un anneau de la formule (1"), R₁ est alkyle C₁₋₇, R₂ est H, R₃ est halogène, R₄ est H ou halogène, R₅ est -SO₂R₈ et R₈ est alkyle C₁₋₇ ou cycloalkyle C₃₋₇.

13. Composé selon la revendication 1, dans lequel B est un anneau hétérocyclique à 5 à 12 membres.

14. Composé selon la revendication 1, dans lequel R₃ est halogène, alkyle C₁₋₇, alkoxy C₁₋₇, halo alkyle C₁₋₇ ou halo alkoxy C₁₋₇.

15. Composé selon l'une quelconque des revendications 1 à 12, qui est
4-(2,4-Difluorophényl)-N-(1-méthyl-1H-pyrazol-3-yl)-6-(5-(1-méthyl-1H-pyrazol-4-yl)-1H-benzo[d]imidazol-1-yl)pyridin-2-amine ;
N-(4-(2,4-difluorophényl)-6-(5-(1-méthyl-1H-pyrazol-4-yl)-1H-benzo[d]imidazol-1-yl)pyridin-2-yl)cyclopropanesulfonamide ;
Sel de sodium de forme imido de N-(4-(2,4-difluorophényl)-6-(5-(1-méthyl-1H-pyrazol-4-yl)-1H-benzo[d]imidazol-l-yl)pyridin-2-yl)cyclopropanesulfonamide ;
N-(4-(2,4-difluorophényl)-6-(5-(1-méthyl-1H-pyrazol-4-yl)-1H-benzo[d]imidazol-1-yl)pyridin-2-yl)méthanesulfonamide ;
N-(4-(2,4-difluorophényl)-6-(5-(1-méthyl-1H-pyrazol-4-yl)-1H-benzo[d]imidazol-1-yl)pyridin-2-yl)éthanesulfonamide ;
Sel de sodium de forme imido de N-(4-(2,4-difluorophényl)-6-(5-(1-méthyl-1H-pyrazol-4-yl)-1H-benzo[d]imidazol-1-yl)pyridin-2-yl)éthanesulfbnamide ;
N-(4-(2,4-difluorophényl)-6-(5-(1-méthyl-1H-pyrazol-4-yl)-1H-benzo[d]imidazol-1-yl)pyridin-2-yl)propane-2-sulfonamide ;
Forme imido de N-(4-(2,4-difluorophényl)-6-(5-(1-méthyl-1H-pyrazol-4-yl)-1H-benzo[d]-imidazol-1-yl)pyridin-2-yl)propane-2-sulfonamide ;
N-(4-(2-fluorophényl)-6-(5-(1-méthyl-1H-pyrazol-4-yl)-1H-benzo[d]imidazol-1-yl)pyridin-2-yl)cyclopropanesulfonamide ;
Sel de sodium de forme imido de N-(4-(2-fluorophényl)-6-(5-(1-méthyl-1H-pyrazol-4-yl)-1H-benzo[d]imidazol-1-yl)pyridin-2-yl)cyclopropanesulfonamide ;
N-(4-(2-fluorophényl)-6-(5-(1-méthyl-1H-pyrazol-4-yl)-1H-benzo[d]imidazol-1-yl)pyridin-2-yl)méthanesulfonamide ;
N-(4-(2-fluorophényl)-6-(5-(1-méthyl-1H-pyrazol-4-yl)-1H-benzo[d]imidazol-1-yl)pyridin-2-yl)éthanesulfonamide ;
Sel de sodium de forme imido de N-(4-(2-fluorophényl)-6-(5-(1-méthyl-1H-pyrazol-4-yl)-1H-benzo[d]imidazol-1-yl)pyridin-2-yl)éthanesulfonamide ;
N-(4-(2-fluorophényl)-6-(5-(1-méthyl-1H-pyrazol-4-yl)-1H-benzo[d]imidazol-1-yl)pyridin-2-yl)propane-2-sulfonamide ;
Sel de sodium de forme imido de N-(4-(2-fluorophényl)-6-(5-(1-méthyl-1H-pyrazol-4-yl)-1H-benzo[d]imidazol-1-yl)pyridin-2-yl)propane-2-sulfonamide ;
N-(4-(4-fluorophényl)-6-(5-(1-méthyl-1H-pyrazol-4-yl)-1H-benzo[d]imidazol-1-yl)pyridin-2-yl)cyclopropanesulfonamide ;
N-(4-(4-fluorophényl)-6-(5-(1-méthyl-1H-pyrazol-4-yl)-1H-benzo[d]imidazol-1-yl)pyridin-2-yl)méthanesulfonamide ;
N-(4-(4-fluorophényl)-6-(5-(1-méthyl-1H-pyrazol-4-yl)-1H-benzo[d]imidazol-1-yl)pyridin-2-yl)propane-2-sulfonamide ;
Sel de sodium de forme imido de N-(4-(4-fluorophényl)-6-(5-(1-méthyl-1H-pyrazol-4-yl)-1H-benzo[d]imidazol-1-yl)pyridin-2-yl)propane-2-sulfonamide ;
N-(3-fluoro-6'-(5-(1-méthyl-1H-pyrazol-4-yl)-1H-benzo[d]imidazol-1-yl)-[2,4'-bipyridin]-2'-yl)cyclopropanesulfonamide ;
N-(3-fluoro-6'-(5-(1-méthyl-1H-pyrazol-4-yl)-1H-benzo[d]imidazol-1-yl)-[2,4'-bipyridin]-2'-yl)acétamide ;
N-(4-(2,4-difluorophényl)-6-(5-(1-(2-(diméthylamino)éthyl)-1H-pyrazol-4-yl)-1H-benzo[d]imidazol-1-yl)pyridin-2-yl)cyclopropanesulfonamide ;
N-(4-(2-fluorophényl)-6-(5-(1-méthyl-1H-pyrazol-4-yl)-1H-benzo[d]imidazol-1-yl)pyrimidin-2-yl)cyclopropanesulfonamide ;
N-(6-(5-(1H-pyrazol-1-yl)-1H-benzo[d]imidazol-1-yl)-4-(2,4-difluorophényl)pyridin-2-yl)cyclopropanesulfonamide ;
N-(3,5-difluoro-6'-(5-(1-méthyl-1H-pyrazol-4-yl)-1H-benzo[d]imidazol-1-yl)-[2,4'-bipyridin]-2'-yl)cyclopropanesulfonamide ;
N-(3,5-difluoro-6'-(5-(1-méthyl-1H-pyrazol-4-yl)-1H-benzo[d]imidazol-1-yl)-[2,4'-bipyridin]-2'-yl)acétamide ;
N-(4-(2-chlorophényl)-6-(5-(1-méthyl-1H-pyrazol-4-yl)-1H-benzo[d]imidazol-1-yl)pyridin-2-yl)cyclopropanesulfonamide ;
N-(3-chloro-6'-(5-(1-méthyl-1H-pyrazol-4-yl)-1H-benzo[d]imidazol-1-yl)-[2,4'-bipyridin]-2'-yl)cyclopropanesulfonamide ;
N-(5-fluoro-6'-(5-(1-méthyl-1H-pyrazol-4-yl)-1H-benzo[d]imidazol-1-yl)-[2,4'-bipyridin]-2'-yl)cyclopropanesulfonamide ;
N-(6-(5-(1H-imidazol-1-yl)-1H-benzo[d]imidazol-1-yl)-4-(2,4-difluorophényl)pyridin-2-yl)cyclopropanesulfonamide ;
N-(4-(2,4-difluorophényl)-6-(5-(1-(2-morpholinoéthyl)-1H-pyrazol-4-yl)-1H-benzo[d]imidazol-1-yl)pyridin-2-yl)cyclopropanesulfonamide ;
N-(4-(2,4-difluorophényl)-6-(5-(1-(pyrrolidin-3-yl)-1H-pyrazol-4-yl)-1H-benzo[d]-imidazol-1-yl)pyridin-2-yl)cyclopropanesulfonamide ;
N-(4-(2,4-difluorophényl)-6-(5-(1-ethyl-1H-1,2,3-triazol-4-yl)-1H-benzo[d]imidazol-1-yl)pyridin-2-yl)cyclopropanesulfonamide ;
N-(4-(2,4-difluorophényl)-6-(5-(1-méthyl-1H-1,2,3-triazol-4-yl)-1H-benzo[d]imidazol-1-yl)pyridin-2-yl)cyclopropanesulfonamide ;
N-(4-(2,4-difluorophényl)-6-(5-(1-méthyl-1H-imidazol-4-yl)-1H-benzo[d]imidazol-1-yl)pyridin-2-yl)cyclopropanesulfonamide ;
N-(4-(2,4-difluorophényl)-6-(5-(1-méthyl-1H-pyrazol-4-yl)-1H-benzo[d]imidazol-1-yl)pyrimidin-2-yl)acétamide ;
1-(1-(6-(cyclopropanesulfonamido)-4-(2,4-difluorophényl)pyridin-2-yl)-1H-benzo[d]imidazol-5-yl)-1H-1,2,3-triazole-4-carboxylate d'éthyle ;
N-(4-(2-(difluoromethoxy)-4-fluorophényl)-6-(5-(1-méthyl-1H-pyrazol-4-yl)-1H-benzo[d]imidazol-1-yl)pyridin-2-yl)cyclopropanesulfonamide ;
N-(4-(2,4-difluorophényl)-6-(5-(1-méthyl-1H-pyrazol-4-yl)-1H-benzo[d]imidazol-1-yl)pyrimidin-2-yl)cyclopropanesulfonamide ;
N-(6-(2,4-difluorophényl)-4-(5-(1-méthyl-1H-pyrazol-4-yl)-1H-benzo[d]imidazol-1-yl)pyridin-2-yl)cyclopropanesulfonamide ;
ou un sel ou tautomère pharmaceutiquement acceptable de ceux-ci.

16. Composition pharmaceutique, comprenant un composé selon l'une quelconque des revendications 1 à 15 avec un vecteur pharmaceutiquement acceptable.

17. Composé selon l'une quelconque des revendications 1 à 15 pour l'utilisation dans une méthode de traitement d'une condition, où l'inhibition de FGFR kinase est souhaitée.

18. Composé selon l'une quelconque des revendications 1 à 15 pour l'utilisation dans une méthode de traitement du cancer.
